(19) Europäisches Patentamt
European Patent Office
Office européen des brevets

(11) EP 2 072 550 A1

(12) EUROPÄISCHE PATENTANMELDUNG

(43) Veröffentlichungstag:
**24.06.2009 Patentblatt 2009/26**

(21) Anmeldenummer: **07150364.3**

(22) Anmeldetag: **21.12.2007**

(51) Int Cl.:
***C08G 18/12*** *(2006.01)* ***C08G 18/30*** *(2006.01)*
***C08G 18/32*** *(2006.01)* ***C08G 18/48*** *(2006.01)*
***C08G 18/50*** *(2006.01)* ***C08G 18/75*** *(2006.01)*
***C08G 18/76*** *(2006.01)* ***C09J 175/08*** *(2006.01)*
***C07C 243/24*** *(2006.01)* ***C08K 5/30*** *(2006.01)*
***C07C 251/72*** *(2006.01)*

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(71) Anmelder: **Sika Technology AG**
**6340 Baar (CH)**

(72) Erfinder: **Burckhardt, Urs**
**8049, Zürich (CH)**

(74) Vertreter: **Sika Patent Attorneys**
**C/o Sika Technology AG**
**Tüffenwies 16**
**Postfach**
**8048 Zürich (CH)**

(54) **Härtbare Zusammensetzungen mit verminderter Ausgasung**

(57) Die vorliegende Erfindung betrifft insbesondere härtbare Zusammensetzungen, welche mindestens ein Polyisocyanat, mindestens ein mittels Aldehyd oder Keton blockiertes Amin, und mindestens ein Hydrazid einer Carbon- oder Sulfonsäure, welches einen Schmelzpunkt von mindestens 100 °C, insbesondere von mindestens 150 °C, aufweist, umfassen.

Es hat sich gezeigt, dass derartige Zusammensetzungen, nachdem sie bei Raumtemperatur mittels Feuchtigkeit ausgehärtet wurden, bei Erwärmung auf erhöhte Temperaturen eine bedeutend tiefere Ausgasung von flüchtigen Bestandteilen zeigen als die entsprechenden Zusammensetzungen ohne das Hydrazid.



Printed by Jouve, 75001 PARIS (FR)

## Beschreibung

### Technisches Gebiet

Die vorliegende Erfindung betrifft das Gebiet der feuchtigkeitshärtenden Polyurethanzusammensetzungen sowie deren Verwendung, insbesondere als elastische Klebstoffe im Fahrzeugbau.

### Stand der Technik

Isocyanatgruppen aufweisende härtbare Zusammensetzungen, auch als Polyurethanzusammensetzungen bezeichnet, werden seit langem in verschiedensten Anwendungen eingesetzt, unter anderem als ein- und zweikomponentige elastische Klebstoffe, Dichtstoffe oder Beschichtungen. Zur besseren Aushärtung solcher Zusammensetzungen können diesen durch Feuchtigkeit aktivierbare Amin-Vernetzer, sogenannte "blockierte Amine" oder "latente Härter", beigesetzt werden, welche die direkte, Kohlendioxid ($CO_2$) erzeugende Reaktion der Isocyanatgruppen mit Feuchtigkeit und damit die Entstehung von unerwünschten Gasblasen in der ausgehärteten Zusammensetzung weitgehend unterbinden.

Der Einsatz von blockierten Aminen kann aber auch Probleme verursachen, insbesondere aufgrund der Tatsache, dass bei der Aushärtungsreaktion Aldehyde oder Ketone entstehen. Diese sogenannten "Abspaltungsprodukte" werden nicht in die Polyurethan-Matrix eingebaut und können deshalb durch Verdampfung oder Migration in die Umgebung entweichen. Die aus den blockierten Aminen entstehenden Aldehyde oder Ketone stellen vielfach geruchsintensive und/oder gesundheitsbelastende Substanzen dar.

Insbesondere bei Anwendungen in Innenräumen kann die Ausgasung von organischen Substanzen aus beispielsweise einem Klebstoff in die Umgebungsluft problematisch sein. Leicht flüchtige Substanzen reichern sich im Innenraum rasch an und können, insbesondere wenn sie geruchsintensiv sind, Übelkeit oder Atemwegsreizungen verursachen. Schwerer flüchtige, geruchsarme Substanzen machen sich meist nicht unmittelbar bemerkbar; sie können sich aber durch Kondensation auf Oberflächen abscheiden und dadurch allmählich zur Bildung von Beschlägen führen, beispielsweise auf Scheiben. In der Automobilindustrie wird die Bildung solcher Beschläge, welche zu störenden Trübungen führen können, als "Fogging" bezeichnet. Bei Materialien wie beispielsweise Klebstoffen, welche im Fahrzeug-Innenraum eingesetzt werden sollen, werden dort bezüglich ausgasbaren Substanzen generell strenge Massstäbe angelegt.

In WO 2004/013088 A1 und WO 2007/036571 A1 sind blockierte Amine in der Form von geruchsfreien Aldiminen und solche Aldimine enthaltende Polyurethanzusammensetzungen offenbart. Bei den bei der Aushärtung der Zusammensetzungen freigesetzten Aldehyden handelt es sich um geruchsfreie, relativ schwerflüchtige Verbindungen, welche bei Raumtemperatur weitgehend in der ausgehärteten Zusammensetzung verbleiben. Bei erhöhter Temperatur kann es aber trotzdem zu einer Ausgasung dieser Aldehyde aus der Zusammensetzung kommen.

Aus DE 1 156 552 und US 6,051,620 sind Hydrazid enthaltende Polyurethanzusammensetzungen bekannt. In diesen Systemen werden die eingesetzten Hydrazide vor oder während der Reaktion der Isocyanatgruppen umgesetzt.

### Darstellung der Erfindung

Aufgabe der vorliegenden Erfindung ist es deshalb, eine Polyurethanzusammensetzung zur Verfügung zu stellen, welche die Vorteile der aus dem Stand der Technik bekannten, mittels blockierten Aminen aushärtenden Zusammensetzungen aufweist, aber im ausgehärteten Zustand eine deutlich reduzierte Ausgasung von Aldehyden oder Ketonen zeigt, insbesondere bei erhöhter Temperatur, beispielsweise bei 80°C oder höher.

Überraschenderweise wurde gefunden, dass härtbare Zusammensetzungen nach Anspruch 1, welche neben einem mittels Aldehyd oder Keton blockierten Amin ein Hydrazid einer Carbon- oder Sulfonsäure mit einem genügend hohen Schmelzpunkt enthalten, diese Aufgabe lösen. Diese Zusammensetzungen härten mit Feuchtigkeit bei Raumtemperatur oder leicht erhöhter Temperatur, insbesondere unterhalb von 40 °C, ohne nennenswerte Beteiligung des Hydrazids aus, wobei Aldehyde und/oder Ketone entstehen. Bei Erwärmung der ausgehärteten Zusammensetzungen, insbesondere auf 80 °C und höher, beginnt das Hydrazid mit den entstandenen Aldehyden und/oder Ketonen zu schwerflüchtigen Kondensationsprodukten zu reagieren, welche auch bei hohen Temperaturen nicht ausgasen. Die ausgehärteten Zusammensetzungen weisen deshalb insbesondere Vorteile beim Fogging-Verhalten auf.

Weitere Gegenstände der Erfindung sind ein Verfahren zum Verkleben gemäss Anspruch 19, zum Abdichten gemäss Anspruch 18 und zum Beschichten gemäss Anspruch 21, die daraus entstehenden Artikel gemäss Anspruch 22, sowie eine ausgehärtete Zusammensetzung gemäss Anspruch 23. Schliesslich bilden ein Aldazid gemäss Anspruch 24 und die Verwendung eines Hydrazids gemäss Anspruch 28 und 29 weitere Aspekte der vorliegenden Erfindung.

Bevorzugte Ausführungsformen der Erfindung sind Gegenstand der Unteransprüche.

## Wege zur Ausführung der Erfindung

Gegenstand der Erfindung ist eine härtbare Zusammensetzung, umfassend

a) mindestens ein Polyisocyanat **P,**
b) mindestens ein mittels Aldehyd oder Keton blockiertes Amin **BA,**
c) mindestens ein Hydrazid **HY** einer Carbon- oder Sulfonsäure, welches einen Schmelzpunkt von mindestens 100 °C, insbesondere von mindestens 150 °C, aufweist,
mit der Massgabe, dass das Hydrazid **HY** in einer Menge von 0.3 bis 1.1 Equivalent Hydrazidgruppen pro Equivalent Aldehyd- oder Ketogruppen, mittels welchen das Amin **BA** blockiert ist, vorhanden ist.

Der Begriff "Hydrazid" bezeichnet im vorliegenden Dokument das Kondensationsprodukt aus einer Carbon- oder Sulfonsäure und Hydrazin.

Die beschriebene härtbare Zusammensetzung umfasst mindestens ein Polyisocyanat **P.**

Der Begriff "Polyisocyanat" umfasst im vorliegenden Dokument Verbindungen mit zwei oder mehr Isocyanatgruppen, unabhängig davon, ob es sich dabei um monomere Diisocyanate, oligomere Polyisocyanate oder Isocyanatgruppen aufweisende Polymere mit einem relativ hohen Molekulargewicht handelt.

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Als Polyisocyanat **P** geeignet ist in einer Ausführungsform ein Isocyanatgruppen aufweisendes Polyurethanpolymer **PUP.**

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind Polyether-Polyurethane, Polyester-Polyurethane, Polyether-Polyharnstoffe, Polyharnstoffe, Polyester-Polyharnstoffe, Polyisocyanurate und Polycarbodiimide.

Ein geeignetes Polyurethanpolymer **PUP** ist insbesondere erhältlich aus der Umsetzung von mindestens einem Polyol mit mindestens einem Polyisocyanat. Diese Umsetzung kann dadurch erfolgen, dass das Polyol und das Polyisocyanat mit üblichen Verfahren, beispielsweise bei Temperaturen von 50 °C bis 100 °C, gegebenenfalls unter Mitverwendung geeigneter Katalysatoren, zur Reaktion gebracht werden, wobei das Polyisocyanat so dosiert ist, dass dessen Isocyanatgruppen im Verhältnis zu den Hydroxylgruppen des Polyols im stöchiometrischen Überschuss vorhanden sind. Vorteilhaft ist das Polyisocyanat so dosiert, dass ein NCO/OH-Verhältnis von 1.3 bis 5, insbesondere eines von 1.5 bis 3, eingehalten wird. Unter dem "NCO/OH-Verhältnis" wird das Verhältnis der Anzahl der eingesetzten Isocyanatgruppen zu der Anzahl der eingesetzten Hydroxylgruppen verstanden. Bevorzugt verbleibt im Polyurethanpolymer **PUP** nach der Umsetzung aller Hydroxylgruppen des Polyols ein Gehalt an freien Isocyanatgruppen von 0.5 bis 15 Gewichts-%, besonders bevorzugt von 0.5 bis 10 Gewichts-%.

Gegebenenfalls kann das Polyurethanpolymer **PUP** unter Mitverwendung von Weichmachern hergestellt werden, wobei die verwendeten Weichmacher keine gegenüber Isocyanaten reaktive Gruppen enthalten.

Als Polyole für die Herstellung eines Polyurethanpolymers **PUP** können beispielsweise die folgenden handelsüblichen Polyole oder Mischungen davon eingesetzt werden:

- Polyoxyalkylenpolyole, auch Polyetherpolyole oder Oligoetherole genannt, welche Polymerisationsprodukte von Ethylenoxid, 1,2-Propylenoxid, 1,2- oder 2,3-Butylenoxid, Oxetan, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit

Hilfe von anionischen Katalysatoren wie NaOH, KOH, CsOH oder Alkalialkoholaten. Besonders geeignet sind Polyoxyalkylendiole oder Polyoxyalkylentriole, insbesondere Polyoxyethylen- und Polyoxypropylendi- und -triole. Speziell geeignet sind Polyoxyalkylendiole und -triole mit einem Ungesättigtheitsgrad tiefer als 0.02 mEq/g und mit einem Molekulargewicht im Bereich von 1'000 - 30'000 g/mol, sowie Polyoxypropylendiole und -triole mit einem Molekulargewicht von 400 - 8'000 g/mol. Ebenfalls besonders geeignet sind sogenannte Ethylenoxid-terminierte ("EO-endcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.

- Styrol-Acrylnitril- oder Acrylnitril-Methylmethacrylat-gepfropfte Polyetherpolyole.
- Polyesterpolyole, auch Oligoesterole genannt, hergestellt beispielsweise aus zwei- bis dreiwertigen Alkoholen wie beispielsweise 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole mit organischen Dicarbonsäuren oder deren Anhydride oder Ester wie beispielsweise Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure und Hexahydrophthalsäure oder Mischungen der vorgenannten Säuren, sowie Polyesterpolyole aus Lactonen wie beispielsweise ε-Caprolacton.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen.
- Polyacrylat- und Polymethacrylatpolyole.
- Polykohlenwasserstoffpolyole, auch Oligohydrocarbonole genannt, wie beispielsweise polyhydroxyfunktionelle Ethylen-Propylen-, Ethylen-Butylen- oder Ethylen-Propylen-Dien-Copolymere, wie sie beispielsweise von der Firma Kraton Polymers hergestellt werden, oder polyhydroxyfunktionelle Copolymere aus Dienen wie 1,3-Butandien oder Diengemischen und Vinylmonomeren wie Styrol, Acrylnitril oder Isobutylen, oder polyhydroxyfunktionelle Polybutadienpolyole, wie beispielsweise solche, die durch Copolymerisation von 1,3-Butadien und Allylalkohol hergestellt werden und auch hydriert sein können.
- Polyhydroxyfunktionelle Acrylonitril/Butadien-Copolymere, wie sie beispielsweise aus Epoxiden oder Aminoalkoholen und carboxylterminierten Acrylonitril/Butadien-Copolymeren (kommerziell erhältlich unter dem Namen Hycar® CTBN von Hanse Chemie) hergestellt werden können.

Diese genannten Polyole weisen bevorzugt ein mittleres Molekulargewicht von 250 - 30'000 g/mol, insbesondere von 400 - 20'000 g/mol, und weisen bevorzugt eine mittlere OH-Funktionalität im Bereich von 1.6 bis 3 auf.

Als Polyole bevorzugt sind Polyether-, Polyester-, Polycarbonat- und Polyacrylatpolyole, bevorzugt Di- und Triole. Besonders bevorzugt sind Polyetherpolyole, insbesondere Polyoxypropylen- und Polyoxypropylenpolyoxyethylenpolyole.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole, 1,1,1-Trimethylolethan, 1,1,1-Trimethylolpropan, Glycerin, Pentaerythrit, Zuckeralkohole wie Xylit, Sorbit oder Mannit, Zucker wie Saccharose, andere höherwertige Alkohole, niedrigmolekulare Alkoxylierungsprodukte der vorgenannten zwei- und mehrwertigen Alkohole, sowie Mischungen der vorgenannten Alkohole bei der Herstellung des Polyurethanpolymers **PUP** mitverwendet werden. Ebenso können kleine Mengen an Polyolen mit einer mittleren OH-Funktionalität von mehr als 3 mitverwendet werden, beispielsweise Zuckerpolyole.

Als Polyisocyanat für die Herstellung eines Isocyanatgruppen aufweisenden Polyurethanpolymers **PUP** werden aromatische oder aliphatische Polyisocyanate, insbesondere die Diisocyanate, eingesetzt.

Als "aromatisches Isocyanat" wird eine organische Verbindung bezeichnet, welche ausschliesslich aromatische Isocyanatgruppen aufweist. Als "aromatisch" wird eine Isocyanatgruppe bezeichnet, die an einen aromatischen oder heteroaromatischen Rest gebunden ist. Als "aliphatisches Isocyanat" wird eine organische Verbindung bezeichnet, die aliphatische Isocyanatgruppen enthält. Als "aliphatisch" wird eine Isocyanatgruppe bezeichnet, die an einen aliphatischen, cycloaliphatischen oder arylaliphatischen Rest gebunden ist.

Als aromatische Polyisocyanate eignen sich beispielsweise monomere Di- oder Triisocyanate wie 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,3,5-Tris-(isocyanatomethyl)-benzol, Tris-(4-isocyanatophenyl)-methan, Tris-(4-isocyanatophenyl)-thiophosphat, Oligomere und Polymere

der vorgenannten Isocyanate, sowie beliebige Mischungen der vorgenannten Isocyanate. Bevorzugt sind MDI und TDI.

Als aliphatische Polyisocyanate eignen sich beispielsweise monomere Di- oder Triisocyanate wie 1,4-Tetramethylendiisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- und -1,4-diisocyanat, 1-Methyl-2,4- und -2,6-diisocyanatocyclohexan und beliebige Gemische dieser Isomeren (HTDI oder $H_6$TDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI oder $H_{12}$MDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-Isocyanato-l-methylethyl)-naphthalin, Dimer- und Trimerfettsäureisocyanate wie 3,6-Bis-(9-isocyanatononyl)-4,5-di-(1-heptenyl)-cydohexen (Dimeryldiisocyanat), $\alpha,\alpha,\alpha',\alpha',\alpha'',\alpha''$-Hexamethyl-1,3,5-mesitylentriisocyanat, Oligomere und Polymere der vorgenannten Isocyanate, sowie beliebige Mischungen der vorgenannten Isocyanate. Bevorzugt sind HDI und IPDI.

Bevorzugt sind Polyurethanpolymere **PUP** mit aromatischen Isocyanatgruppen.

Als Polyisocyanat **P** geeignet ist in einer weiteren Ausführungsform ein Polyisocyanat **PI** in der Form eines monomeren Di- oder Triisocyanates oder eines Oligomeren eines monomeren Diisocyanates, wobei als monomeres Di- oder Triisocyanat beispielsweise die vorgenannten aromatischen und aliphatischen Di- und Triisocyanate geeignet sind.

Als Oligomer eines monomeren Diisocyanates geeignet sind insbesondere die Oligomere von HDI, IPDI und TDI. Solche Oligomere stellen in der Praxis üblicherweise Gemische von Substanzen mit unterschiedlichen Oligomerisationsgraden und/oder chemischen Strukturen dar. Vorzugsweise weisen sie eine mittlere NCO-Funktionalität von 2.1 bis 4.0 auf und enthalten insbesondere Isocyanurat-, Iminooxadiazindion-, Uretdion-, Urethan-, Biuret-, Allophanat-, Carbodiimid-, Uretonimin- oder Oxadiazintrion-Gruppen. Bevorzugt weisen sie einen niedrigen Gehalt an monomeren Diisocyanaten auf. Kommerziell erhältliche Typen sind insbesondere HDI-Biurete, beispielsweise Desmodur® N 100 und Desmodur® N 3200 (von Bayer), Tolonate® HDB und Tolonate® HDB-LV (von Rhodia) sowie Duranate® 24A-100 (von Asahi Kasei); HDI-Isocyanurate, beispielsweise Desmodur® N 3300, Desmodur® N 3600 und Desmodur® N 3790 BA (von Bayer), Tolonate® HDT, Tolonate® HDT-LV und Tolonate® HDT-LV2 (von Rhodia), Duranate® TPA-100 und Duranate® THA-100 (von Asahi Kasei) sowie Coronate® HX (von Nippon Polyurethane); HDI-Uretdione, beispielsweise Desmodur® N 3400 (von Bayer); HDI-Iminooxadiazindione, beispielsweise Desmodur® XP 2410 (von Bayer); HDI-Allophanate, beispielsweise Desmodur® VP LS 2102 (von Bayer); IPDI-Isocyanurate, beispielsweise Desmodur® Z 4470 (von Bayer) und Vestanat® T1890/100 (von Evonik); TDI-Oligomere, beispielsweise Desmodur® IL (von Bayer); sowie gemischte Isocyanurate auf Basis TDI / HDI, zum Beispiel Desmodur® HL (von Bayer).

In einer weiteren Ausführungsform stellt das Polyisocyanat **P** ein Polyisocyanat **PI** in der Form einer bei Raumtemperatur flüssigen Form von MDI oder einer Form von polymerem MDI (PMDI) dar.

Bei Raumtemperatur flüssige Formen von MDI (sogenanntes "modifiziertes MDI") stellen Gemische von MDI mit MDI-Derivaten, wie beispielsweise MDI-Carbodiimiden, MDI-Uretoniminen oder MDI-Urethanen, dar. Kommerziell erhältliche Typen von modifiziertem MDI sind beispielsweise Desmodur® CD, Desmodur® PF und Desmodur® PC (von Bayer), Lupranat® MM 103 (von BASF), Isonate® M 143 (von Dow) sowie Suprasec® 2020 und Suprasec® 2388 (von Huntsman).

Als polymeres MDI oder PMDI werden Gemische aus MDI und MDI-Homologen bezeichnet. Kommerziell erhältliche Typen von PMDI sind beispielsweise Desmodur® VL, Desmodur® VL 50, Desmodur® VL R 10, Desmodur® VL R 20 und Desmodur® VKS 20 F (von Bayer), Lupranat® M 10 R und Lupranat® M 20 R (von BASF), Isonate® M 309, Voranate® M 229 und Voranate M® 580 (von Dow) sowie Suprasec® 5025, Suprasec® 2050 und Suprasec® 2487 (von Huntsman).

Als Polyisocyanat **PI** bevorzugt sind PMDI, bei Raumtemperatur flüssige Formen von MDI, sowie Oligomere von HDI, IPDI und TDI, insbesondere die Isocyanurate.

Als Polyisocyanat **PI** besonders bevorzugt sind solche mit aromatischen Isocyanatgruppen.

Als Polyisocyanat **PI** am meisten bevorzugt sind MDI, insbesondere MDI-Typen mit hohem Anteil, insbesondere 50 Gewichts-% oder mehr, an 2,4'-Isomer, PMDI, sowie bei Raumtemperatur flüssige Formen von MDI.

In einer weiteren Ausführungsform ist das Polyisocyanat **P** eine Mischung bestehend aus mindestens einem Polyurethanpolymer **PUP** und mindestens einem Polyisocyanat **PI,** wie sie vorgängig beschrieben wurden.

Üblicherweise ist das Polyisocyanat **P** in einer Menge von 5 bis 95 Gewichts-%, bevorzugt in einer Menge von 10 bis 90 Gewichts-%, bezogen auf die gesamte Zusammensetzung, vorhanden. In gefüllten Zusammensetzungen, d.h. Zusammensetzungen, welche einen Füllstoff enthalten, ist das Polyisocyanat **P** bevorzugt in einer Menge von 5 bis 60 Gewichts-%, insbesondere 10 bis 50 Gewichts-%, bezogen auf die gesamte Zusammensetzung, vorhanden.

Die beschriebene härtbare Zusammensetzung umfasst weiterhin mindestens ein mittels Aldehyd oder Keton blok-

kiertes Amin **BA.**

Unter einem mittels Aldehyd oder Keton blockierten Amin **BA** wird eine Verbindung verstanden, welche mindestens eine mittels Aldehyd oder Keton blockierte Aminogruppe, ausgewählt aus der Gruppe bestehend aus Aldiminogruppen, Ketiminogruppen, Enaminogruppen und Oxazolidinogruppen, enthält. Das besagte Aldimin oder Keton ist hierbei frei von OH-, SH- und NH-Gruppen.

Als blockiertes Amin **BA** geeignet ist in einer Ausführungsform ein Aldimin **BA1** der Formel (I),

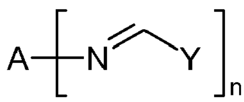

(I)

wobei

n für eine ganze Zahl von 1 bis 5 steht,

A für den Rest eines Amins B nach Entfernung von n primären Aminogruppen steht, und

Y für einen organischen, gegebenenfalls Heteroatome aufweisenden, Rest mit 1 bis 35 C-Atomen steht.

Als "primäre Aminogruppe" wird im vorliegenden Dokument eine $NH_2$-Gruppe bezeichnet, die an einen organischen Rest gebunden ist, und als "sekundäre Aminogruppe" wird eine NH-Gruppe bezeichnet, die an zwei organische Reste, welche auch gemeinsam Teil eines Rings sein können, gebunden ist.

Bevorzugte Aldimine BA1 der Formel (I) sind Aldimine BA1 der Formel (I a) und (I b),

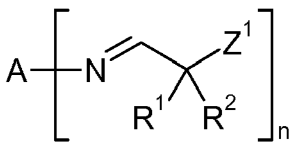

(I a)

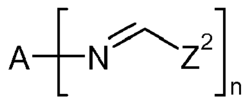

(I b)

wobei

$R^1$ und $R^2$ entweder

unabhängig voneinander jeweils für einen einwertigen Kohlenwasserstoffrest mit 1 bis 12 C-Atomen stehen,

oder zusammen für einen zweiwertigen Kohlenwasserstoffrest mit 4 bis 20 C-Atomen, der Teil eines, gegebenenfalls substituierten, carbocyclischen Rings mit 5 bis 8, bevorzugt 6, C-Atomen ist, stehen;

$Z^1$ für einen einwertigen Kohlenwasserstoffrest mit 1 bis 32 C-Atomen steht, welcher gegebenenfalls mindestens ein Heteroatom, insbesondere Sauerstoff in der Form von Ether-, Carbonyl- oder Ester-Gruppen, oder insbesondere Stickstoff in Form von tertiären Aminogruppen, aufweist;

$Z^2$ entweder

für eine substituierte oder unsubstituierte Aryl- oder Heteroaryl-Gruppe, welche eine Ringgrösse von 5 bis 8, bevorzugt 6, Atomen aufweist, steht, oder für

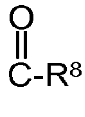

steht,

wobei $R^8$ für ein Wasserstoffatom oder für eine Alkoxygruppe steht, oder für eine substituierte oder unsubstituierte Alkenyl- oder Arylalkenylgruppe mit mindestens 6 C-Atomen steht;

und A und n die bereits erwähnten Bedeutungen aufweisen.

Bevorzugt stehen $R^1$ und $R^2$ in Formel (I a) jeweils für eine Methylgruppe.

Weiterhin bevorzugt steht $Z^1$ in Formel (I a) für einen Rest der Formel (II) oder (III) oder (IV),

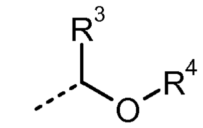

(II)

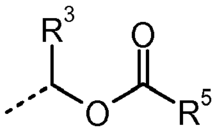

(III)

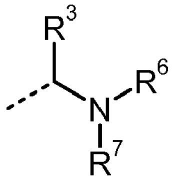

(IV)

wobei
$R^3$ für ein Wasserstoffatom oder für eine Alkylgruppe oder für eine Cycloalkylgruppe oder für eine Arylalkylgruppe mit 1 bis 12 C-Atomen steht;
$R^4$ für einen Kohlenwasserstoffrest mit 1 bis 30 C-Atomen, welcher gegebenenfalls Ethersauerstoffatome enthält, steht;
$R^5$ entweder
für ein Wasserstoffatom steht,
oder für einen linearen oder verzweigten Alkylrest mit 1 bis 30 C-Atomen, gegebenenfalls mit cyclischen Anteilen und gegebenenfalls mit mindestens einem Heteroatom, insbesondere Sauerstoff in der Form von Ether-, Carbonyl- oder Ester-Gruppen, steht,
oder für einen einfach oder mehrfach ungesättigten, linearen oder verzweigten Kohlenwasserstoffrest mit 5 bis 30 C-Atomen steht, oder für einen, gegebenenfalls substituierten, aromatischen oder hetero aromatischen 5- oder 6-gliedrigen Ring steht; und $R^6$ und $R^7$ entweder
unabhängig voneinander jeweils für einen einwertigen aliphatischen, cycloaliphatischen oder arylaliphatischen Rest mit 1 bis 20 C-Atomen stehen und welcher gegebenenfalls Heteroatome in der Form von Ether-Sauerstoff oder tertiärem Amin-Stickstoff enthält, oder zusammen für einen zweiwertigen aliphatischen Rest mit 3 bis 20 C-Atomen, der Teil eines, gegebenenfalls substituierten, heterocyclischen Rings mit 5 bis 8, bevorzugt 6, Ringatomen ist und neben dem Stickstoffatom gegebenenfalls weitere Heteroatome in der Form von Ether-Sauerstoff oder tertiärem Amin-Stickstoff enthält, stehen.

Gestrichelte Linien in den Formeln in diesem Dokument stellen jeweils die Bindung zwischen einem Substituenten und dem zugehörigen Molekülrest dar.

Bevorzugt steht $R^3$ in den Formeln (II), (III) und (IV) für ein Wasserstoffatom.

Bevorzugt steht $R^4$ in Formel (II) für einen Kohlenwasserstoffrest mit 6 bis 30, insbesondere mit 11 bis 30, C-Atomen, welcher gegebenenfalls Ethersauerstoffatome enthält.

Bevorzugt steht $R^5$ in Formel (III) für einen linearen oder verzweigten Alkylrest mit 6 bis 30, insbesondere mit 11 bis 30, C-Atomen, gegebenenfalls mit cyclischen Anteilen und gegebenenfalls mit mindestens einem Heteroatom, oder für einen einfach oder mehrfach ungesättigten, linearen oder verzweigten Kohlenwasserstoffrest mit 6 bis 30, insbesondere mit 11 bis 30, C-Atomen.

Am meisten bevorzugt steht $R^5$ in Formel (III) für einen $C_{11}$-Alkylrest.

Bevorzugt stehen $R^6$ und $R^7$ in Formel (IV) jeweils unabhängig voneinander für Methyl, Ethyl, Propyl, Isopropyl, Butyl, Isobutyl, 2-Ethylhexyl, Cyclohexyl oder Benzyl, oder sie bilden zusammen - unter Einbezug des Stickstoffatoms - einen Ring, insbesondere einen Pyrrolidin-, Piperidin-, Morpholin- oder N-Alkylpiperazin-Ring, wobei dieser Ring gegebenenfalls substituiert ist.

Ein Aldimin **BA1** der Formel (I) ist erhältlich durch eine Kondensationsreaktion unter Abspaltung von Wasser zwischen mindestens einem Amin **B** der Formel (V) und mindestens einem Aldehyd **ALD** der Formel (VI). Der Aldehyd **ALD** der Formel (VI) wird hierbei in Bezug auf die Aminogruppen des Amins **B** stöchiometrisch oder in stöchiometrischem Überschuss eingesetzt.

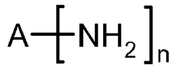

(V)

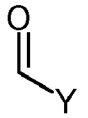

(VI)

In den Formeln (V) und (VI) weisen A, n und Y die bereits erwähnten Bedeutungen auf.

Als Amin **B** der Formel (V) geeignet sind in einer Ausführungsform Polyamine mit mindestens zwei primären Aminogruppen, wie beispielsweise

- aliphatische, cycloaliphatische oder arylaliphatische Diamine, beispielsweise Ethylendiamin, 1,2-Propandiamin, 1,3-Propandiamin, 2-Methyl-1,2-propandiamin, 2,2-Dimethyl-1,3-propandiamin, 1,3-Butandiamin, 1,4-Butandiamin, 1,3-Pentandiamin (DAMP), 1,5-Pentandiamin, 1,5-Diamino-2-methylpentan (MPMD), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin und Methyl-bis-(3-aminopropyl) amin, 1,2-, 1,3- und 1,4-Diaminocyclohexan, Bis-(4-aminocyclohexyl)-methan, Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 2,5(2,6)-Bis-(aminomethyl)-bicyclo[2.2.1]heptan (NBDA), 3(4),8(9)-Bis-(aminomethyl)-tricyclo [$5.2.1.0^{2,6}$]-decan, 1,4-Diamino-2,2,6-trimethylcyclohexan (TMCDA), 1,8-Menthandiamin, 3,9-Bis-(3-aminopropyl)-2,4,8,10-tetraoxaspiro[5.5]undecan sowie 1,3- und 1,4-Xylylendiamin;
- Ethergruppen-haltige aliphatische Diamine, beispielsweise Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxado-decan-3,10-diamin und höhere Oligomere dieser Diamine, Bis-(3-aminopropyl)-polytetrahydrofurane und andere Polytetrahydrofuran-diamine mit Molekulargewichten im Bereich von beispielsweise 350 bis 5200, sowie Polyoxyalkylen-Diamine. Letztere stellen typischerweise Produkte aus der Aminierung von Polyoxyalkylen-Diolen dar und sind beispielsweise erhältlich unter dem Namen Jeffamine® (von Huntsman), unter dem Namen Polyetheramin (von BASF) oder unter dem Namen PC Amine® (von Nitroil). Insbesondere geeignete Polyoxyalkylen-Diamine sind Jeffamine® D-230, Jeffamine® D-400, Jeffamine® D-2000, Jeffamine® D-4000, Jeffamine® XTJ-511, Jeffamine® ED-600, Jeffamine® ED-900, Jeffamine® ED-2003, Jeffamine® XTJ-568, Jeffamine® XTJ-569, Jeffamine® XTJ-523, Jeffamine® XTJ-536, Jeffamine® XTJ-542, Jeffamine® XTJ-559; Polyetheramin D 230, Polyetheramin D 400 und Polyetheramin D 2000, PC Amine® DA 250, PC Amine® DA 400, PC Amine® DA 650 und PC Amine® DA 2000;
- aliphatische, cycloaliphatische oder arylaliphatische Triamine wie 4-Aminomethyl-1,8-octandiamin, 1,3,5-Tris(aminomethyl)-benzol, 1,3,5-Tris-(aminomethyl)-cyclohexan;
- Polyoxyalkylen-Triamine, welche typischerweise Produkte aus der Aminierung von Polyoxyalkylen-Triolen darstellen und beispielsweise erhältlich sind unter dem Handelsnamen Jeffamine® (von Huntsman), unter dem Namen Polyetheramin (von BASF) oder unter dem Namen PC Amine® (von Nitroil), wie zum Beispiel Jeffamine® T-403, Jeffamine® T-5000; Polyetheramin T403, Polyetheramin T5000; und PC Amine® TA 403, PC Amine® TA 5000;
- aromatische Di- und Triamine, wie beispielsweise 1,2-, 1,3- und 1,4-Phenylendiamin, 2,4- und 2,6-Toluylendiamin (TDA), 3,4-Toluylendiamin, 3,5-Dimethylthio-2,4- und -2,6-toluylendiamin, 3,5-Diethyl-2,4- und -2,6-toluylendiamin (DETDA), 2,4,6-Triethyl-1,3-phenylendiamin, 2,4,6-Triisopropyl-1,3-phenylendiamin, 3-Ethyl-5-methyl-2,4-toluylendiamin, 3,5-Diisopropyl-2,4-toluylendiamin, 3,5-Bis-(1-methylpropyl)-2,4-toluylendiamin, 3,5-Bis-(tert.butyl)-2,4-toluylendiamin, 3-Ethyl-5-isopropyl-2,4-toluylendiamin, 5-Isopropyl-2,4-toluylendiamin, 5-(tert.Butyl)-2,4-toluylendiamin, 4,6-Bis-(1-methylpropyl)-1,3-phenylendiamin, 4-Isopropyl-6-(tert.butyl)-1,3-phenylendiamin, 4-Ethyl-6-isopropyl-1,3-phenylendiamin, 4-Ethyl-6-(2-methylpropyl)-1,3-phenylendiamin, 4-Ethyl-6-(1-methylpropyl)-1,3-phenylendiamin, 4-Ethyl-6-(2-methylpropyl)-1,3-phenylendiamin, 4-Isopropyl-6-(1-methylpropyl)-1,3-phenylendiamin, 4-(tert.Butyl)-6-(2-methylpropyl)-1,3-phenylendiamin, 4-Cyclopentyl-6-ethyl-1,3-phenylendiamin, 4-Cyclopentyl-6-isopropyl-1,3-phenylendiamin, 4,6-Dicyclopentyl-1,3-phenylendiamin, 3-Isopropyl-2,6-toluylendiamin, 2-Methylpropyl-(4-chloro-3,5-diaminobenzoat), tert.Butyl-(4-chloro-3,5-diaminobenzoat), 2,6-Diaminopyridin, Melamin, 4,4'-, 2,4'- und 2,2'-Diaminodiphenylmethan (MDA), 3,3'-Dimethyl-4,4'-diaminodiphenylmethan, 3,3'-Dichloro-4,4'-diaminodiphenylmethan (MOCA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 3,3',5,5'-Tetra-(1-methylpropyl)-4,4'-diaminodiphenylmethan, 3,3'-Dimethyl-5,5'-di-tert.butyl-4,4'-diaminodiphenylmethan, 3,3'-Di-tert.butyl-4,4'-diaminodiphenylmethan, 4,4'-Diaminodiphenylsulfon (DDS), 4-Amino-N-(4-aminophenyl)-benzolsulfonamid, 5,5'-Methylendianthranilsäure, Dimethyl-(5,5'-Methylendianthranilat), 1,3-Propylen-bis-(4-aminobenzoat), 1,4-Butylen-bis-(4-aminobenzoat), Polytetramethylenoxid-bis-(4-aminobenzoat) (erhältlich als Versalink® von Air Products) und 1,2-Bis-(2-aminophenylthio)-ethan.

Bevorzugt ist ein Polyamin mit mindestens zwei primärem Aminogruppen ausgewählt aus der Gruppe bestehend aus 1,6-Hexamethylendiamin, MPMD, DAMP, IPDA, TMD, 1,3-Xylylendiamin, 1,3-Bis-(aminomethyl)cyclohexan, Bis-(4-aminocyclohexyl)-methan, Bis-(4-amino-3-methylcyclohexyl)-methan, 3(4),8(9)-Bis-(aminomethyl)-tricyclo [5.2.1.0$^{2,6}$]decan, 1,2-, 1,3- und 1,4-Diaminocyclohexan, 1,4-Diamino-2,2,6-trimethylcyclohexan, 3,6-Dioxaoctan-1,8-diamin, 4,7-Dioxadecan-1,10-diamin, 4-Aminomethyl-1,8-octandiamin, Polyoxyalkylen-Polyamine mit zwei oder drei Aminogruppen, insbesondere die unter dem Handelsnamen Jeffamine® erhältlichen Typen D-230, D-400, D-2000, T-403 und T-5000 von Huntsman und dazu analoge Verbindungen von BASF oder Nitroil, 1,3- und 1,4-Phenylendiamin, 2,4- und 2,6-Toluylendiamin, 4,4'-, 2,4'- und 2,2'-Diaminodiphenylmethan, 3,3'-Dichlor-4,4'-diaminodiphenylmethan und Mischungen der genannten Polyamine.

Als Amin **B** der Formel (V) geeignet sind in einer weiteren Ausführungsform Amine, welche mindestens eine primären Aminogruppe und mindestens eine weitere reaktive Gruppe, welche entweder eine Hydroxylgruppe, eine sekundäre Aminogruppe oder eine Mercaptogruppe darstellt, aufweisen, wie beispielsweise

- Amine mit einer oder zwei primären und einer sekundären Aminogruppe, wie beispielsweise N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-(2-Aminoethyl)piperazin, Diethylentriamin (DETA), Bis-hexamethylentriamin (BHMT), 3-(2-Aminoethyl)aminopropylamin; Di- und Triamine aus der Cyanoethylierung oder Cyanobutylierung und anschliessender Hydrierung von primären Mono- und Diaminen, beispielsweise N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentylamin, 3-Ethylamino-1-pentylamin, 3-Butylamino-1-pentylamin, 3-Hexylamino-1-pentylamin, 3-(2-Ethylhexyl)amino-1-pentylamin, 3-Dodecylamino-1-pentylamin, 3-Cyclohexylamino-1-pentylamin, Dipropylentriamin (DPTA), N3-(3-Aminopentyl)-1,3-pentandiamin, N5-(3-Aminopropyl)-2-methyl-1,5-pentandiamin, N5-(3-Amino-1-ethylpropyl)-2-methyl-1,5-pentandiamin, und Fettdiamine wie N-Cocoalkyl-1,3-propandiamin, N-Oleyl-1,3-propandiamin, N-Soyaalkyl-1,3-propandiamin, N-Talgalkyl-1,3-propandiamin oder N-($C_{16-22}$-Alkyl)-1,3-propandiamin, wie sie beispielsweise unter dem Handelsnamen Duomeen® von Akzo Nobel erhältlich sind; die Produkte aus der Michael-artigen Addition von aliphatischen primären Di- oder Triaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1;
- Hydroxyamine mit einer Hydroxylgruppe und einer primären Aminogruppe, wie beispielsweise 2-Aminoethanol, 2-Methylaminoethanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol; eine primäre Aminogruppe tragende Derivate von Glykolen wie Diethylenglykol, Dipropylenglykol, Dibutylenglykol und höheren Oligomeren und Polymeren dieser Glykole, beispielsweise 2-(2-Aminoethoxy)-ethanol, Triethylenglykolmonoamin, $\alpha$-(2-Hydroxymethylethyl)-$\omega$-(2-aminomethylethoxy)-poly(oxy(methyl-1,2-ethandiyl)); eine Hydroxylgruppe und eine primäre Aminogruppen tragende Derivate von polyalkoxylierten drei- oder höherwertigen Alkoholen; Produkte aus der einfachen Cyanoethylierung und anschliessender Hydrierung von Glykolen, beispielsweise 3-(2-Hydroxyethoxy)-propylamin, 3-(2-(2-Hydroxyethoxy)-ethoxy)-propylamin und 3-(6-Hydroxyhexyloxy)-propylamin;
- Mercaptoamine, wie beispielsweise 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol, 12-Amino-1-dodecanthiol und Aminothiozucker wie 2-Amino-2-deoxy-6-thioglucose;
- Amine mit einer oder mehreren primären und mehr als einer sekundären Aminogruppe, wie N,N'-Bis-(3-aminopropyl) ethylendiamin, Triethylentetramin (TETA), Tetraethylenpentamin (TEPA), Pentaethylenhexamin und höhere Homologe linearer Polyethylenamine, N,N'-Bis-(3-aminopropyl)-ethylendiamin, Produkte aus der mehrfachen Cyanoethylierung oder Cyanobutylierung und anschliessender Hydrierung von primären Di- und Polyaminen mit mehreren primären Aminogruppen, wie N,N'-Bis-(3-aminopropyl)-ethylendiamin, N,N'-Bis-(3-aminopropyl)-1,4-diaminobutan, N,N'-Bis-(3-aminopropyl)-2-methyl-1,5-pentandiamin, N,N'-Bis-(3-amino-1-ethylpropyl)-2-methyl-1,5-pentandiamin sowie Polyethylenimine unterschiedlichen Polymerisationsgrades (Molmassen-Bereich 500 bis 1'000'000 g/mol), wie sie beispielsweise unter dem Handelsnamen Lupasol® von BASF in reiner Form oder als wässrige

Lösungen erhältlich sind, wobei diese Polyethylenimine neben primären und sekundären auch tertiäre Aminogruppen enthalten;

- Hydroxyamine mit mindestens einer Hydroxylgruppe, mindestens einer sekundären und mindestens einer primären Aminogruppe, wie sie beispielsweise aus der Cyanoethylierung oder Cyanobutylierung und anschliessender Hydrierung von einfachen aliphatischen Hydroxyaminen erhältlich sind, beispielsweise N-Hydroxyethyl-1,2-ethandiamin, N-Hydroxypropyl-1,2-ethandiamin, N-Hydroxyethyl-1,3-propandiamin, N3-Hydroxyethyl-1,3-pentandiamin;
- Hydroxyamine mit mehr als einer Hydroxylgruppe und einer oder mehreren primären Aminogruppen, insbesondere Derivate von polyalkoxylierten drei- oder höherwertigen Alkoholen oder von polyalkoxylierten Polyaminen, sowie Aminozucker, beispielsweise Glucosamin oder Galactosamin.

Davon bevorzugt sind Amine, welche ausgewählt sind aus der Gruppe bestehend aus N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT, Fettdiamine wie N-Cocoalkyl-1,3-propandiamin, N-Oleyl-1,3-propandiamin, N-Soyaalkyl-1,3-propandiamin und N-Talgalkyl-1,3-propandiamin, 5-Amino-1-pentanol, 6-Amino-1-hexanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykolmonoamin, 3-(2-Hydroxyethoxy)-propylamin, 3-(2-(2-Hydroxyethoxy)-ethoxy)-propylamin und 3-(6-Hydroxyhexyloxy)-propylamin.

Als Aldehyd ALD der Formel (VI) geeignet sind primäre und sekundäre aliphatische Aldehyde wie Propanal, 2-Methylpropanal, Butanal, 2-Methylbutanal, 2-Ethylbutanal, Pentanal, 2-Methylpentanal, 3-Methylpentanal, 4-Methylpentanal, 2,3-Dimethylpentanal, Hexanal, 2-Ethyl-hexanal, Heptanal, Octanal, Nonanal, Decanal, Undecanal, 2-Methylundecanal, Dodecanal, Methoxyacetaldehyd, Cyclopropancarboxaldehyd, Cyclopentancarboxaldehyd, Cyclohexancarboxaldehyd und Diphenylacetaldehyd.

Als Aldehyd **ALD** der Formel (VI) besonders geeignet sind Aldehyde, welche nicht enolisierbar sind, da diese bei der Umsetzung mit primären Aminen Aldiminogruppen bilden, welche nicht zu Enaminogruppen tautomerisieren können und deshalb besonders gut blockierte Aminogruppen darstellen. Insbesondere tertiäre aliphatische sowie aromatische Aldehyde stellen nicht-enolisierbare Aldehyde dar.

Als Aldehyd **ALD** der Formel (VI) besonders geeignet sind tertiäre aliphatische Aldehyde **ALD1** der Formel (VI a),

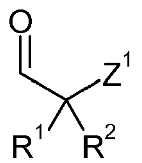

(VI a)

wobei $R^1$, $R^2$ und $Z^1$ die bereits erwähnten Bedeutungen aufweisen.

Geeignete Aldehyd **ALD1** der Formel (VI a) sind beispielsweise Pivalaldehyd (= 2,2-Dimethyl-propanal), 2,2-Dimethyl-butanal, 2,2-Diethyl-butanal, 1-Methyl-cyclopentancarboxaldehyd, 1-Methyl-cyclohexancarboxaldehyd; Ether aus 2-Hydroxy-2-methylpropanal und Alkoholen wie Propanol, Isopropanol, Butanol und 2-Ethylhexanol; Ester aus 2-Formyl-2-methylpropionsäure oder 3-Formyl-3-methylbuttersäure und Alkoholen wie Propanol, Isopropanol, Butanol und 2-Ethylhexanol; Ester aus 2-Hydroxy-2-methylpropanal und Carbonsäuren wie Buttersäure, Isobuttersäure und 2-Ethylhexansäure; sowie die im folgenden als besonders geeignet beschriebenen Ether und Ester von 2,2-disubstituierten 3-Hydroxypropanalen, -butanalen oder analogen höheren Aldehyden, insbesondere von 2,2-Dimethyl-3-hydroxypropanal.

Besonders geeignete Aldehyde **ALD1** der Formel (VI a) sind in einer Ausführungsform Aldehyde **ALD2** der Formel (VI b),

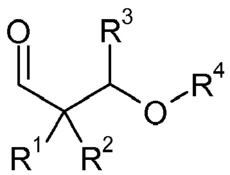

(VI b)

wobei $R^1$, $R^2$, $R^3$ und $R^4$ die bereits erwähnten Bedeutungen aufweisen.

Die Aldehyde **ALD2** der Formel (VI b) stellen Ether von aliphatischen, cycloaliphatischen oder arylaliphatischen

2,2-disubstituierten 3-Hydroxyaldehyden mit Alkoholen oder Phenolen der Formel $R^4$-OH dar, beispielsweise Fettalkoholen oder Phenolen. Geeignete 2,2-disubstituierte 3-Hydroxyaldehyde sind ihrerseits erhältlich aus Aldol-Reaktionen, insbesondere gekreuzten Aldol-Reaktionen, zwischen primären oder sekundären aliphatischen Aldehyden, insbesondere Formaldehyd, und sekundären aliphatischen, sekundären cycloaliphatischen oder sekundären arylaliphatischen Aldehyden, wie beispielsweise Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd (Hydratropaldehyd) oder Diphenylacetaldehyd. Beispiele geeigneter 2,2-disubstituierter 3-Hydroxyaldehyde sind 2,2-Dimethyl-3-hydroxypropanal, 2-Hydroxymethyl-2-methyl-butanal, 2-Hydroxymethyl-2-ethyl-butanal, 2-Hydroxymethyl-2-methyl-pentanal, 2-Hydroxymethyl-2-ethyl-hexanal, 1-Hydroxymethyl-cyclopentancarboxaldehyd, 1-Hydroxymethyl-cyclohexancarboxaldehyd 1-Hydroxymethyl-cyclohex-3-encarboxaldehyd, 2-Hydroxymethyl-2-methyl-3-phenyl-propanal, 3-Hydroxy-2-methyl-2-phenyl-propanal und 3-Hydroxy-2,2-diphenyl-propanal.

Besonders geeignete Aldehyde **ALD2** der Formel (VI b) sind 2,2-Dimethyl-3-phenoxy-propanal, 3-Cyclohexyloxy-2,2-dimethyl-propanal, 2,2-Dimethyl-3-(2-ethylhexyloxy)-propanal, 2,2-Dimethyl-3-lauroxy-propanal und 2,2-Dimethyl-3-stearoxy-propanal.

Besonders geeignete Aldehyde **ALD1** der Formel (VI a) sind in einer weiteren Ausführungsform Aldehyde **ALD3** der Formel (VI c),

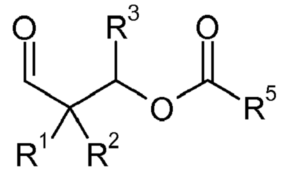

(VI c)

wobei $R^1$, $R^2$, $R^3$ und $R^5$ die bereits erwähnten Bedeutungen aufweisen.

Die Aldehyde **ALD3** der Formel (VIc) stellen Ester der bereits beschriebenen 2,2-disubstituierten 3-Hydroxyaldehyde, wie beispielsweise 2,2-Dimethyl-3-hydroxypropanal, 2-Hydroxymethyl-2-methyl-butanal, 2-Hydroxymethyl-2-ethyl-butanal, 2-Hydroxymethyl-2-methyl-pentanal, 2-Hydroxymethyl-2-ethyl-hexanal, 1-Hydroxymethyl-cyclopentancarboxaldehyd, 1-Hydroxymethyl-cyclohexancarboxaldehyd 1-Hydroxymethyl-cyclohex-3-encarboxaldehyd, 2-Hydroxymethyl-2-methyl-3-phenyl-propanal, 3-Hydroxy-2-methyl-2-phenyl-propanal und 3-Hydroxy-2,2-diphenyl-propanal, mit geeigneten Carbonsäuren dar.

Für diese Umsetzung geeignete Carbonsäuren sind beispielsweise gesättigte aliphatische Carbonsäuren, wie Ameisensäure, Essigsäure, Propionsäure, Buttersäure, Isobuttersäure, Valeriansäure, Capronsäure, 2-Ethylcapronsäure, Oenanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure; einfach ungesättigte aliphatische Carbonsäuren wie Palmitoleinsäure, Ölsäure, Erucasäure; mehrfach ungesättigte aliphatische Carbonsäuren wie Linolsäure, Linolensäure, Elaeostearinsäure, Arachidonsäure; cycloaliphatische Carbonsäuren wie Cyclohexancarbonsäure, arylaliphatische Carbonsäuren wie Phenylessigsäure; aromatische Carbonsäuren wie Benzoesäure, Naphthoesäure, Toluylsäure, Anissäure; Isomere dieser Säuren; Fettsäuregemische aus der technischen Verseifung von natürlichen Ölen und Fetten wie beispielsweise Rapsöl, Sonnenblumenöl, Leinöl, Ölbaumöl, Kokosnussöl, Ölpalmkernöl und Ölpalmöl; sowie Dicarbonsäuremonoalkyl- und -arylester, wie sie aus der einfachen Veresterung von Dicarbonsäuren wie Bernsteinsäure, Glutarsäure, Adipinsäure, Pimelinsäure, Korksäure, Azelainsäure, Sebacinsäure, 1,12-Dodecandisäure, Maleinsäure, Fumarsäure, Hexahydrophthalsäure, Hexahydroisophthalsäure, Hexahydroterephthalsäure, 3,6,9-Trioxaundecandisäure und ähnliche Derivate von Polyethylenglykol, mit Alkoholen wie Methanol, Ethanol, Propanol, Butanol, höheren Homologen und Isomeren dieser Alkohole erhalten werden. Bevorzugt sind Carbonsäuren mit mindestens 7 C-Atomen, insbesondere solche mit mindestens 11 C-Atomen.

Besonders geeignete Aldehyde **ALD1** der Formel (VI a) sind in einer weiteren Ausführungsform Aldehyde **ALD4** der Formel (VI d),

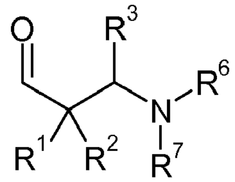

(VI d)

wobei $R^1$, $R^2$, $R^3$, $R^6$ und $R^7$ die bereits erwähnten Bedeutungen aufweisen.

Ein Aldehyd **ALD4** der Formel (VId) ist insbesondere erhältlich als Produkt einer Mannich-Reaktion oder einer der Mannich-Reaktion analogen $\alpha$-Aminoalkylierung, wie sie aus der Fachliteratur bekannt ist; er kann deshalb auch als Mannich-Base bezeichnet werden. Dabei werden ein sekundärer Aldehyd **A1,** ein weiterer Aldehyd **A2** und ein sekundäres aliphatisches Amin **A3** unter Wasserabspaltung zu einem Aldehyd **ALD4** umgesetzt.

Als Aldehyd **A1** geeignet sind beispielsweise Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd und Diphenylacetaldehyd. Bevorzugt ist Isobutyraldehyd.

Als Aldehyd **A2** geeignet sind beispielsweise Formaldehyd, Acetaldehyd, Propionaldehyd, Butyraldehyd, Isobutyraldehyd, Phenylacetaldehyd, Benzaldehyd und substituierte Benzaldehyde, sowie Glyoxylsäureester, insbesondere Glyoxylsäureethylester. Bevorzugt ist Formaldehyd.

Als sekundäres aliphatisches Amin **A3** geeignet sind beispielsweise Dimethylamin, Diethylamin, Dipropylamin, Diisopropylamin, Dibutylamin, Diisobutylamin, Di-sek.-Butylamin, Dihexylamin, Di-(2-ethylhexyl)amin, Dicyclohexylamin, N-Methylbutylamin, N-Ethylbutylamin, N-Methylcyclohexylamin, N-Ethylcyclohexylamin, Di-2-methoxyethylamin, Pyrrolidin, Piperidin, N-Methyl-benzylamin, N-Isopropyl-benzylamin, N-tert.Butyl-benzylamin, Dibenzylamin, Morpholin, 2,6-Dimethylmorpholin, Bis-(3-Dimethylaminopropyl)amin, N-Methyl- oder N-Ethylpiperazin. Davon bevorzugt sind Dimethylamin, Diethylamin, Dipropylamin, Diisopropylamin, Dibutylamin, Diisobutylamin, Bis-(2-Ethylhexyl)amin, N-Methylcyclohexylamin, N-Methyl-benzylamin, N-Isopropyl-benzylamin, N-tert.Butyl-benzylamin, Dibenzylamin, Pyrrolidin, Piperidin, Hexamethylenimin, Morpholin und 2,6-Dimethylmorpholin.

Als Aldehyd **ALD** der Formel (VI) besonders geeignet sind in einer weiteren Ausführungsform Aldehyde ALD5 der Formel (VI e),

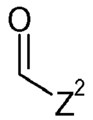

(VI e)

wobei $Z^2$ die bereits genannten Bedeutungen aufweist.

Solche Aldehyde **ALD5** sind beispielsweise aromatische Aldehyde, wie Benzaldehyd, 2- und 3- und 4-Tolualdehyd, 4-Ethyl- und 4-Propyl- und 4-Isopropyl und 4-Butyl-benzaldehyd, 2,4-Dimethylbenzaldehyd, 2,4,5-Trimethylbenzaldehyd, 4-Acetoxybenzaldehyd, 4-Anisaldehyd, 4-Ethoxybenzaldehyd, die isomeren Di- und Trialkoxybenzaldehyde, 2-, 3- und 4-Nitrobenzaldehyd, 2-und 3- und 4-Formylpyridin, 2-Furfuraldehyd, 2-Thiophencarbaldehyd, 1- und 2-Naphthylaldehyd, 3- und 4-Phenyloxy-benzaldehyd, Chinolin-2-carbaldehyd und dessen 3-, 4-, 5-, 6-, 7- und 8-Stellungsisomere, sowie Anthracen-9-carbaldehyd; sowie weiterhin Glyoxal, Glyoxalsäureester wie zum Beispiel Glyoxalsäuremethylester, Zimtaldehyd und substituierte Zimtaldehyde.

Als Aldehyd **ALD** der Formel (VI) bevorzugt sind die genannten Aldehyde **ALD2** der Formel (VI b), **ALD3** der Formel (VI c), **ALD4** der Formel (VI d) und **ALD5** der Formel (VI e). Davon besonders bevorzugt sind die Aldehyde **ALD3** der Formel (VI c), insbesondere diejenigen, bei welchen der Rest $R^5$ 6 bis 30 C-Atome aufweist. Am meisten bevorzugt sind geruchsfreie Aldehyde **ALD3** der Formel (VI c), bei welchen der Rest $R^5$ 11 bis 30 C-Atome aufweist.

Als Aldehyd **ALD** am meisten bevorzugt ist 2,2-Dimethyl-3-lauroyloxypropanal.

Als blockiertes Amin **BA** geeignet ist in einer weiteren Ausführungsform ein Ketimin **BA2** der Formel (VII),

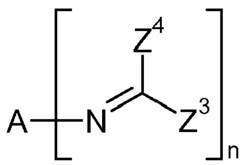

(VII)

wobei

$Z^3$ und $Z^4$ entweder

unabhängig voneinander jeweils für einen einwertigen Kohlenwasserstoffrest mit 1 bis 12 C-Atomen stehen,

oder zusammen für einen zweiwertigen Kohlenwasserstoffrest mit 4 bis 20 C-Atomen, der Teil eines, gegebenenfalls substituierten, carbocyclischen Rings mit 5 bis 8, bevorzugt 6, C-Atomen ist, stehen;

und n und A die bereits erwähnten Bedeutungen aufweisen.

Ein Ketimin **BA2** der Formel (VII) ist erhältlich durch eine Kondensationsreaktion unter Abspaltung von Wasser zwischen mindestens einem Amin **B** der Formel (V) und mindestens einem Keton der Formel (VIII). Das Keton der Formel (VIII) wird hierbei in Bezug auf die Aminogruppen des Amins **B** stöchiometrisch oder in stöchiometrischem Überschuss eingesetzt.

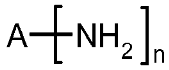

(V)

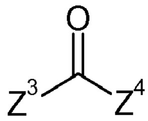

(VIII)

In Formel (VIII) weisen $Z^3$ und $Z^4$ die bereits erwähnten Bedeutungen auf.

Als Keton der Formel (VIII) insbesondere geeignet sind Aceton, Methylethylketon, Methylpropylketon, Methylisopropylketon, Methylisobutylketon, Methylpentylketon, Methylisopentylketon, Diethylketon, Dipropylketon, Diisopropylketon, Dibutylketon, Diisobutylketon, Cyclopentanon, Cyclohexanon und Actetophenon.

Als Amin **B** der Formel (V) geeignet sind die bereits vorgängig erwähnten Amine **B.**

Geeignete kommerzielle Diketimine sind beispielsweise Epikure® Curing Agent 3502 (von Resolution Performance Products) und Desmophen® LS 2965A (von Bayer).

Als blockiertes Amin **BA** geeignet ist in einer weiteren Ausführungsform ein Enamin **BA3,** welches mindestens eine Enaminogruppe der Formel (IX) aufweist, und welches beispielsweise erhältlich ist aus der Umsetzung von mindestens einem sekundären Amin **C** mit mindestens einem aliphatischen oder cycloaliphatischen Aldehyd oder Keton der Formel (X) unter Abspaltung von Wasser.

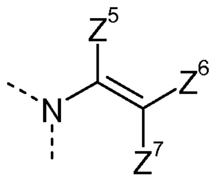

(IX)

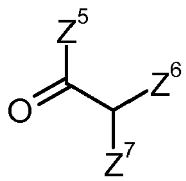

(X)

In den Formeln (IX) und (X) stehen

$Z^5$ und $Z^6$ entweder

unabhängig voneinander jeweils für ein Wasserstoffatom oder für einen einwertigen Kohlenwasserstoffrest mit 1 bis 12 C-Atomen,

oder zusammen für einen zweiwertigen Kohlenwasserstoffrest mit 3 bis 20 C-Atomen, der Teil eines, gegebenenfalls substituierten, carbocyclischen Rings mit 5 bis 8, bevorzugt 6, C-Atomen ist;

und

$Z^7$ steht für ein Wasserstoffatom oder für einen einwertigen Kohlenwasserstoffrest mit 1 bis 12 C-Atomen.

Als sekundäres Amin C geeignet sind in einer Ausführungsform Amine mit mindestens zwei sekundären Aminogruppen, wie beispielsweise Piperazin, Tetramethylpiperazin, Homopiperazin, 1,3-Di(piperidin-4-yl)-propan, N,N'-Dimethylhexamethylendiamin und Homologe mit höheren Alkyl- oder Cycloalkylgruppen anstelle der Methylgruppen, N,N'-Dimethyl-diethylentriamin und N,N'-Dimethyl-dipropylentriamin.

Als sekundäres Amin C geeignet sind in einer weiteren Ausführungsform Amine mit einer Hydroxylgruppe und einer sekundären Aminogruppe, wie beispielsweise N-(2-Hydroxyethyl)-piperazin, 4-Hydroxy-piperidin sowie monoalkoxylierte primäre Monoamine, das heisst Umsetzungsprodukte von primären Monoaminen wie beispielsweise Methylamin, Ethylamin, Propylamin, Butylamin, Hexylamin, 2-Ethylhexylamin, Benzylamin und Fettamine wie Laurylamin oder Stearylamin, mit Epoxiden wie Ethylenoxid, Propylenoxid oder Butylenoxid im stöchiometrischen Verhältnis 1:1, beispielsweise N-Methylethanolamin, N-Ethyl-ethanolamin, N-Butyl-ethanolamin und N-Butyl-isopropanolamin.

Als sekundäres Amin **C** geeignet sind in einer weiteren Ausführungsform Amine mit einer Mercaptogruppe und einer sekundären Aminogruppe, wie beispielsweise N-(2-Mercaptoethyl)-piperazin, 4-Mercaptopiperidin und 2-Mercaptoethyl-butylamin.

Als Aldehyd oder Keton der Formel (X) geeignet sind Aldehyde, welche in α-Stellung zur Carbonylgruppe mindestens ein Wasserstoffatom aufweisen und damit enolisierbar sind, wie beispielsweise Propanal, 2-Methylpropanal, Butanal, 2-Methylbutanal, 2-Ethylbutanal, Pentanal, 2-Methylpentanal, 3-Methylpentanal, 4-Methylpentanal, 2,3-Dimethylpentanal, Hexanal, 2-Ethyl-hexanal, Heptanal, Octanal, Nonanal, Decanal, Undecanal, 2-Methyl-undecanal, Dodecanal, Methoxyacetaldehyd, Cyclopropancarboxaldehyd, Cyclopentancarboxaldehyd, Cyclohexancarboxaldehyd und Diphenylacetaldehyd; sowie Ketone, welche in α-Stellung zur Carbonylgruppe mindestens ein Wasserstoffatom aufweisen und damit enolisierbar sind, wie Aceton, Methylethylketon, Methylpropylketon, Methylisopropylketon, Methylisobutylketon, Methylpentylketon, Methylisopentylketon, Diethylketon, Dipropylketon, Diisopropylketon, Dibutylketon, Diisobutylketon, sowie insbesondere cyclische Ketone, wie beispielsweise Cyclopentanon und Cyclohexanon.

Als blockiertes Amin **BA** geeignet ist in einer weiteren Ausführungsform ein Oxazolidin **BA4** der Formel (XI),

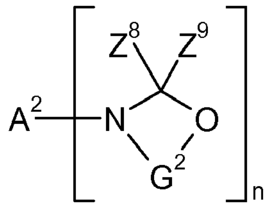

(XI)

wobei

$A^2$ für den Rest eines Amins nach Entfernung von n sekundären Aminogruppen steht;

$G^2$ für einen, gegebenenfalls substituierten, $C_2$- oder $C_3$-Alkylenrest steht;

$Z^8$ und $Z^9$ unabhängig voneinander jeweils für ein Wasserstoffatom oder für einen einwertigen Kohlenwasserstoffrest mit 1 bis 12 C-Atomen stehen;

und n die bereits erwähnten Bedeutungen aufweist.

Als "Oxazolidinogruppe" werden im vorliegenden Dokument sowohl Tetrahydrooxazol-Gruppen (5-Ring) als auch Tetrahydrooxazin-Gruppen (6-Ring) bezeichnet.

Ein Oxazolidin **BA4** der Formel (XI) ist beispielsweise erhältlich aus der Umsetzung von mindestens einem Amin **D** der Formel (XII) mit mindestens einem Aldehyd oder Keton der Formel (XIII) unter Abspaltung von Wasser.

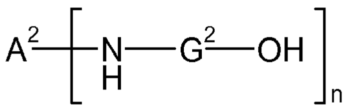

(XII)

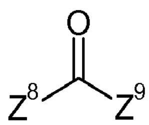

(XIII)

In den Formeln (XII) und (XIII) weisen $A^2$, $G^2$, n, $Z^8$ und $Z^9$ die bereits erwähnten Bedeutungen auf.

Als Amin **D** der Formel (XII) geeignet sind aliphatische Hydroxyamine mit einer sekundären Aminogruppe, wie beispielsweise Diethanolamin, Dipropanolamin und Diisopropanolamin.

Als Amin **D** bevorzugt ist Diethanolamin, welches mit einem Aldehyd oder Keton der Formel (XIII) zu einem Oxazolidin der Formel (XI a) umgesetzt werden kann.

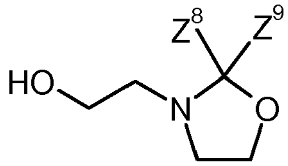

(XI a)

In Formel (XI a) weisen $Z^8$ und $Z^9$ die bereits erwähnten Bedeutungen auf.

Ein Oxazolidin der Formel (XI a) kann mit einem Polyisocyanat so umgesetzt werden, dass die Hydroxylgruppen mit Isocyanatgruppen reagieren. Auf diese Weise sind Polyoxazolidine erhältlich.

Als Aldehyd oder Keton der Formel (XIII) geeignet sind die bereits erwähnten Aldehyde oder Ketone der Formel (XI), sowie weiterhin beispielsweise Formaldehyd, Benzaldehyd und substituierte Benzaldehyde. Bevorzugt ist 2-Methylpropanal.

Geeignete kommerzielle Oxazolidine sind beispielsweise Härter OZ (von Bayer), Zoldine® RD-20, Zoldine® MS-52 und Zoldine® MS Plus (von Angus Chemical), sowie Incozol® 2, Incozol® 3, Incozol® LV, Incozol® 4, Incozol® HP und Incozol® NC (von Industrial Copolymers).

Als blockiertes Amin **BA** geeignet sind auch Verbindungen mit zwei oder mehr der beschriebenen blockierten Aminogruppen, welche untereinander verschieden sind.

Ein geeignetes kommerzielles blockiertes Amin mit einer Aldimino- und einer Oxazolidinogruppe ist beispielsweise Zoldine® RD-4 (von Angus Chemical).

In den Formeln (I), (Ia), (Ib), (VII) und (XI) steht der Index n bevorzugt für 1 oder 2 oder 3, wobei blockierte Amine **BA** der Formeln (I), (Ia), (Ib), (VII) und (XI) mit dem Index n = 1 neben der blockierten Aminogruppe bevorzugt mindestens eine Reaktivgruppe in Form einer Hydroxyl-, einer sekundären Aminogruppe oder einer Mercaptogruppe am Aminteil aufweisen.

Die blockierten Aminogruppen in Form von Aldiminogruppen, Ketiminogruppen, Enaminogruppen und/oder Oxazolidinogruppen des blockierten Amins **BA** reagieren unter Ausschluss von Feuchtigkeit mit Isocyanatgruppen nicht oder nur äusserst langsam.

Das blockierte Amin **BA** ist in der härtbaren Zusammensetzung vorteilhaft in einer derartigen Menge vorhanden, dass das Verhältnis zwischen der Anzahl der blockierten Aminogruppen und gegebenenfalls vorhandener Hydroxyl-, Mercapto- und sekundären Aminogruppen und der Anzahl der Isocyanatgruppen 0.1 bis 1.1, bevorzugt 0.2 bis 0.9, insbesondere 0.5 bis 0.9, beträgt. Falls blockierte Aminogruppen in Form von Oxazolidinogruppen vorhanden sind, werden diese doppelt gezählt, da diese nach erfolgter Hydrolyse gegenüber Isocyanatgruppen formal difunktionell sind.

Als blockiertes Amin **BA** in der beschriebenen härtbaren Zusammensetzung bevorzugt sind die Aldimine **BA1** der Formel (I), die Ketimine **BA2** der Formel (VII), die Enamine **BA3** enthaltend Enaminogruppen der Formel (IX) und die Oxazolidine **BA4** der Formel (XI).

Als blockiertes Amin **BA** besonders bevorzugt sind die Aldimine **BA1,** die Ketimine **BA2** und die Oxazolidine **BA4**. Insbesondere bevorzugt sind die Aldimine **BA1** der Formel (I a) und der Formel (I b) und die Oxazolidine **BA4.**

Als blockiertes Amin **BA** am meisten bevorzugt sind die Aldimine **BA1** der Formel (Ia), in welchen $Z^1$ für einen Rest der Formel (II) oder (III) oder (IV), insbesondere für einen Rest der Formel (III), steht.

Die beschriebene härtbare Zusammensetzung umfasst weiterhin mindestens ein Hydrazid **HY** einer Carbon- oder Sulfonsäure, welches einen Schmelzpunkt von mindestens 100 °C, insbesondere von mindestens 150 °C, aufweist. Bevorzugt liegt der Schmelzpunkt des Hydrazids **HY** unterhalb von 300 °C.

Im vorliegenden Dokument umfasst der Begriff "Carbonsäure" auch die Kohlensäure.

Ein Hydrazid **HY** einer Carbonsäure weist insbesondere die Formel (XIVa) oder (XIVb) auf, während ein Hydrazid **HY** einer Sulfonsäure die Formel (XV) aufweist.

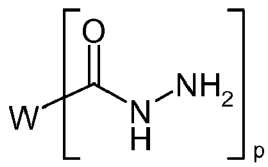

(XIV a)

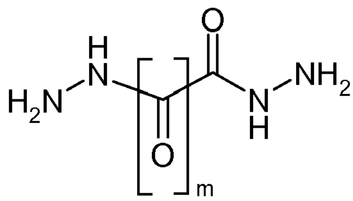

(XIV b)

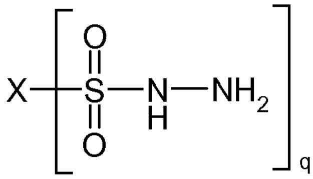

(XV)

In den Formeln (XIV a), (XIV b) und (XV) stehen

W für den p-wertigen Rest eines Hydrazids **HY** einer Carbonsäure, welches einen Schmelzpunkt von mindestens 100 °C, insbesondere von mindestens 150 °C, aufweist, nach Entfernung von p Carbonsäurehydrazid-gruppen;

X für den q-wertigen Rest eines Hydrazids **HY** einer Sulfonsäure, welches einen Schmelzpunkt von mindestens 100 °C, insbesondere von mindestens 150 °C, aufweist, nach Entfernung von q Sulfonsäurehydrazidgruppen;
m für null oder 1,
p für 1 oder 2 oder 3 oder 4, und
q für 1 oder 2 oder 3 oder 4.
Bevorzugt steht p für 2.

Das Hydrazid **HY** ist beispielsweise erhältlich durch die Kondensation geeigneter Carbon- oder Sulfonsäuren mit Hydrazin oder Hydrazinhydrat. Geeignete Carbon- oder Sulfonsäurehydrazide mit einem Schmelzpunkt von über 100 °C sind beispielsweise die Folgenden:

Hydrazide von aliphatischen und arylaliphatischen Carbonsäuren wie Laurinsäure, Palmitinsäure, Stearinsäure, Cyanessigsäure, 2,4-Dichlorphenoxyessigsäure, 4-Nitrophenoxyessigsäure, 1-Naphthylessigsäure; Hydrazide von aromatischen und heteroaromatischen Carbonsäuren wie Benzoesäure, 2-, 3-und 4-Chlorbenzoesäure, 2-, 3-und 4-Brombenzoesäure, 2- und 4-Toluylsäure, 2-, 3- und 4-Nitrobenzoesäure, Salicylsäure, 4-tert.Butylbenzoesäure, 4-Methoxybenzoesäure, 4-Ethoxybenzoesäure, 4-Trifluorbenzoesäure, 4-Dimethylaminobenzoesäure, die isomeren Dichlorbenzoesäuren, Dimethoxybenzoesäuren und Trimethoxybenzoesäuren, Terephthalsäuremonomethylester, 1-Naphthylcarbonsäure, 3-Hydroxy-2-naphthylcarbonsäure, 4-Biphenylcarbonsäure, Nicotinsäure, Isonicotinsäure, 2-Thiophencarbonsäure, 4-Imidazolcarbonsäure, 3-Pyrazolcarbonsäure; Monohydrazine und Dihydrazide von Dicarbonsäuren wie Oxalsäure, Bernsteinsäure, Glutarsäure, Adipinsäure, Pimelinsäure, Korksäure, Azelainsäure, Sebacinsäure, Dodecandicarbonsäure, Isophthalsäure, Terephthalsäure; Mono-, Di- und Trihydrazide von Tricarbonsäuren wie Benzoltricarbonsäure; das Dihydrazid von Kohlensäure (Carbodihydrazid); Hydrazide von Sulfonsäuren wie Benzolsulfonsäure, 4-Toluolsulfonsäure; Dihydrazide von Disulfonsäuren wie Propylendisulfonsäure, Butylendisulfonsäure, o-, m- und p-Benzoldisulfonsäure und Naphthalindisulfonsäure.

Als Hydrazid **HY** weiterhin geeignet sind cyclische Hydrazide von Dicarbonsäuren, welche N-Aminoimide darstellen, beispielsweise N-Aminophthalimid, N-Aminosuccinimid, 2-Amino-3a,4,7,7a-tetrahydro-isoindol-1,3-dion, 4-Amino-4-aza-tricyclo[5.2.1.0$^{2,6}$]dec-8-en-3,5-dion, 4-Amino-4-aza-10-oxa-tricyclo[5.2.1.0$^{2,6}$]dec-8-en-3,5-dion.

Als Hydrazid **HY** bevorzugt sind Carbonsäurehydrazide.

Als Hydrazid **HY** besonders bevorzugt sind Carbonsäuredihydrazide.

Bevorzugt ist das Carbonsäuredihydrazid **HY** ausgewählt aus der Gruppe bestehend aus Carbodihydrazid, Oxalsäuredihydrazid, Bernsteinsäuredihydrazid, Adipinsäuredihydrazid, Korksäuredihydrazid, Azelainsäuredihydrazid, Sebacinsäuredihydrazid, Dodecansäuredihydrazid und Isophtalsäuredihydrazid.

Als Hydrazid **HY** am meisten bevorzugt ist Adipinsäuredihydrazid.

Hydrazidgruppen können grundsätzlich sowohl mit Isocyanatgruppen als auch mit Aldehyd- und Ketogruppen reagieren. Bei der Reaktion von Carbonsäurehydrazid-Gruppen mit Isocyanatgruppen werden Acylsemicarbazidgruppen der Formel (XVI a) gebildet, während bei der Reaktion mit Aldehyd- oder Ketogruppen unter Wasserabspaltung Gruppen der Formel (XVII a) gebildet werden. Sulfonsäurehydrazid-Gruppen bilden dazu analoge Gruppen der Formel (XVI b) und (XVII b).

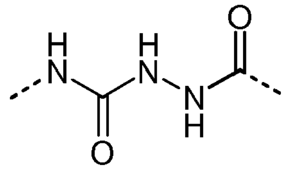

(XVI a)

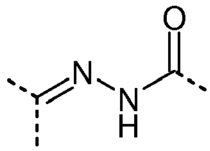

(XVII a)

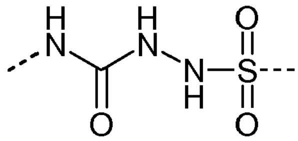

(XVI b)

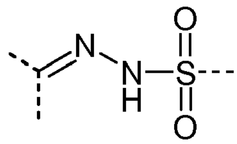

(XVII b)

Das Hydrazid **HY** ist bei Raumtemperatur fest und weist einen Schmelzpunkt von mindestens 100 °C, bevorzugt von mindestens 150 °C, auf. Seine Reaktivität gegenüber Isocyanatgruppen und seine Reaktivität gegenüber Aldehyd- und Ketogruppen ist bei Temperaturen deutlich unter seinem Schmelzpunkt stark eingeschränkt. Bei Raumtemperatur reagiert es weder mit Isocyanatgruppen noch mit Aldehyd- oder Ketogruppen in nennenswertem Ausmass. Das Hydrazid **HY** ist mit Isocyanatgruppen bei Raumtemperatur oder leicht erhöhter Temperatur lagerstabil. Erst bei stärker erhöhter Temperatur, insbesondere bei 80 °C und höher, finden die erwähnten Reaktionen in nennenswertem Ausmass statt.

Das Hydrazid **HY** ist in der härtbaren Zusammensetzung in einer Menge von 0.3 bis 1.1 Equivalent Hydrazidgruppen pro Equivalent Aldehyd- und Ketogruppen, mittels welchem das Amin **BA** blockiert ist, vorhanden. Bevorzugt ist das Hydrazid **HY** in der härtbaren Zusammensetzung in einer Menge von 0.5 bis 1.0 Equivalent, besonders bevorzugt 0.75 bis 1.0 Equivalent, Hydrazidgruppen pro Equivalent Aldehyd- oder Ketogruppen, mittels welchem das Amin **BA** blockiert ist, vorhanden.

Ist das Hydrazid **HY** in einer geringeren Menge als 0.3 Equivalent Hydrazidgruppen pro Equivalent Aldehyd- und Ketogruppen, mittels welchem das Amin **BA** blockiert ist, vorhanden, so wird die Aldehyd- oder Keton-Ausgasung aufgrund der Stöchiometrie nur in einem schwachen Ausmass reduziert. Ist das Hydrazid **HY** in einer höheren Menge als 1.1 Equivalent Hydrazidgruppen pro Equivalent Aldehyd- und Ketogruppen, mittels welchem das Amin **BA** blockiert ist, vorhanden, so wird keine zusätzliche Reduktion der Aldehyd- oder Keton-Ausgasung mehr erreicht. Um eine maximale Reduktion der Aldehyd- oder Keton-Ausgasung zu erzielen, ist das Hydrazid **HY** vorteilhaft in einer stöchiometrischen oder nahezu stöchiometrischen Menge bezogen auf die Aldehyd- und Ketogruppen, mittels welchem das Amin **BA** blockiert ist, vorhanden.

Die härtbare Zusammensetzung kann zusätzlich zu mindestens einem Polyisocyanat **P,** mindestens einem mittels Aldehyd oder Keton blockierten Amin **BA** und mindestens einem Hydrazid **HY** einer Carbon- oder Sulfonsäure weitere Hilfs- und Zusatzstoffe enthalten.

Die härtbare Zusammensetzung kann in der Form einer einkomponentigen Zusammensetzung oder in der Form einer zweikomponentigen Zusammensetzung vorliegen.

Als "einkomponentige Zusammensetzung" wird im vorliegenden Dokument eine härtbare Zusammensetzung bezeichnet, deren Bestandteile in vermischter Form im gleichen Gebinde gelagert werden, und welche bei Raumtemperatur über einen längeren Zeitraum lagerstabil ist, sich also in ihren Anwendungs- oder Gebrauchseigenschaften durch die Lagerung nicht oder nur unwesentlich verändert, und welche nach der Applikation durch die Einwirkung von Feuchtigkeit aushärtet.

Als "zweikomponentige Zusammensetzung" wird im vorliegenden Dokument eine härtbare Zusammensetzung bezeichnet, deren Bestandteile in zwei verschiedenen Komponenten vorliegen, die in voneinander getrennten Gebinden gelagert werden und jeweils für sich lagerstabil sind. Erst kurz vor oder während der Applikation der Zusammensetzung werden die beiden Komponenten miteinander vermischt, worauf die vermischte Zusammensetzung aushärtet, wobei die Aushärtung unter Umständen erst durch die Einwirkung von Feuchtigkeit abläuft oder vervollständigt wird.

Einkomponentige Zusammensetzungen weisen den Vorteil auf, dass sie ohne Mischvorgang applizierbar sind, während zweikomponentige Zusammensetzungen den Vorteil aufweisen, dass sie rascher aushärten und als Bestandteile Substanzen enthalten können, welche zusammen mit Isocyanaten nicht lagerfähig sind.

In einer Ausführungsform liegt die härtbare Zusammensetzung in der Form einer einkomponentigen Zusammensetzung vor.

Als Polyisocyanat **P** ist in der einkomponentigen Zusammensetzung ein Polyurethanpolymer **PUP,** oder eine Mischung aus einem Polyurethanpolymer **PUP** und einem Polyisocyanat **PI**, wie sie vorgängig beschrieben wurden, bevorzugt.

Als blockiertes Amin **BA** ist in der einkomponentigen Zusammensetzung ein Aldimin **BA1** der Formel (I a), insbesondere mit einem Rest $Z^1$ der Formel (II) oder (III) oder (IV), oder ein Aldimin **BA1** der Formel (I b), oder ein Oxazolidin **BA4** der Formel (XI) bevorzugt.

Für den Fall, dass das blockierte Amin **BA** am Aminteil gegenüber Isocyanaten reaktive Gruppen wie Hydroxylgruppen, Mercaptogruppen oder nicht blockierte Aminogruppen aufweist, beträgt deren Anzahl pro Molekül bevorzugt eins. Blockierte Amine **BA** mit gegenüber Isocyanaten reaktive Gruppen reagieren beim Vermischen mit dem Polyisocyanat **P,** indem sie Addukte bilden.

Bevorzugt enthält das blockierte Amin **BA** jedoch keine gegenüber Isocyanaten reaktive Gruppen wie Hydroxylgruppen, Mercaptogruppen oder nicht blockierte Aminogruppen.

Als Hilfs- und Zusatzstoffe für eine einkomponentige Zusammensetzung sind beispielsweise die folgenden Substanzen geeignet:

- Weichmacher, beispielsweise Carbonsäureester wie Phthalate, beispielsweise Dioctylphthalat, Diisononylphthalat oder Diisodecylphthalat, Adipate, beispielsweise Dioctyladipat, Azelate und Sebacate, organische Phosphor- und Sulfonsäureester oder Polybutene;
- nicht-reaktive thermoplastische Polymere, wie beispielsweise Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere Polyethylene (PE), Polypropylene (PP), Polyisobutylene, Ethylenvinylacetat-Copolymere (EVA) und ataktische Poly-$\alpha$-Olefine (APAO);
- Lösemittel;
- anorganische und organische Füllstoffe, zum Beispiel gemahlene oder gefällte Calciumcarbonate, welche gegebenenfalls mit Fettsäuren, insbesondere Stearaten, beschichtet sind, Baryt ($BaSO_4$, auch Schwerspat genannt), Quarzmehle, calcinierte Kaoline, Aluminiumoxide, Aluminiumhydroxide, Kieselsäuren, insbesondere hochdisperse Kieselsäuren aus Pyrolyseprozessen, Russe, insbesondere industriell hergestellte Russe (im Folgenden als "Russ" bezeichnet), PVC-Pulver oder Hohlkugeln;
- Fasern, beispielsweise aus Polyethylen;
- Pigmente, beispielsweise Titandioxid oder Eisenoxide;
- Katalysatoren, welche die Hydrolyse der blockierten Aminogruppen beschleunigen, insbesondere Säuren oder zu Säuren hydrolysierbare Verbindungen, beispielsweise organische Carbonsäuren wie Benzoesäure, Salicylsäure oder 2-Nitrobenzoesäure, organische Carbonsäureanhydride wie Phthalsäureanhydrid, Hexahydrophthalsäureanhydrid und Hexahydromethylphthalsäureanhydrid, Silylester von organischen Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester;
- Katalysatoren, welche die Reaktion der Isocyanatgruppen mit Wasser beschleunigen, insbesondere Metallverbindungen, beispielsweise Organozinnverbindungen wie Dibutylzinndiacetat, Dibutylzinndilaurat, Dibutylzinndichlorid, Dibutylzinndiacetylacetonat und Dioctylzinndilaurat, Bismutverbindungen wie Bismuttrioctoat und Bismuttris(neodecanoat), und tertiäre Aminogruppen enthaltende Verbindungen wie 2,2'-Dimorpholinodiethylether und 1,4-Diazabicyclo[2.2.2]octan;
- Rheologie-Modifizierer wie beispielsweise Verdickungsmittel oder Thixotropiermittel, zum Beispiel Harnstoffverbindungen, Polyamidwachse, Bentonite oder pyrogene Kieselsäuren;
- Reaktivverdünner und Vernetzer, beispielsweise monomere Diisocyanate sowie Oligomere und Derivate dieser Diisocyanate, Addukte monomerer Diisocyanate mit kurzkettigen Polyolen;
- Trocknungsmittel, wie beispielsweise Molekularsiebe, Calciumoxid, hochreaktive Isocyanate wie p-Tosylisocyanat, Orthoameisensäureester, Tetraalkoxysilane wie Tetraethoxysilan; Trialkoxysilane, wie Methyltrimethoxysilan, Isooctyltrimethoxysilan und Vinyltrimethoxysilan;

- Haftvermittler, insbesondere Organoalkoxysilane, im Folgenden auch "Silane" genannt, beispielsweise Epoxysilane, wie 3-Glycidoxypropyltrimethoxysilan und 3-Glycidoxypropyltriethoxysilan, (Meth)acrylsilane wie 3-Methacryloxypropyltrimethoxysilan, Isocyanatosilane, wie 3-Isocyanatopropyltrimethoxysilan, S-(Alkylcarbonyl)-mercaptosilane, wie S-Octanoyl-3-mercaptopropyltriethoxysilan, und Aldiminosilane, wie N-Benzyliden-3-aminopropyltrimethoxysilan, sowie oligomere Formen dieser Silane;
- Stabilisatoren gegen Wärme, Licht- und UV-Strahlung;
- flammhemmende Substanzen;
- oberflächenaktive Substanzen wie beispielsweise Netzmittel, Verlaufsmittel, Entlüftungsmittel oder Entschäumer;
- Biozide wie beispielsweise Algizide, Fungizide oder das Pilzwachstum hemmende Substanzen.

Es ist vorteilhaft, darauf zu achten, dass solche Zusätze die Lagerstabilität der Zusammensetzung nicht beeinträchtigen. Das heisst, dass diese Zusätze während der Lagerung die zur Vernetzung führenden Reaktionen wie Hydrolyse der blockierten Aminogruppen oder Vernetzung der Isocyanatgruppen nicht in signifikantem Ausmass auslösen dürfen. Insbesondere bedeutet dies, dass all diese Zusätze kein oder höchstens Spuren von Wasser enthalten sollten. Es kann deshalb sinnvoll sein, gewisse Zusätze vor dem Einmischen in die Zusammensetzung chemisch oder physikalisch zu trocknen.

Bevorzugt enthält die einkomponentige Zusammensetzung mindestens einen Katalysator. Insbesondere enthält die Zusammensetzung als Katalysator eine Carbonsäure wie Benzoesäure oder Salicylsäure und/oder eine Zinnverbindung und/oder eine Bismutverbindung. Es kann vorteilhaft sein, wenn unterschiedliche Katalysatoren, bzw. unterschiedliche Katalysatorenarten wie beispielsweise eine Säure und eine Metallverbindung, miteinander gemischt werden.

Weiterhin bevorzugt enthält die einkomponentige Zusammensetzung mindestens einen weiteren Hilfs- und Zusatzstoff, insbesondere ausgewählt aus der Gruppe umfassend Weichmacher, Füllstoffe und Verdickungsmittel.

Die beschriebene einkomponentige Zusammensetzung wird vorzugsweise unter Ausschluss von Feuchtigkeit hergestellt und bei Raumtemperatur oder leicht erhöhter Temperatur aufbewahrt. In einer geeigneten klimadichten Verpackung oder Anordnung, wie beispielsweise einem Fass, einem Beutel oder einer Kartusche, verfügt sie über eine gute Lagerstabilität. Mit den Begriffen "lagerstabil" und "Lagerstabilität" in Zusammenhang mit einer härtbaren Zusammensetzung wird im vorliegenden Dokument der Sachverhalt bezeichnet, dass die Viskosität der Zusammensetzung bei gegebener Applikationstemperatur und bei geeigneter Lagerung in der betrachteten Zeitspanne nicht oder höchstens so stark ansteigt, dass die Zusammensetzung auf die vorgesehene Weise verwendbar bleibt.

In einer weiteren Ausführungsform liegt die härtbare Zusammensetzung in der Form einer zweikomponentigen Zusammensetzung vor. Die zweikomponentige Zusammensetzung besteht aus einer Komponente K1 und einer Komponente **K2,** welche getrennt voneinander gelagert und erst kurz vor der Applikation miteinander vermischt werden.

In einer Ausführungsform der zweikomponentigen Zusammensetzung sind das Polyisocyanat **P** und das blockierte Amin **BA** Teil der Komponente **K1**, während die Komponente **K2** gegenüber Isocyanatgruppen reaktive Verbindungen, insbesondere Wasser und/oder Polyole und/oder Polyamine und/oder Aminoalkohole und/oder Polythiole enthält, und das Hydrazid **HY** entweder in der Komponente **K1** oder in der Komponente **K2** oder in beiden Komponenten enthalten ist.

In einer anderen Ausführungsform der zweikomponentigen Zusammensetzung ist das Polyisocyanat P Teil der Komponente **K1**, während die Komponente **K2** das blockierte Amin **BA** sowie gegenüber Isocyanatgruppen reaktive Verbindungen, insbesondere Wasser und/oder Polyole und/oder Polyamine und/oder Aminoalkohole und/oder Polythiole, enthält, und das Hydrazid **HY** entweder in der Komponente **K1** oder in der Komponente **K2** oder in beiden Komponenten enthalten ist.

Bevorzugt enthält die Komponente **K2** mindestens ein blockiertes Amin **BA** und Wasser.

Bevorzugt ist das Hydrazid **HY** ein Bestandteil der Komponente **K2.**

Als Polyisocyanat **P** ist in der zweikomponentigen Zusammensetzung ein Polyisocyanat **PI**, oder eine Mischung aus einem Polyurethanpolymer **PUP** und einem Polyisocyanat **PI**, wie sie vorgängig beschrieben wurden, bevorzugt.

Als Polyole in der Komponente **K2** geeignet sind dieselben handelsüblichen Polyole, wie sie bereits als geeignet zur Herstellung eines Polyurethanpolymers **PUP** erwähnt wurden, sowie diejenigen niedrigmolekularen zwei- oder mehrwertigen Alkohole, wie sie vorgängig als geeignet zum Mitverwenden bei der Herstellung eines Polyurethanpolymers **PUP** erwähnt wurden. Als Polyamine in der Komponente **K2** geeignet sind handelsübliche aliphatische oder aromatische Polyamine mit primären und/ oder sekundären Aminogruppen, wie sie üblicherweise in zweikomponentigen Polyurethanzusammensetzungen eingesetzt werden, wie beispielsweise 1,5-Diamino-2-methylpentan (MPMD), 1,3-Xylylendiamin (MXDA), N,N'-Dibutylethylendiamin, 3,5-Diethyl-2,4(6)-diaminotoluol (DETDA), 3,5-Dimethylthio-2,4(6)-diaminotoluol (beispielsweise erhältlich als Ethacure® 300 von Albemarle) sowie primäre und sekundäre Polyoxyalkylen-Diamine, wie sie zum Beispiel unter dem Namen Jeffamine® (von Huntsman) erhältlich sind. Als Aminoalkohole in der Komponente **K2** geeignet sind Verbindungen, welche mindestens eine primäre oder sekundäre Aminogruppe und mindestens eine Hydroxylgruppe aufweisen, wie beispielsweise 2-Aminoethanol, 2-Methylaminoethanol, 1-Amino-2-propanol und Diethanolamin. Als Polythiole in der Komponente **K2** geeignet sind beispielsweise die unter dem Markennamen Thiokol® bekannten flüssigen Mercapto-terminierten Polymere, sowie Polyester von Thiocarbonsäuren.

Zudem können beide Komponenten weitere Hilfs- und Zusatzstoffe, wie sie bereits vorgängig für eine einkomponentige Zusammensetzung erwähnt wurden, enthalten. Im Falle der Komponente **K2** sind jedoch zusätzlich noch weitere Hilfs- und Zusatzstoffe möglich. Insbesondere sind dies solche Hilfs- und Zusatzstoffe, welche zusammen mit aromatischen Isocyanatgruppen nicht oder nur kurzzeitig lagerfähig sind. Insbesondere sind dies Katalysatoren wie:

Verbindungen von Zink, Mangan, Eisen, Chrom, Cobalt, Kupfer, Nickel, Molybdän, Blei, Cadmium, Quecksilber, Antimon, Vanadium, Titan, Zirconium oder Kalium, wie Zink(II)-acetat, Zink(II)-2-ethylhexanoat, Zink(II)-laurat, Zink(II)-oleat, Zink(II)-naphthenat, Zink(II)-acetylacetonat, Zink(II)-salicylat, Mangan(II)-2-ethylhexanoat, Eisen(III)-2-ethylhexanoat, Eisen(III)-acetylacetonat, Chrom(III)-2-ethylhexanoat, Cobalt(II)-naphthenat, Cobalt(II)-2-ethylhexanoat, Kupfer(II)-2-ethylhexanoat, Nickel(II)-naphthenat, Phenylquecksilber-neodecanoat, Blei(II)-acetat, Blei(II)-2-ethylhexanoat, Blei(II)-neodecanoat, Blei(II)-acetylacetonat, Aluminiumlactat, Aluminiumoleat, Aluminium(III)-acetylacetonat, Diisopropoxytitan-bis-(ethylacetoacetat), Dibutoxytitan-bis-(ethylacetoacetat), Dibutoxytitan-bis-(acetylacetonat), Kalium-acetat, Kaliumoctoat; tertiäre Amine wie Triethylamin, Tributylamin, N-Ethyl-diisopropylamin, N,N,N',N'-Tetramethyl-ethylendiamin, Pentamethyl-diethylentriamin und höhere Homologe davon, N,N,N',N'-Tetramethyl-propylendiamin, Pentamethyl-dipropylentriamin und höhere Homologe davon, N,N,N',N'-Tetramethyl-1,3-butandiamin, N,N,N',N'-Tetramethyl-1,6-hexandiamin, Bis-(dimethylamino)-methan, N,N-Dimethylbenzylamin, N,N-Dimethylcyclohexylamin, N-Methyl-dicyclohexylamin, N,N-Dimethyl-hexadecylamin, Bis-(N,N-diethylaminoethyl)-adipat, N,N-Dimethyl-2-phenylethylamin, Tris-(3-dimethylaminopropyl)-amin, 1,4-Diazabicyclo[2.2.2]octan (DABCO), 1,8-Diazabicyclo[5.4.0]undec-7-en (DBU), 1,5-Diazabicyclo[4.3.0]non-5-en (DBN), N-Methylmorpholin, N-Ethylmorpholin, N-Cocomorpholin, N,N'-Dimethylpiperazin, N-Methyl-N'-dimethylaminoethyl-piperazin, Bis-(dimethylaminoethyl)-piperazin, 1,3,5-Tris-(dimethylaminopropyl)-hexahydrotriazin, Bis-(2-dimethylaminoethyl)-ether; stickstoffaromatische Verbindungen wie 4-Dimethylamino-pyridin, N-Methylimidazol, N-Vinylimidazol oder 1,2-Dimethylimidazol; Amidine und Guanidine wie 1,1,3,3-Tetramethylguanidin; tertiäre Amine enthaltend aktive Wasserstoffatome, wie Triethanolamin, Triisopropanolamin, N-Methyldiethanolamin, N,N-Dimethylethanolamin, 3-(Dimethylamino)-propyl-diisopropanolamin, Bis-(3-(dimethylamino)-propyl)-isopropanolamin, Bis-(3-dimethylaminopropyl)amin, 3-(Dimethylamino)-propylharnstoff, Mannich-Basen wie 2,4,6-Tris-(dimethylaminomethyl)-phenol oder 2,4,6-Tris-(3-(dimethylamino)-propylaminomethyl)-phenol, N-Hydroxypropylimidazol, N-(3-Aminopropyl)-imidazol, sowie Alkoxylierungs- und Polyalkoxylierungsprodukte dieser Verbindungen, beispielsweise Dimethylaminoethoxyethanol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen, wie Umsetzungsprodukte aus tertiären Aminen und Carbonsäuren oder Phenolen, beispielsweise aus 1,4-Diazabicyclo[2.2.2]octan oder DBU und Ameisensäure oder Essigsäure; sowie Kombinationen der genannten Verbindungen, insbesondere von Metallverbindungen und tertiären Aminen.

Bevorzugt enthält die zweikomponentige Zusammensetzung mindestens einen Katalysator. Insbesondere enthält die Zusammensetzung als Katalysator eine Carbonsäure wie Benzoesäure oder Salicylsäure und/oder eine Zinnverbindung und/oder eine Bismutverbindung. Es kann vorteilhaft sein, wenn unterschiedliche Katalysatoren, bzw. unterschiedliche Arten von Katalysatoren, wie beispielsweise eine Säure und eine Metallverbindung, miteinander gemischt werden.

Weiterhin bevorzugt enthält die zweikomponentige Zusammensetzung mindestens einen weiteren Hilfs- und Zusatzstoff, insbesondere ausgewählt aus der Gruppe umfassend Weichmacher, Vernetzer, Füllstoffe und Verdickungsmittel.

Bevorzugt enthält die Komponente K2 keine Isocyanatgruppen.

Die Herstellung der beschriebenen Komponenten **K1** und **K2** erfolgt getrennt voneinander, mindestens für die Komponente **K1** unter Ausschluss von Feuchtigkeit. Die beiden Komponenten **K1** und **K2** sind getrennt voneinander bei Raumtemperatur oder leicht erhöhter Temperatur lagerstabil, d.h. sie können jede in einer geeigneten Verpackung oder Anordnung, wie beispielsweise in einem Fass, einem Hobbock, einem Beutel, einem Eimer oder einer Kartusche, vor ihrer Anwendung während mehreren Monaten bis zu einem Jahr und länger aufbewahrt werden, ohne dass sie sich in ihren jeweiligen Eigenschaften in einem für ihren Gebrauch relevanten Ausmass verändern.

Das Mischungsverhältnis zwischen den Komponenten **K1** und **K2** wird bevorzugt so gewählt, dass die gegenüber Isocyanatgruppen reaktiven Gruppen der Komponenten **K1** und **K2** in einem geeigneten Verhältnis zu den Isocyanatgruppen der Komponente **K1** stehen. In der beschriebenen zweikomponentigen Zusammensetzung sind vor der Aushärtung geeigneterweise 0.1 bis 1.1, bevorzugt 0.5 bis 0.95, besonders bevorzugt 0.6 bis 0.95 Equivalent der Summe der gegenüber Isocyanaten reaktiven Gruppen pro Equivalent Isocyanatgruppen vorhanden, wobei die blockierten Aminogruppen zu den gegenüber Isocyanaten reaktiven Gruppen gezählt werden, und Wasser nicht zu den gegenüber Isocyanaten reaktiven Gruppen gerechnet wird. Überschüssige Isocyanatgruppen reagieren insbesondere direkt mit

Wasser, beispielsweise mit Luftfeuchtigkeit.

Die beschriebene härtbare Zusammensetzung wird geeigneterweise bei einer Temperatur unterhalb von 40 °C appliziert. Dabei ist dem Fachmann klar, dass die Applikationstemperatur der Zusammensetzung vorteilhaft deutlich unterhalb dem Schmelzpunkt des vorhandenen Hydrazids **HY** liegt, um eine vorzeitige Reaktion des Hydrazids **HY** mit den vorhandenen Isocyanatgruppen zu vermeiden. Die Applikation erfolgt, indem die Zusammensetzung mit einer Festkörperoberfläche kontaktiert wird, gegebenenfalls mittels eines geeigneten Hilfsmittels. Liegt die Zusammensetzung in pastöser Form vor, kann die Applikation beispielsweise aus handelsüblichen Kartuschen, welche für kleinere Anwendungen bevorzugt manuell betrieben werden, erfolgen. Eine Applikation mittels Druckluft aus einer Kartusche oder aus einem Fass oder Hobbock mittels einer Förderpumpe oder eines Extruders, gegebenenfalls mittels eines Applikations-Roboters, ist ebenfalls möglich. Derartige Applikationsarten werden insbesondere in Anwendungen der industriellen Fertigung oder bei grossen Applikationen bevorzugt. Im Fall einer zweikomponentigen Zusammensetzung werden die Komponenten **K1** und **K2** vor oder während der Applikation mittels eines geeigneten Verfahrens miteinander vermischt. Das Mischen kann kontinuierlich oder batchweise erfolgen. Falls das Vermischen vor der Applikation erfolgt, muss darauf geachtet werden, dass zwischen dem Vermischen der Komponenten **K1** und **K2** und der Applikation nicht zuviel Zeit vergeht, da es dadurch zu Störungen, wie beispielsweise einem verlangsamten oder unvollständigen Aufbau der Haftung zur Festkörperoberfläche, kommen kann.

Während und nach der Applikation beginnt die Aushärtung der Zusammensetzung. Die härtbare Zusammensetzung reagiert mit Wasser bzw. Feuchtigkeit und wird dadurch vernetzt. Ist genügend Wasser vorhanden, um einen Grossteil oder alle Isocyanatgruppen umzusetzen, entsteht eine ausgehärtete Zusammensetzung, die gute mechanische Eigenschaften aufweist. Die Zusammensetzung kann deshalb als "feuchtigkeitshärtend" bezeichnet werden. Geeigneterweise findet dieser - auch als "Aushärtung" oder "Vernetzung" bezeichnete - Vorgang bei Raumtemperatur oder leicht erhöhter Temperatur, insbesondere unterhalb von 40 °C, statt.

Das blockierte Amin **BA** beginnt mit vorhandenen Isocyanatgruppen unter Freisetzung von Aldehyden und/ oder Ketonen zu reagieren, sobald es mit Wasser in Kontakt kommt. Vorhandene Aldiminogruppen können bei Zutritt von Feuchtigkeit, beziehungsweise Wasser, über Zwischenstufen zu primären Aminogruppen hydrolysieren, wobei der entsprechende Aldehyd freigesetzt wird. Da diese Hydrolysereaktion reversibel ist und das chemische Gleichgewicht deutlich auf der Aldiminseite liegt, ist davon auszugehen, dass in Abwesenheit von gegenüber Aminen reaktiven Gruppen nur ein Teil der Aldiminogruppen hydrolysiert. In Gegenwart von Isocyanatgruppen verschiebt sich das Hydrolysegleichgewicht, da die hydrolysierenden Aldiminogruppen mit den Isocyanatgruppen irreversibel zu Harnstoffgruppen reagieren. Die Reaktion der hydrolysierenden Aldiminogruppen mit Isocyanatgruppen muss dabei nicht notwendigerweise über freie Aminogruppen erfolgen. Selbstverständlich sind auch Reaktionen mit Zwischenstufen der Hydrolysereaktion möglich. Beispielsweise ist es denkbar, dass eine hydrolysierende Aldiminogruppe in der Form einer Halbaminalgruppe direkt mit einer Isocyanatgruppe reagiert. Vorhandene Ketiminogruppen können bei Zutritt von Feuchtigkeit über Zwischenstufen zu primären Aminogruppen hydrolysieren, wobei das entsprechende Keton freigesetzt wird. In Gegenwart von Isocyanatgruppen werden dabei ebenfalls Harnstoffgruppen gebildet. Auch vorhandene Enaminogruppen reagieren bei Zutritt von Feuchtigkeit unter Freisetzung des entsprechenden Aldehyds oder Ketons mit Isocyanatgruppen zu Harnstoffgruppen. Bei der Hydrolyse von vorhandenen Oxazolidinogruppen entstehen unter Freisetzung eines Aldehyds oder Ketons pro Oxazolidinogruppe formal eine sekundäre Aminogruppe und eine Hydroxylgruppe. In Gegenwart von Isocyanatgruppen reagieren die sekundären Aminogruppen zu Harnstoffgruppen und die Hydroxylgruppen zu Urethangruppen. Oxazolidinogruppen sind bei ihrer Hydrolyse gegenüber Isocyanatgruppen also formal difunktionell. Das zur Aushärtung der Zusammensetzung benötigte Wasser ist entweder in der applizierten Zusammensetzung bereits vorhanden, beispielsweise - für den Fall einer zweikomponentigen Zusammensetzung - indem es ein Bestandteil der Komponente **K2** war, oder indem es der Zusammensetzung kurz vor oder während der Applikation zugesetzt wurde, oder das Wasser diffundiert in der Form von Luftfeuchtigkeit in die Zusammensetzung. Im letztgenannten Fall erfolgt die Reaktion des blockierten Amins **BA** mit den Isocyanatgruppen von aussen nach innen, parallel zum Eindringen der Luftfeuchtigkeit in die Zusammensetzung. Für den Fall einer zweikomponentigen Zusammensetzung, welche Hydroxyl-, Mercapto- oder primäre oder sekundäre Aminogruppen aufweist, reagieren diese ebenfalls mit vorhandenen Isocyanatgruppen. Überschüssige Isocyanatgruppen reagieren insbesondere direkt mit Wasser. Als Ergebnis dieser Reaktionen vernetzt die vermischte Zusammensetzung und härtet schliesslich zu einem festen Material aus.

Die Aushärtung erfolgt im Allgemeinen blasenfrei, insbesondere auch bei hoher Aushärtungsgeschwindigkeit.

Die Aushärtungsgeschwindigkeit lässt sich über die Art und Menge eines oder mehrerer gegebenenfalls vorhandener Katalysatoren und / oder über die Luftfeuchtigkeit bzw. gegebenenfalls über die Menge des über eine Komponente **K2** eingebrachten Wassers beeinflussen.

Geeigneterweise erfolgt die Aushärtung bei Raumtemperatur oder leicht erhöhter Temperatur, insbesondere unterhalb von 40 °C. Das Hydrazid **HY** reagiert bei diesen Bedingungen aufgrund seines hohen Schmelzpunktes nicht in nennenswertem Ausmass mit Isocyanaten, sondern verbleibt weitgehend unreagiert in der ausgehärteten Zusammensetzung.

Für den Fall, dass aus dem blockierten Amin **BA** bei der Aushärtung der Zusammensetzung relativ schwerflüchtige Aldehyde oder Ketone freigesetzt werden, insbesondere die geruchsfreien Aldehyde **ALD2** der Formel (VI b), bzw. **ALD3** der Formel (VI c), mit den Resten $R^4$ bzw. $R^5$ mit 11 oder mehr C-Atomen, so verbleiben diese bei den genannten Bedingungen nach der Aushärtung ebenfalls weitgehend in der Zusammensetzung.

Bei Erwärmung der ausgehärteten Zusammensetzung, insbesondere auf 80 °C und höher, beginnt das Hydrazid **HY** mit den vorhandenen Aldehyden und/oder Ketonen zu reagieren, insbesondere mit den bei der Hydrolyse des blockierten Amins **BA** freigesetzten Aldehyden und/oder Ketonen. Die bei der Reaktion zwischen Hydraziden und Aldehyden bzw. Ketonen entstehenden Kondensationsprodukte, auch Hydrazone genannt, werden im vorliegenden Dokument als "Aldazide" bzw. als "Ketazide" bezeichnet.

Das Erwärmen der ausgehärteten Zusammensetzung kann beispielsweise im Verlauf des normalen Gebrauchs der ausgehärteten Zusammensetzung erfolgen, indem beispielsweise ein Automobil, dessen Frontscheibe mit Hilfe der beschriebenen Zusammensetzung verklebt ist, durch Sonneneinstrahlung stark aufgeheizt wird.

Das Erwärmen der ausgehärteten Zusammensetzung kann aber auch gezielt herbeigeführt werden, indem beispielsweise das erwähnte Automobil - oder ein Anbauteil davon enthaltend die verklebte Frontscheibe - gezielt auf beispielsweise 80 °C aufgeheizt wird, so dass vorhandene Aldehyde und/oder Ketone mit dem Hydrazid **HY** reagieren. Somit kann das gezielte Aufheizen ein Prozessschritt beim Herstellverfahren darstellen.

Die beschriebene härtbare Zusammensetzung unterscheidet sich bei geeigneter Applikations- und Aushärtungstemperatur in ihrem Aushärtungsverhalten und den mechanischen Eigenschaften kaum von entsprechenden Zusammensetzungen ohne Hydrazid **HY**. Die Isocyanatgruppen reagieren, wie bereits beschrieben, mit den hydrolysierenden blockierten Aminogruppen des blockierten Amins **BA** sowie mit gegebenenfalls vorhandenen Hydroxylgruppen, Mercaptogruppen und nicht blockierten Aminogruppen, und schliesslich mit Wasser, während das Hydrazid **HY** aufgrund seines hohen Schmelzpunktes nicht in nennenswertem Ausmass an der Aushärtungsreaktion teilnimmt. Im ausgehärteten Zustand zeigt die beschriebene härtbare Zusammensetzung aber eine deutlich reduzierte Ausgasung, insbesondere bei erhöhter Temperatur. Durch die Ausgasung von Aldehyden oder Ketonen verursachte Emissionen können sich störend auswirken, insbesondere in Innenräumen, beispielsweise indem sie einen unangenehmen Geruch verursachen, oder indem sie zu Reizungen der Haut oder der Atemwege führen, oder indem sie Beschläge auf Oberflächen bilden. In Fahrzeugen können solche Beschläge zur Eintrübung der Scheiben führen, was auch als "Fogging" bezeichnet wird (und wie in DIN 75201 beschrieben bestimmt werden kann). Insbesondere in der Automobilindustrie bestehen oft Grenzwerte für die aus den im Fahrzeug-Innenraum eingesetzten Materialien, beispielsweise Klebstoffen, durch Ausgasung entweichenden flüchtigen Anteile.

Bei Erwärmung der ausgehärteten Zusammensetzung, insbesondere auf 80 °C und höher, beginnt das Hydrazid mit den bei der Hydrolyse des blockierten Amins **BA** freigesetzten Aldehyden und/oder Ketonen zu reagieren. Die dabei gebildeten Aldazide bzw. Ketazide sind schwerflüchtig genug, um auch bei höheren Temperaturen nicht auszugasen.

Somit kann das beschriebene Hydrazid **HY** zur Reduktion der Aldehyd- oder Keton-Ausgasung aus der ausgehärteten Zusammensetzung verwendet werden.

Die bevorzugten Aldimine **BA1** der Formel (I a), bei welchen $Z^1$ für einen Rest der Formel (II) oder (III) oder (IV) steht, setzen bei der Aushärtungsreaktion relativ schwerflüchtige Aldehyde **ALD2** der Formel (VI b) oder **ALD3** der Formel (VI c) oder **ALD4** der Formel (VI d) frei. Insbesondere die besonders bevorzugten geruchsfreien Aldehyde **ALD2** oder **ALD3,** bei welchen $R^4$ in Formel (VI b) oder $R^5$ in Formel (VI c) 11 bis 30 C-Atome aufweisen, verbleiben aufgrund ihrer geringen Flüchtigkeit bei Raumtemperatur über sehr lange Zeit in der ausgehärteten Zusammensetzung. In Abwesenheit eines Hydrazids **HY** entweichen solche Aldehyde bei erhöhter Temperatur, insbesondere bei Temperaturen von 80 °C und höher, aber allmählich aus der ausgehärteten Zusammensetzung. In Gegenwart eines Hydrazids **HY** hingegen können solche Aldehyde bei Erwärmung der Zusammensetzung, auch wenn diese sehr lange Zeit nach der Aushärtung erfolgt, noch wirkungsvoll gebunden werden. Die Ausgasung solcher Aldehyde aus den beschriebenen Zusammensetzungen ist durch die äusserst geringe Flüchtigkeit der entsprechenden Aldazide auch bei deutlich erhöhten Temperaturen, beispielsweise bei 100 °C oder mehr, sehr stark vermindert; dies geht mit einem deutlich verminderten Gewichtsverlust der Zusammensetzung einher. Ein in Bezug auf Aldehyd- und Keotgruppen nahezu stöchiometrischer Gehalt an Hydrazidgruppen bewirkt eine besonders deutliche Verminderung des Gewichtsverlustes der ausgehärteten Zusammensetzung. Das Hydrazid HY weist, wie bereits erwähnt, einen Schmelzpunkt von mindestens 100 °C, insbesondere von mindestens 150 °C, auf. Ein zu tiefer Schmelzpunkt des eingesetzten Hydrazids kann eine vorzeitige Reaktion mit Isocyanatgruppen zur Folge haben, was zu einer unvollständigen Aushärtung der Zusammensetzung und/oder einer ungenügenden Lagerstabilität des Hydrazids zusammen mit Isocyanatgruppen führen kann. Die Lagerstabilität einer einkomponentigen Zusammensetzung steht mit dem Schmelzpunkt des vorhandenen Hydrazids in einem Zusammenhang. Während eine einkomponentige Zusammensetzung enthaltend Benzhydrazid mit einem Schmelzpunkt von ca. 112 °C bei Raumtemperatur oder höchstens leicht erhöhter Temperatur lagerstabil ist, weist eine entsprechende Zusammensetzung enthaltend Adipinsäuredihydrazid mit einem Schmelzpunkt von ca. 180 °C auch bei einer Lagertemperatur von 60 °C eine ausgezeichnete Lagerstabilität auf. Eine einkomponentige Zusammensetzung enthaltend beispielsweise Essigsäurehydrazid mit einem Schmelzpunkt von ca. 63 °C, d.h. unter 100 °C, ist hingegen bereits bei Raumtemperatur nicht lagerstabil.

Eine härtbare Zusammensetzung, welche, neben mindestens einem Polyisocyanat **P,** als Hydrazid **HY** das insbesondere bevorzugte Adipinsäuredihydrazid in der bereits genannten Menge, und als blockiertes Amin **BA** ein Aldimin **BA1** der Formel (I a) mit $Z^1$ der Formel (II) oder (III) mit Resten $R^4$ oder $R^5$ mit 11 bis 30 C-Atomen umfasst, weist besonders vorteilhafte Eigenschaften auf. Sie weist eine sehr gute Lagerstabilität auf, härtet bei Raumtemperatur oder leicht erhöhter Temperatur schnell und geruchsfrei aus und verfügt im ausgehärteten Zustand über gute mechanische Eigenschaften. Bei erhöhter Temperatur zeigt sie eine überraschend geringe Aldehyd-Ausgasung; ihr Gewichtsverlust bei 80 °C ist, verglichen mit dem einer entsprechenden Zusammensetzung ohne Hydrazid **HY,** sehr niedrig.

Weiterhin wurde festgestellt, dass die beschriebene härtbare Zusammensetzung, insbesondere wenn sie aromatische Isocyanatgruppen oder Umsetzungsprodukte davon enthält, eine deutlich verringerte Neigung zur Vergilbung zeigt im Vergleich mit entsprechenden Zusammensetzungen ohne Hydrazid **HY.** Die geringere Vergilbungsneigung zeigt sich insbesondere bei der ausgehärteten Zusammensetzung, insbesondere dann, wenn diese während einiger Stunden oder Tage erhitzt wird, beispielsweise auf Temperaturen von 80 bis 130 °C.

Somit kann ein beschriebenes Hydrazid **HY** oder ein Aldazid daraus zur Reduktion der Vergilbung einer ausgehärteten Polyurethanzusammensetzung verwendet werden.

Bevorzugte Anwendungen der beschriebenen härtbaren Zusammensetzung, insbesondere der bevorzugten Ausführungsformen davon, sind Klebstoffe, Dichtstoffe, Vergussmassen, Beschichtungen, Bodenbeläge, Anstriche, Lacke, Primer oder Schäume.

Mit ihrem geringen Ausgasungsverhalten sind die beschriebenen härtbaren Zusammensetzungen besonders geeignet als einkomponentige oder zweikomponentige elastische Klebstoffe im Fahrzeugbau, insbesondere als Scheibenklebstoffe, wo bezüglich Ausgasung, beziehungsweise Fogging, hohe Anforderungen an die eingesetzten Materialien gestellt werden.

Mit ihrer geringeren Vergilbungsneigung sind die beschriebenen härtbaren Zusammensetzungen weiterhin besonders geeignet als einkomponentige oder zweikomponentige Bodenbeläge.

Ein weiterer Aspekt der vorliegenden Erfindung betrifft ein Verfahren zum Verkleben von einem Substrat **S1** mit einem Substrat **S2,** welches die Schritte umfasst:

i) Applikation einer vorgängig beschriebenen härtbaren Zusammensetzung auf ein Substrat **S1** bei einer Temperatur unterhalb von 40 °C;
ii) Kontaktieren der applizierten Zusammensetzung mit einem Substrat **S2** innerhalb der Offenzeit der Zusammensetzung;
iii) Aushärten der applizierten Zusammensetzung bei einer Temperatur unterhalb von 40 °C;
oder
i') Applikation einer vorgängig beschriebenen härtbaren Zusammensetzung auf ein Substrat **S1** und auf ein Substrat **S2** bei einer Temperatur unterhalb von 40 °C;
ii') Kontaktieren der applizierten Zusammensetzung miteinander innerhalb der Offenzeit der Zusammensetzung;
iii') Aushärten der applizierten Zusammensetzung bei einer Temperatur unterhalb von 40 °C;

wobei das Substrat **S2** aus dem gleichen oder einem unterschiedlichen Material wie das Substrat **S1** besteht.

Ein weiterer Aspekt der vorliegenden Erfindung betrifft ein Verfahren zum Abdichten. Dieses umfasst die Schritte:

i'') Applikation einer vorgängig beschriebenen härtbaren Zusammensetzung bei einer Temperatur unterhalb von 40 °C zwischen ein Substrat **S1** und ein Substrat **S2,** so dass die Zusammensetzung mit dem Substrat **S1** und dem Substrat S2 in Kontakt steht;
ii'') Aushärten der applizierten Zusammensetzung bei einer Temperatur unterhalb von 40 °C;

wobei das Substrat **S2** aus dem gleichen oder einem unterschiedlichen Material wie das Substrat **S1** besteht.
Üblicherweise wird die Zusammensetzung in eine sogenannte Fuge eingepresst.

Ein weiterer Aspekt der vorliegenden Erfindung betrifft ein Verfahren zum Beschichten eines Substrates **S1**. Dieses umfasst die Schritte:

i''') Applikation einer vorgängig beschriebenen härtbaren Zusammensetzung bei einer Temperatur unterhalb von 40 °C auf ein Substrat **S1** innerhalb der Offenzeit der Zusammensetzung;
ii''') Aushärten der applizierten Zusammensetzung bei einer Temperatur unterhalb von 40 °C.

In diesen drei Verfahren sind geeignete Substrate **S1** und/oder **S2** beispielsweise anorganische Substrate wie Glas, Glaskeramik, Beton, Mörtel, Backstein, Ziegel, Gips und Natursteine wie Granit oder Marmor; Metalle oder Legierungen wie Aluminium, Stahl, Buntmetalle, verzinkte Metalle; organische Substrate wie Leder, Stoffe, Papier, Holz, mit

Harz gebundene Holzwerkstoffe, Harz-Texil-Compositewerkstoffe, Kunststoffe wie Polyvinylchlorid (Hart- und Weich-PVC), Acrylonitril-Butadien-Styrol-Copolymere (ABS), SMC (Sheet Moulding Composites), Polycarbonat (PC), Polyamid (PA), Polyester, PMMA, Polyester, Epoxidharze, Polyurethane (PUR), Polyoxymethylen (POM), Polyolefine (PO), insbesondere mittels Plasma, Corona oder Flammen oberflächenbehandeltes Polyethylen (PE) oder Polypropylen (PP), Ethylen/Propylen-Copolymere (EPM) und Ethylen/Propylen-Dien-Terpolymere (EPDM); beschichtete Substrate wie pulverbeschichtete Metalle oder Legierungen; sowie Farben und Lacke, insbesondere Automobillacke.

Die Substrate können bei Bedarf vor dem Applizieren der Zusammensetzung vorbehandelt werden. Derartige Vorbehandlungen umfassen insbesondere physikalische und/oder chemische Reinigungsverfahren, beispielsweise Schleifen, Sandstrahlen, Bürsten oder dergleichen, oder Behandeln mit Reinigern oder Lösemitteln oder das Aufbringen eines Haftvermittlers, einer Haftvermittlerlösung oder eines Primers.

Im Fall einer zweikomponentigen Zusammensetzung werden die beiden Komponenten **K1** und **K2** kurz vor der Applikation miteinander vermischt.

Aus den beschriebenen Verfahren zum Verkleben, Abdichten bzw. Beschichten - beziehungsweise aus der Verwendung einer der beschriebenen härtbaren Zusammensetzungen als Klebstoff, Dichtstoff, Vergussmasse, Beschichtung, Bodenbelag, Anstrich, Lack, Primer oder Schaum - entsteht ein Artikel.

Dieser Artikel ist insbesondere ein Bauwerk, insbesondere ein Bauwerk des Hoch- oder Tiefbaus, oder ein industrielles Gut oder ein Konsumgut, insbesondere ein Fenster, eine Haushaltmaschine, oder ein Transportmittel, insbesondere ein Fahrzeug zu Wasser oder zu Lande, bevorzugt ein Automobil, ein Bus, ein Lastkraftwagen, ein Zug oder ein Schiff, oder ein Anbauteil eines Transportmittels, oder ein Artikel der Möbel-, Textil- oder Verpackungsindustrie.

Ein weiterer Gegenstand der vorliegenden Erfindung ist eine ausgehärtete Zusammensetzung **AZ**, erhalten durch die Aushärtung der vorgängig beschriebenen härtbaren Zusammensetzung bei einer Temperatur unterhalb von 40 °C mittels Einwirkung von Wasser, beispielsweise in der Form von Luftfeuchtigkeit, und anschliessender Aufwärmung der so erhaltenen ausgehärteten Zusammensetzung auf eine Temperatur von 80 °C oder höher,
wobei diese ausgehärtete Zusammensetzung mindestens eine Verbindung der Formel (XVIII a) oder (XVIII b) oder (XIX) enthält,

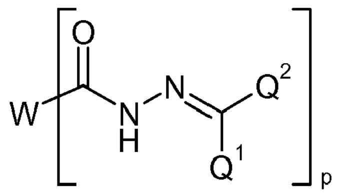

(XVIII a)

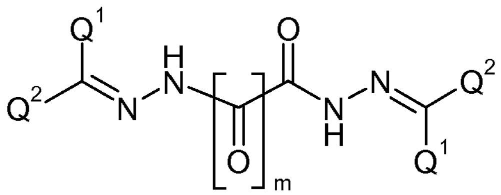

(XVIII b)

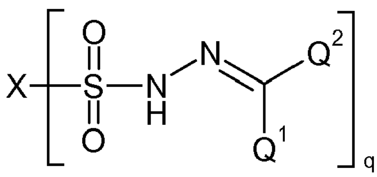

(XIX)

wobei $Q^1$ für ein Wasserstoffatom oder für $Z^3$ oder für $Z^5$ oder für $Z^8$ steht,
wobei
falls $Q^1$ für ein Wasserstoffatom steht, $Q^2$ für Y steht,
oder
falls $Q^1$ für $Z^3$ steht, $Q^2$ für $Z^4$ steht,
oder
falls $Q^1$ für $Z^5$ steht, $Q^2$ für

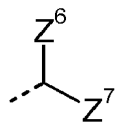

steht,
oder
falls $Q^1$ für $Z^8$ steht, $Q^2$ für $Z^9$ steht.
W, X, m, p, q, Y, $Z^3$, $Z^4$, $Z^5$, $Z^5$, $Z^6$, $Z^7$, $Z^8$ und $Z^9$ weisen die bereits beschriebenen Bedeutungen auf.

Die Verbindungen der Formel (XVIII a), (XVIII b) und (XIX) stellen Aldazide oder Ketazide von Carbon- bzw. Sulfonsäurehydraziden dar. Die Verbindungen der Formel (XVIII a) und (XVIII b) werden gebildet durch die Reaktion eines Hydrazids **HY** in Form eines Carbonsäurehydrazids mit einem Aldehyd oder Keton, während die Verbindung der Formel (XIX) gebildet wird durch die Reaktion eines Hydrazids **HY** in Form eines Sulfonsäurehydrazids mit einem Aldehyd oder Keton, und wobei das Aldehyd oder Keton bei der Aushärtung der Zusammensetzung aus dem blockierten Amin **BA** freigesetzt wurde.

Bevorzugt enthält die ausgehärtete Zusammensetzung **AZ** eine Verbindung der Formel (XVIII a) oder (XVIII b). Besonders bevorzugt wird die Verbindung der Formel (XVIII a) oder (XVIII b) aus der Reaktion eines Carbonsäuredihydrazids mit einem Aldehyd oder Keton gebildet, wobei das Carbonsäuredihydrazid bevorzugt ausgewählt ist aus der Gruppe bestehend aus Carbodihydrazid, Oxalsäuredihydrazid, Bernsteinsäuredihydrazid, Adipinsäuredihydrazid, Korksäuredihydrazid, Azelainsäuredihydrazid, Sebacinsäuredihydrazid, Dodecansäuredihydrazid und Isophtalsäuredihydrazid. Davon am meisten bevorzugt ist Adipinsäuredihydrazid.

Bevorzugt steht $Q^1$ für ein Wasserstoffatom und $Q^2$ für $-C(R^1)(R^2)(Z^1)$, wobei $Z^1$ insbesondere für einen Rest der Formel (II) oder (III) oder (IV) steht.

Ein weiterer Gegenstand der vorliegenden Erfindung ist ein Aldazid der Formel (XX a) oder (XX b),

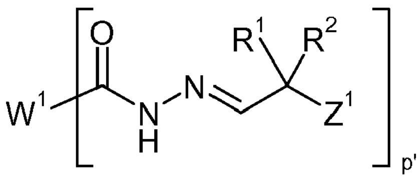

(XX a)

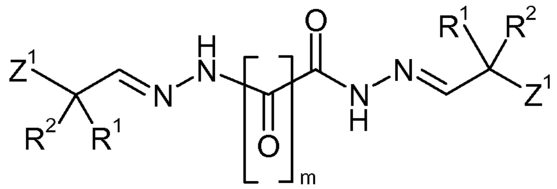

(XX b)

wobei
$W^1$ für den p'-wertigen Rest eines Hydrazids HY einer Carbonsäure, welches einen Schmelzpunkt von mindestens 100 °C, insbesondere von mindestens 150 °C, aufweist, nach Entfernung von p' Carbonsäurehydrazid-gruppen steht;
p' für 2 oder 3 oder 4 steht; und
m, $R^1$, $R^2$ und $Z^1$ die bereits erwähnten Bedeutungen aufweisen.
Bevorzugt steht p' für 2.
Bevorzugt steht $Z^1$ für einen Rest der Formel (II) oder (III) oder (IV).

## Beispiele

Beschreibung der Messmethoden

**Infrarotspektren** wurden auf einem FT-IR Gerät 1600 von Perkin-Elmer gemessen, feste Substanzen als KBr-Presslinge im Direktstrahl, Flüssigkeiten als unverdünnte Filme und ausgehärtete Polymere als angepresste Filme auf einer horizontalen ATR-Messeinheit mit ZnSe-Kristall, die Absorptionsbanden sind in Wellenzahlen ($cm^{-1}$) angegeben

(Messfenster: 4000-650 $cm^{-1}$), der Zusatz sh weist auf eine als Schulter erscheinende Bande hin, der Zusatz br auf eine breite Bande.

**$^1$H-NMR-Spektren** wurden auf einem Spektrometer des Typs Bruker DPX-300 bei 300.13 MHz gemessen; die chemischen Verschiebungen $\delta$ sind angegeben in ppm relativ zu Tetramethylsilan (TMS), Kopplungskonstanten *J* sind angegeben in Hz. Echte und Pseudo-Kopplungsmuster wurden nicht unterschieden.

Die **Viskosität** wurde auf einem thermostatisierten Kegel-Platten-Viskosimeter Physica UM (Kegeldurchmesser 20 mm, Kegelwinkel 1°, Kegelspitze-Platten-Abstand 0.05 mm, Scherrate 10 bis 1000 $s^{-1}$) gemessen.

Der **Amingehalt,** das heisst der totale Gehalt an freien Aminogruppen und blockierten Aminogruppen (Aldiminogruppen) in den hergestellten Verbindungen, wurde titrimetrisch bestimmt (mit $0.1_N$ $HClO_4$ in Eisessig, gegen Kristallviolett) und ist stets angegeben in mmol N/g.

Als **Normklima** wird eine Temperatur von 23±1 °C bei einer relativen Luftfeuchtigkeit von 50±5% bezeichnet.

Herstellung von Aldiminen

**Aldimin *A-1***

In einem Rundkolben wurden unter Stickstoffatmosphäre 23.0 g (0.22 mol) Benzaldehyd vorgelegt. Unter kräftigem Rühren wurden aus einem Eintropftrichter 25.0 g (0.21 mol N) Polyetherdiamin (Polyoxypropylen-Diamin mit einem mittleren Molekulargewicht von ca. 240 g/mol; Jeffamine® D-230, Huntsman; Amingehalt 8.29 mmol N/g) langsam zugegeben. Danach wurden bei 80 °C die flüchtigen Bestandteile im Vakuum entfernt (10 mbar, 80 °C). Ausbeute: 44.0 g eines gelblichen, bei Raumtemperatur flüssigen Öls mit einem Amingehalt von 4.72 mmol N/g.

**Aldimin *A-2***

In einem Rundkolben wurden unter Stickstoffatmosphäre 74.3 g (0.26 mol) destilliertes 2,2-Dimethyl-3-lauroyloxy-propanal vorgelegt. Unter kräftigem Rühren wurden aus einem Eintropftrichter 30.0 g (0.25 mol N) Polyetherdiamin (Polyoxypropylen-Diamin mit einem mittleren Molekulargewicht von ca. 240 g/mol; Jeffamine® D-230, Huntsman; Amingehalt 8.29 mmol N/g) langsam zugegeben. Danach wurden die flüchtigen Bestandteile im Vakuum entfernt (10 mbar, 80 °C). Ausbeute: 99.5 g eines klaren, blassgelben Öls mit einem Amingehalt von 2.50 mmol N/g.

**Aldimin *A-3***

In einem Rundkolben wurden unter Stickstoffatmosphäre 52.4 g (0.18 mol) destilliertes 2,2-Dimethyl-3-lauroyloxy-propanal vorgelegt. Unter kräftigem Rühren wurden aus einem beheizten Eintropftrichter 10.0 g (0.17 mol N) 1,6-Hexamethylendiamin (BASF; Amingehalt 17.0 mmol N/g) langsam zugegeben. Danach wurden die flüchtigen Bestandteile im Vakuum entfernt (10 mbar, 80 °C). Ausbeute: 57.7 g eines klaren, blassgelben Öls mit einem Amingehalt von 2.85 mmol N/g.

Herstellung von Aldaziden

**Beispiel 1**

In einem Rundkolben wurden unter Stickstoffatmosphäre 5.00 g (0.029 mol) fein geriebenes Adipinsäuredihydrazid (Schmelzpunkt 180-182 °C) in 17.96 g (0.063 mol) 2,2-Dimethyl-3-lauroyloxy-propanal und 50 ml abs. Ethanol suspendiert. Die Suspension wurde unter kräftigem Rühren auf 90-95 °C erwärmt und dabei leicht evakuiert. Danach wurden die flüchtigen Bestandteile durch weiteres Aufwärmen auf 120 °C und Anlegen von Vakuum ($5 \cdot 10^{-2}$ mbar) vollständig entfernt. Ausbeute: 21.8 g eines opaleszenten, farb- und geruchlosen Öls, das beim Stehenlassen bei Raumtemperatur innerhalb von einigen Stunden zu einem weissen Körper kristallisierte. Schmelzpunkt (unkorrigiert): 62-63 °C.
IR: 3203w br ($\nu_{N-H}$), 3073w br ($\nu_{N-H}$), 2954m sh, 2921s, 2869m sh, 2852s, 1738s ($\nu_{OC=}$O), 1672vs br ($\nu_{C=N}$), 1630m ($\nu_{NC=O}$), 1553w br ($\delta_{N-H}$), 1485w sh, 1465m, 1439m sh, 1393m, 1376m, 1365m sh, 1347w, 1298m sh, 1282m, 1248m, 1233m, 1159s, 1112s, 1075w, 1059w, 1018m, 1002m, 933m, 887w sh, 865w, 797w, 740m sh, 721.
$^1$H-NMR ($CDCl_3$, 300 K): $\delta$ 9.35 (*s*, 2 H, =N-NH-CO), 7.05 (*s*, 2 H, CH=N), 4.00 (*s*, 4 H, $C(CH_3)_2$-$CH_2$-O), 2.64 (*t, J* ≈ 6.8, 4 H, NHC(O)-C*H*$_2$-$CH_2$), 2.31 (*t, J* ≈ 7.5, 4 H, OC(O)-C*H*$_2$-$CH_2$), 1.74 (t, *J* ≈ 7.0, 4 H, NHC(O)-$CH_2$-C*H*$_2$), 1.61 (*m*, 4 H, OC(O)-$CH_2$-C*H*$_2$), 1.25 (*m*, 32 H, $CH_3$-(C*H*$_2$)$_8$-$CH_2$-$CH_2$-CO), 1.14 (*s*, 12 H, C(C*H*$_3$)$_2$-$CH_2$-O), 0.88 (t, *J* ≈ 6.8, 6 H, C*H*$_3$-$(CH_2)_{10}$-CO).

**Beispiel 2**

In einem Rundkolben wurden unter Stickstoffatmosphäre 1.00 g (11.1 mmol) fein geriebenes Carbohydrazid (= Carbodihydrazid; Schmelzpunkt 150-153 °C) in 6.63 g (23.3 mmol) 2,2-Dimethyl-3-lauroyloxy-propanal suspendiert. Die Suspension wurde unter kräftigem Rühren auf 70 °C erwärmt und gleichzeitig im Hochvakuum evakuiert ($5 \cdot 10^{-2}$ mbar). Es entstand ein klares, farbloses Öl, welches plötzlich zu einem weissen Körper erstarrte. Nach Zermörserung und erneutem Evakuieren im Hochvakuum bei 70 °C erhielt man 7.19 g Produkt als weisses, geruchloses Pulver. Schmelzpunkt (unkorrigiert): 85-87 °C.
IR: 3190w br ($\nu_{N-H}$), 3086w br ($\nu_{N-H}$), 2948m sh, 2914s, 2868m sh, 2848s, 1730vs ($\nu_{OC=O}$), 1686vs ($\nu_{C=N}$), 1548s ($\nu_{NC=O}$), 1470m, 1430w, 1418w, 1396w, 1384w, 1366m, 1350w, 1326w, 1312w, 1299w, 1284w, 1258m, 1236m, 1214w sh, 1206m, 1178s, 1140m, 1106s, 1076w, 1060w, 1051w, 1028m, 1000m, 972w, 930m, 875w, 831 vw, 810w, 790w, 775vw, 746m, 728m sh, 718m, 664w.

Aus den Beispielen 1 und 2 ist ersichtlich, dass der Aldehyd 2,2-Dimethyl-3-lauroyloxy-propanal mit jeweils einem Carbonsäurehydrazid mit einem Schmelzpunkt von mindestens 100 °C bei erhöhter Temperatur mit hohem Umsatz zum entsprechenden Aldazid reagiert.

Verwendete Hydrazide

| | | |
|---|---|---|
| Hydrazid ***HY-1***: | Adipinsäuredihydrazid | Schmelzpunkt 180-182 °C |
| Hydrazid ***HY-2***: | Benzhydrazid | Schmelzpunkt 112-114 °C |
| Hydrazid ***HY-3***: | 2-Thiophencarbonsäurehydrazid | Schmelzpunkt 136-139 °C |

Herstellung von Polyurethan-Zusammensetzungen

**Beispiele 3 bis 7 und Vergleichsbeispiele 8 bis 9**

In einem Polypropylenbecher mit Schraubverschluss wurde das Polymer ***P-1***, dessen Herstellung nachfolgend beschrieben wird, mittels eines Zentrifugalmischers (SpeedMixer™ DAC 150, FlackTek Inc.; 1 min. bei 2500 U/min) mit den in der Tabelle 1 aufgeführten Zutaten in den angegebenen Gewichtsteilen zu einer homogenen Masse vermischt.
Das Polymer ***P-1*** wurde wie folgt hergestellt:
1300 g Polyoxypropylen-Diol (Acclaim® 4200 N, Bayer; OH-Zahl 28.5 mg KOH/g), 2600 g Polyoxypropylenpolyoxyethylen-Triol (Caradol® MD34-02, Shell; OH-Zahl 35.0 mg KOH/g), 600 g 4,4'-Methylendiphenyldiisocyanat (4,4'-MDI; Desmodur® 44 MC L, Bayer) und 500 g Diisodecylphthalat (DIDP; Palatino® Z, BASF) wurden bei 80 °C zu einem NCO-terminierten Polyurethanpolymeren mit einem Gehalt an freien Isocyanat-Gruppen von 2.05 Gewichts-% umgesetzt.

Tabelle 1: Zusammensetzung der Beispiele 3 bis 7 und der Vergleichsbeispiele 8 bis 9.

| Beispiel | **3** | **4** | **5** | **6** | **7** | **8(Vgl.)** | **9(Vgl.)** |
|---|---|---|---|---|---|---|---|
| Polymer ***P-1*** | 50.0 | 50.0 | 50.0 | 50.0 | 50.0 | 50.0 | 50.0 |
| Aldimin ***A-1*** | - | - | - | - | 2.58 | - | - |
| Aldimin ***A-2*** | 4.89 | 4.89 | 4.89 | 4.89 | - | 4.89 | - |
| Hydrazid ***HY-1*** | 1.06 | 0.53 | - | - | 1.06 | - | - |
| Hydrazid ***HY-2*** | - | - | 1.67 | - | - | - | - |
| Hydrazid ***HY-3*** | - | - | - | 1.75 | - | - | - |
| Säure-Katalysator[a] | 0.50 | 0.50 | 0.50 | 0.50 | 0.50 | 0.50 | 0.50 |
| Amin-Katalysator[b] | 0.05 | 0.05 | 0.05 | 0.05 | 0.05 | 0.05 | 0.05 |
| [Aldimin/NCO][c] | 0.50 | 0.50 | 0.50 | 0.50 | 0.50 | 0.50 | 0 |
| [Hydrazid/Aldimin][d] | 1 | 0.5 | 1 | 1 | 1 | 0 | - |

[a] 5 Gew.-% Salicylsäure in Dioctyladipat. [b] 2,2'-Dimorpholinodiethylether (DABCO® DMDEE Catalyst, Air Products). [c] Verhältnis zwischen Aldiminogruppen und Isocyanatgruppen. [d] Verhältnis zwischen Hydrazidgruppen und Aldiminogruppen.

Die so erhaltenen Zusammensetzungen wurden auf Viskosität, Lagerstabilität, Hautbildungszeit, Blasenbildung, mechanische Eigenschaften, Ausgasungsverlust und Ausgasungsgrad des Aldehyds geprüft.

Die **Lagerstabilität** wurde über die Veränderung (Zunahme) der Viskosität während der Lagerung in der Wärme bestimmt. Dazu wurde die Zusammensetzung in der verschlossenen Tube im Ofen bei 60 °C gelagert und die Viskosität bei 20 °C ein erstes Mal nach 6 Stunden und ein zweites Mal nach 7 Tagen Lagerdauer gemessen. Die Lagerstabilität ergibt sich aus der prozentualen Zunahme des zweiten Viskositätswerts gegenüber dem ersten.

Zur Messung der **Hautbildungszeit** (Zeit bis zur Klebefreiheit, "tackfree time") wurden einige Gramm der während 6 Stunden bei 60 °C gelagerten, raumtemperaturwarmen Zusammensetzung in einer Schichtdicke von ca. 2 mm auf Pappkarton aufgetragen und im Normklima die Zeit bestimmt, die es dauerte, bis beim leichten Antippen der Oberfläche der Zusammensetzung mittels einer Pipette aus LDPE erstmals keine Rückstände auf der Pipette mehr zurückblieben.

Die **Blasenbildung** wurde qualitativ anhand der Menge Blasen, die während der Aushärtung der Zusammensetzung auftraten, beurteilt.

Als **mechanische Eigenschaften** wurden die **Zugfestigkeit** (Bruchkraft), die **Bruchdehnung** und das **E-Modul** der ausgehärteten Zusammensetzung gemessen. Dazu wurde die während 6 Stunden bei 60 °C gelagerte, raumtemperaturwarme Zusammensetzung in einer planen PTFE-Form zu einem Film von ca. 2 mm Dicke gegossen und dieser während 7 Tagen im Normklima ausgehärtet. Aus dem Film wurden Hanteln mit einer Länge von 75 mm, bei einer Steglänge von 30 mm und einer Stegbreite von 4 mm, ausgestanzt und diese gemäss DIN EN 53504 bei einer Zuggeschwindigkeit von 200 mm/min geprüft.

Der **Ausgasungsverlust** der ausgehärteten Zusammensetzung wurde bestimmt, indem aus dem wie oben beschrieben hergestellten Film Hanteln gleicher Dimension ausgestanzt, diese während 7 Tagen im Ofen bei 100 °C offen gelagert und anschliessend auf Gewichtsverlust relativ zu ihrem Anfangsgewicht geprüft wurden (Angabe in Gewichts-%). Der angegebene Wert ist jeweils der Mittelwert von drei Hanteln desselben Films.

Der **Ausgasungsgrad des Aldehyds** wurde aus dem Ausgasungsverlust der ausgehärteten Zusammensetzung berechnet, wobei dieser um den Ausgasungsverlust des Aldimin-freien Vergleichsbeispiels 9 ("Nullwert") korrigiert wurde, gemäss der Formel: Ausgasungsgrad des Aldehyds = [(Ausgasungsverlust - Ausgasungsverlust Bsp. 9) / Aldehydgehalt] x 100%. Der Aldehydgehalt einer Zusammensetzung kann direkt aus dem Aldimingehalt berechnet werden. Je tiefer der Ausgasungsgrad des Aldehyds liegt, desto weniger Aldehyd wurde ausgegast.

Zur Bestimmung des **Fogging**-Verhaltens wurde wie folgt vorgegangen: Ein Stück des wie oben beschrieben hergestellten Films wurde in Stückchen von ca. 2x2 mm Grösse geschnitten. Etwa 5 g dieser Stücke wurden in eine Kristallisierschale eingewogen, diese mit einem tarierten Uhrglas abgedeckt und die Schale bei geringer Eintauchtiefe im Oelbad während 24 Stunden auf 100 °C bzw. während 12 Stunden auf 130 °C erwärmt, wobei sich am Uhrglas ein Kondensatbeschlag bildete. Die Menge des Kondensats wurde durch Rückwägung des beschlagenen Uhrglases bestimmt und in Prozent der eingewogenen Filmmenge als "Fogging" angegeben.

Die Neigung zur **Vergilbung** wurde qualitativ an den während 7 Tagen bei 100 °C gelagerten Proben gegen eine nicht erhitzte Referenzprobe auf einer Skala von 0 bis 3 bewertet, wobei "0" keine Vergilbung, "1" schwache Vergilbung (blassgelb), "2" mittlere Vergilbung (tiefgelb) und "3" starke Vergilbung (braungelb) bedeuten. Die Ergebnisse der Prüfungen sind in der Tabelle 2 aufgeführt.

Tabelle 2: Eigenschaften der Beispiele 3-7 und der Vergleichsbeispiele 8-9.

| Beispiel | **3** | **4** | **5** | **6** | **7** | **8(Vgl.)** | **9(Vgl.)** |
|---|---|---|---|---|---|---|---|
| Viskosität nach 6h[a] | n.b.<br>35 | n.b.<br>33 | 32<br>n.b. | 27<br>n.b. | n.b.<br>49 | n.b.<br>32 | n.b.<br>60 |
| Viskosität nach 7d[a] | n.b.<br>40 | n.b.<br>38 | 55<br>n.b. | 42<br>n.b. | n.b.<br>83 | n.b.<br>37 | n.b.<br>63 |
| Viskositätszunahme (%)[b] | 14 | 15 | 72 | 56 | 69 | 16 | 5 |
| Hautbildungszeit (min) | 18 | 20 | 21 | 19 | 55 | 24 | 45 |
| Blasenbildung[c] | ok | ok | ok | ok | B | ok | BB |
| Zugfestigkeit (MPa)[d] Zugfestigkeit (MPa)[d] | 0.54<br>0.72 | 0.55<br>0.93 | 0.44<br>0.63 | 0.67<br>0.90 | 0.42<br>0.65 | 0.50<br>0.71 | 0.69<br>0.71 |
| Bruchdehnung (%)[d] | 188<br>321 | 191<br>345 | 240<br>319 | 214<br>277 | 349<br>667 | 133<br>186 | 86<br>93 |

(fortgesetzt)

| Beispiel | 3 | 4 | 5 | 6 | 7 | 8(Vgl.) | 9(Vgl.) |
|---|---|---|---|---|---|---|---|
| E-Modul bei 0.5-5% Dehnung (MPa)[d] | 0.59<br>0.42 | 0.50<br>0.52 | 0.26<br>0.38 | 0.34<br>0.60 | 0.10<br>0.25 | 0.50<br>0.64 | 1.08<br>0.95 |
| Ausgasungsverlust nach 7d/100 °C (%) | 3.28 | 5.40 | 3.13 | 6.00 | 2.13 | 7.95 | 1.33 |
| Ausgasungsgrad des Aldehyds (%) | 30 | 62 | 28 | 73 | 33 | 100 | - |
| Fogging nach 24h / 100 °C (%) | 0.02 | n.b. | n.b. | n.b. | n.b. | 0.55 | 0.01 |
| Fogging nach 12h / 130 °C (%) | 0.12 | n.b. | n.b. | n.b. | n.b. | 2.30 | 0.05 |
| Vergilbung nach 7d/100°C | 1 | 1-2 | 1-2 | 1-2 | 1-2 | 3 | 2-3 |

[a] in Pa·s, 1. Zahl: Lagerung bei Raumtemperatur, 2. Zahl: Lagerung bei 60°C. [b] = [(Viskosität nach 7d / Viskosität nach 6h) - 1] × 100%. [c] ok = keine Blasen; B = Blasen; BB = viele Blasen. [d] obere Zahl: Prüfung nach Aushärtung im Normklima; untere Zahl: Prüfung nach Wärmebelastung des ausgehärteten Materials (7d/100 °C). n.b. = nicht bestimmt.

Aus der Tabelle 2 ist ersichtlich, dass die erfindungsgemässen Zusammensetzungen der Beispiele 3 bis 7, welche neben einem Aldimin ein Hydrazid enthalten, gegenüber der Zusammensetzung des Vergleichsbeispiels 8 ohne Hydrazid einen leicht bis deutlich verminderten Ausgasungsverlust, ein geringeres Fogging und eine niedrige Neigung zur Vergilbung aufweisen. In Abhängigkeit des eingesetzen Hydrazids zeigen sich Unterschiede bei der Lagerstabilität; die Beispiele 3, 4 und 7 sind bei 60 °C lagerstabil, während die Beispiele 5 und 6 bei Raumtemperatur lagerstabil sind. Das Referenzbeispiel 9, welches kein Aldimin enthält, zeigt einen geringen Ausgasungsverlust und niedriges Fogging, neigt aber zu Blasenbildung und Vergilbung.

Figur 1 zeigt Infrarotspektren. Das als **IR-1** bezeichnete Spektrum wurde von der Polyurethanzusammensetzung des Beispiels 3 nach Aushärtung im Normklima aufgenommen. Das als **IR-2** bezeichnete Spektrum wurde von der Polyurethanzusammensetzung des Beispiels 3 nach Aushärtung im Normklima und anschliessendem Erwärmen während 24 Stunden auf 100 °C aufgenommen. Das als **IR-R1** bezeichnete Spektrum ist das Spektrum des Aldazids des Beispiels 1 (Kondensationsprodukt aus Adipinsäuredihydrazid und 2,2-Dimethyl-3-lauroyloxy-propanal).

Aus dem Vergleich dieser drei Spektren, insbesondere durch die für das Aldazid charakteristische Bande bei 1672 $cm^{-1}$($\nu_{C=N}$), ist ersichtlich, dass sich durch das Aufheizen der Polyurethanzusammensetzung des Beispiels 3 das Aldazid des Beispiels 1 gebildet hat. Die Erwärmung des bei Normklima ausgehärteten Films auf 100 °C hat also zu einem deutlichen Umsatz des Adipinsäuredihydrazids mit dem aus dem Aldimin ***A-2*** freigesetzten Aldehyd (2,2-Dimethyl-3-lauroyloxy-propanal) zum entsprechenden Aldazid geführt.

Für die Beispiele 3 und 8 wurde anschliessend an die Bestimmung des Foggings bei 130 °C vom jeweiligen Kondensat auf dem Uhrglas ein Infrarotspektrum aufgenommen. In Figur 2 zeigt das als **IR-3** bezeichnete Spektrum das Kondensat von Beispiel 3, während das als **IR-R2** bezeichnete Spektrum das Kondensat von Beispiel 8 zeigt. Das als **IR-R3** bezeichnete Spektrum stammt von 2,2-Dimethyl-3-lauroyloxy-propanal, also dem aus dem Aldimin ***A-2*** freigesetzten Aldehyd. Aus dem Vergleich dieser Spektren ist ersichtlich, dass das Kondensat des Beispiels 8 im Wesentlichen reines 2,2-Dimethyl-3-lauroyloxy-propanal darstellt, während das in der Menge bedeutend geringere (vgl. Tabelle 2) Kondensat des Beispiels 3 aufgrund der Abwesenheit der markierten Aldehyd-typischen Bande ($\nu_{CHO}$) offensichtlich wenig oder kein Aldehyd enthält.

Herstellung von einkomponentigen Polyurethan-Klebstoffen

**Beispiele 10 bis 12 und Vergleichsbeispiele 13 bis 14: Elastischer Scheibenklebstoff**

Für jedes Beispiel wurden die jeweiligen Bestandteile gemäss Tabelle 3 in den angegebenen Gewichtsteilen in einem Vakuummischer unter Feuchtigkeitsausschluss zu einer homogenen Paste verarbeitet, diese unverzüglich in eine innenlackierte Aluminium-Kartusche abgefüllt und die Kartusche luftdicht verschlossen. Das Polymer ***P-1*** wurde wie in Beispiel 3 beschrieben hergestellt.

Tabelle 3: Zusammensetzung der Klebstofife der Beispiele 10 bis 12 und der Vergleichsbeispiele 13 bis 14.

| Beispiel | **10** | **11** | **12** | **13 (Vgl.)** | **14(Vgl.)** |
|---|---|---|---|---|---|
| Polymer ***P-1*** | 40.00 | 40.00 | 40.00 | 40.00 | 40.00 |
| Aldimin ***A-2*** | 5.47 | 5.47 | - | 5.47 | - |
| Aldimin ***A-3*** | - | - | 4.79 | - | - |
| Hydrazid ***HY-1*** | 1.19 | 0.59 | 1.19 | - | - |
| Weichmacher[a] | 15.53 | 15.53 | 16.21 | 15.53 | 21.00 |
| Kaolin[b] | 20.81 | 21.41 | 20.81 | 22.00 | 22.00 |
| Russ[b] | 12.00 | 12.00 | 12.00 | 12.00 | 12.00 |
| Verdickungsmittel[c] | 4.00 | 4.00 | 4.00 | 4.00 | 4.00 |
| Trocknungsmittel[d] | 0.20 | 0.20 | 0.20 | 0.20 | 0.20 |
| Epoxysilan[e] | 0.20 | 0.20 | 0.20 | 0.20 | 0.20 |
| Säure-Katalysator[f] | 0.50 | 0.50 | 0.50 | 0.50 | 0.50 |
| Amin-Katalysator[g] | 0.10 | 0.10 | 0.10 | 0.10 | 0.10 |
| [Aldimin/NCO][h] | 0.70 | 0.70 | 0.70 | 0.70 | 0 |
| [Hydrazid/Aldimin][i] | 1 | 0.5 | 1 | 0 | - |

[a] Diisodecylphthalat (DIDP; Palatinol® Z, BASF). [b] getrocknet bei 130 °C. [c] hydrophobe pyrogene Kieselsäure (Aerosil® R972, Degussa). [d] p-Toluolsulfonylisocyanat (Zusatzmittel TI, Bayer). [e] 3-Glycidoxypropyltriethoxysilan (Dynasylan® GLYEO, Degussa). [f] 5 Gew.-% Salicylsäure in Dioctyladipat. [g] 2,2'-Dimorpholinodiethylether (DABCO® DMDEE Catalyst, Air Products). [h] Verhältnis zwischen Aldiminogruppen und Isocyanatgruppen. [i] Verhältnis zwischen Hydrazidgruppen und Aldiminogruppen.

Die so erhaltenen Klebstoffe wurden auf Applikationseigenschaften, Hautbildungszeit, mechanische Eigenschaften, Ausgasungsverlust und Ausgasungsgrad des Aldehyds geprüft.

Als Mass für die **Applikationseigenschaften** wurden die **Standfestigkeit** und der Fadenzug herangezogen. Zur Bestimmung der Standfestigkeit wurde der Klebstoff mittels Kartuschenpistole über eine Dreiecksdüse als waagrecht verlaufende Dreiecksraupe mit einem Basisdurchmesser von 8 mm und einer Höhe (Abstand der Dreiecksspitze von der Basis) von 20 mm auf ein senkrecht stehendes Stück Pappkarton aufgetragen. Nach 5 Minuten wurde gemessen, wie weit sich die Spitze abgesenkt, d.h. von der ursprünglichen Position in der Mitte der Dreiecksraupe wegbewegt, hatte. Als "sehr gut" wurde bewertet, wenn sich die Spitze in vollständig oder annährend unveränderter Position befand, als "gut", wenn die Spitze sich zwischen Mitte und Basisende befand. Der **Fadenzug** wurde qualitativ bestimmt, indem etwas Klebstoff mittels Kartuschenpistole auf ein an der Wand befestigtes Stück Pappkarton aufgetragen wurde, die Kartuschenpistole beim Auftragsende durch rasches Zurückziehen vom aufgetragenen Klebstoff weggezogen und die Länge des dabei an der Abrissstelle zurückbleibenden Fadens gemessen wurde.

Zur Bestimmung der mechanischen Eigenschaften nach der Aushärtung wurden die Shore A-Härte, die Zugfestigkeit, Bruchdehnung und das E-Modul gemessen. Die **Shore A-Härte** wurde bestimmt nach DIN 53505 an während 14 Tagen im Normklima ausgehärteten Prüfkörpern.

Die übrigen Eigenschaften wurden wie bei Beispiel 3 beschrieben geprüft, wobei der Ausgasungsverlust auch nach 7d/80 °C bestimmt wurde. Zur Berechnung des Ausgasungsgrades wurde als Nullwert der Ausgasungsverlust des Aldimin-freien Vergleichsbeispiels 14 eingesetzt.

Alle Klebstoffe härteten vollständig blasenfrei aus.

Die Ergebnisse der Prüfungen sind in der Tabelle 4 aufgeführt.

Tabelle 4: Eigenschaften der Klebstoffe der Beispiele 10 bis 12 und der Vergleichsbeispiele 13 bis 14.

| Beispiel | **10** | **11** | **121** | **13 (Vgl.)** | **14 (Vgl.)** |
|---|---|---|---|---|---|
| Standfestigkeit | sehr gut | sehr gut | gut | sehr gut | sehr gut |
| Fadenzug (cm) | 0.3 | 0.3 | 1.2 | 1.0 | 1.0 |
| Hautbildungszeit (min) | 26 | 23 | 24 | 26 | 115 |

(fortgesetzt)

| Beispiel | 10 | 11 | 121 | 13 (Vgl.) | 14 (Vgl.) |
|---|---|---|---|---|---|
| Shore A-Härte | 51 | 52 | 62 | 49 | 59 |
| Zugfestigkeit (MPa)[a] | 6.4<br>5.8<br>5.9 | 6.3<br>6.4<br>6.6 | 5.0<br>6.5<br>4.8 | 6.5<br>6.8<br>7.3 | 6.6<br>7.5<br>7.5 |
| Bruchdehnung (%)[a] | 650<br>520<br>670<br>580 | 660<br>510<br>620<br>550 | 530<br>300<br>420<br>290 | 650<br>470<br>570<br>450 | 470<br>370<br>400<br>320 |
| E-Modul bei 0.5-5% Dehnung (MPa)[a] | 2.8<br>2.1<br>2.3<br>2.2 | 2.9<br>2.3<br>3.1<br>2.9 | 3.9<br>4.2<br>4.2<br>3.8 | 2.7<br>1.8<br>3.5<br>4.2 | 3.6<br>3.4<br>4.0<br>4.2 |
| Ausgasungsverlust (%)[b] | 1.55<br>1.84 | 2.53<br>3.07 | 1.47<br>1.80 | 4.03<br>5.36 | 0.88<br>1.80 |
| Ausgasungsgrad des Aldehyds (%)[b] | 16<br>1 | 40<br>31 | 14<br>0 | 77<br>87 | -<br>- |

[a] 1. Zahl: Prüfung nach Aushärtung im Normklima; 2. Zahl: Prüfung nach Schwitzwasserbelastung (7d/70 °C) ("Kataplasma") des im Normklima ausgehärteten Materials; 3. Zahl: Prüfung nach Wärmebelastung (7d/80 °C) des im Normklima ausgehärteten Materials; 4. Zahl: Prüfung nach Wärmebelastung (7d/100 °C) des im Normklima ausgehärteten Materials. [b] obere Zahl: nach 7d/80 °C; untere Zahl: nach 7d/100 °C.

Aus der Tabelle 4 ist ersichtlich, dass die erfindungsgemässen Klebstoffe der Beispiele 10 bis 12, welche neben einem Aldimin ein Hydrazid enthalten, gegenüber dem Klebstoff des Vergleichsbeispiels 13 ohne Hydrazid einen stark verminderten Ausgasungsverlust aufweisen. Bei stöchiometrischem Einsatz des Hydrazids in Bezug auf das Aldimin (Beispiele 10 und 11) werden bei 100 °C Ausgasungsverluste erhalten, die jenem des Referenzbeispiels 14 ohne Aldimin nahekommen; der berechnete Ausgasungsgrad des Aldehyds sinkt dabei auf gegen 0%, was bedeutet, dass praktisch kein Aldehyd mehr entweicht.

**Beispiele 15 bis 16 und Vergleichsbeispiele 17 bis 18: Elastischer Montageklebstoff**

Für jedes Beispiel wurden die jeweiligen Bestandteile gemäss Tabelle 5 in den angegebenen Gewichtsteilen in einem Vakuummischer unter Feuchtigkeitsausschluss zu einer homogenen Paste verarbeitet, diese unverzüglich in eine innenlackierte Aluminium-Kartusche abgefüllt und die Kartusche luftdicht verschlossen.
Das Polymer ***P-1*** wurde wie in Beispiel 3 beschrieben hergestellt.
Das Polyurethanpolymer ***P-2*** wurde wie folgt hergestellt:
590 g Polyol Acclaim® 4200 N (Polypropylenoxid-Diol, OH-Zahl 28.5 mg KOH/g; Bayer), 1180 g Polyol Caradol® MD34-02 (Polypropylenoxidpolyethylenoxid-Triol, OH-Zahl 35.0 mg KOH/g; Shell) und 230 g Isophorondiisocyanat (IPDI; Vestanat® IPDI, Degussa) wurden nach bekanntem Verfahren bei 80 °C zu einem NCO-terminierten Polyurethanpolymeren mit einem titrimetrisch bestimmten Gehalt an freien Isocyanat-Gruppen von 2.1 Gewichts-% umgesetzt.
Das Harnstoff-Verdickungsmittel wurde wie folgt hergestellt:
In einem Vakuummischer wurden 3000 g Diisodecylphthalat (DIDP; Palatinol® Z, BASF) und 480 g 4,4'-Methylendiphenyldiisocyanat (4,4'-MDI; Desmodur® 44 MC L, Bayer) vorgelegt und leicht aufgewärmt. Dann wurden unter starkem Rühren 270 g Monobutylamin langsam zugetropft. Die entstehende Paste wurde unter Vakuum und Kühlung eine Stunde weitergerührt.

Tabelle 5: Zusammensetzung der Klebstoffe der Beispiele 15 bis 16 und der Vergleichsbeispiele 17 bis 18.

| Beispiel | 15 | 16 | 17 (Vergleich) | 18 (Vergleich) |
|---|---|---|---|---|
| Polymer ***P-1*** | 30.00 | 30.00 | 30.00 | 30.00 |
| Polymer ***P-2*** | 5.00 | 5.00 | 5.00 | 5.00 |

(fortgesetzt)

| Beispiel | **15** | **16** | **17 (Vergleich)** | **18 (Vergleich)** |
|---|---|---|---|---|
| Aldimin ***A-2*** | 4.81 | 4.81 | 4.81 | - |
| Hydrazid ***HY-1*** | 1.04 | 0.52 | - | - |
| Kreide | 29.96 | 30.48 | 31.00 | 31.00 |
| Verdickungsmittel[a] | 23.74 | 23.74 | 23.74 | 28.55 |
| Titandioxid | 4.50 | 4.50 | 4.50 | 4.50 |
| Epoxysilan[b] | 0.25 | 0.25 | 0.25 | 0.25 |
| Säure-Katalysator[c] | 0.50 | 0.50 | 0.50 | 0.50 |
| Zinn-Katalysator[d] | 0.10 | 0.10 | 0.10 | 0.10 |
| Amin-Katalysator[e] | 0.10 | 0.10 | 0.10 | 0.10 |
| [Aldimin/NCO][f] | 0.70 | 0.70 | 0.70 | 0 |
| [Hydrazid/Aldimin][g] | 1 | 0.5 | 0 | - |

[a] Harnstoff-Verdickungsmittel. [b] 3-Glycidoxypropyltriethoxysilan (Dynasylan® GLYEO, Degussa). [c] 5 Gew.-% Salicylsäure in Dioctyladipat. [d] Dibutylzinndilaurat (5 Gew.-% in Diisodecylphthalat). [e] 2,2'-Dimorpholinodiethylether (DABCO® DMDEE Catalyst, Air Products). [f] Verhältnis zwischen Aldiminogruppen und Isocyanatgruppen. [g] Verhältnis zwischen Hydrazidgruppen und Aldiminogruppen.

Die so erhaltenen Klebstoffe wurden auf Applikationseigenschaften, Hautbildungszeit, mechanische Eigenschaften, Ausgasungsverlust und Ausgasungsgrad des Aldehyds geprüft wie bei Beispiel 10 beschrieben. Zur Berechnung des Ausgasungsgrades wurde als Nullwert der Ausgasungsverlust des Aldimin-freien Vergleichsbeispiels 18 eingesetzt. Ausserdem wurde die Blasenbildung geprüft wie bei Beispiel 3 beschrieben.

Die Ergebnisse der Prüfungen sind in der Tabelle 6 aufgeführt.

Tabelle 6: Eigenschaften der Klebstoffe der Beispiele 15 bis 16 und der Vergleichsbeispiele 17 bis 18.

| Beispiel | **15** | **16** | **17 (Vergleich)** | **18 (Vergleich)** |
|---|---|---|---|---|
| Standfestigkeit | sehr gut | sehr gut | sehr gut | sehr gut |
| Fadenzug (cm) | 10 | 11 | 8 | 9 |
| Hautbildungszeit (min) | 30 | 28 | 37 | 46 |
| Blasenbildung | keine | keine | keine | einige |
| Shore A-Härte | 40 | 42 | 41 | 50 |
| Zugfestigkeit (MPa)[a] | 1.45<br>1.91<br>1.91<br>1.88 | 1.47<br>1.60<br>1.86<br>2.10 | 1.80<br>1.83<br>2.11<br>2.23 | 1.70<br>n.b.<br>n.b.<br>n.b. |
| Bruchdehnung (%)[a] | 720<br>730<br>920<br>1080 | 730<br>750<br>910<br>1080 | 840<br>790<br>890<br>1030 | 275<br>n.b.<br>n.b.<br>n.b. |
| E-Modul bei 0.5-5% Dehnung (MPa)[a] | 2.35<br>2.55<br>2.28<br>2.62 | 2.40<br>2.51<br>1.93<br>2.79 | 2.27<br>1.92<br>3.26<br>3.63 | 8.35<br>n.b.<br>n.b.<br>n.b. |
| Ausgasungsverlust (%)[b] | 1.36<br>1.72 | 2.02<br>3.02 | 2.91<br>4.08 | 0.74<br>1.45 |

(fortgesetzt)

| Beispiel | **15** | **16** | **17 (Vergleich)** | **18 (Vergleich)** |
|---|---|---|---|---|
| Ausgasungsgrad des Aldehyds (%)[b] | 17<br>8 | 36<br>44 | 61<br>73 | -<br>- |

[a] 1. Zahl: Prüfung nach Aushärtung im Normklima; 2. Zahl: Prüfung nach Schwitzwasserbelastung (7d/70 °C) ("Kataplasma") des im Normklima ausgehärteten Materials; 3. Zahl: Prüfung nach Wärmebelastung (7d/80 °C) des im Normklima ausgehärteten Materials ; 4. Zahl: Prüfung nach Wärmebelastung (7d/100 °C) des im Normklima ausgehärteten Materials. [b] obere Zahl: nach 7d/80 °C; untere Zahl: nach 7d/100 °C. n.b. = nicht bestimmt.

Aus der Tabelle 6 ist ersichtlich, dass die erfindungsgemässen Klebstoffe der Beispiele 15 bis 16, welche neben dem Aldimin ***A-2*** Adipinsäuredihydrazid enthalten, gegenüber dem Klebstoff des Vergleichsbeispiels 17 ohne Hydrazid einen deutlich verminderten Ausgasungsverlust aufweisen. Bei stöchiometrischem Einsatz des Hydrazids in Bezug auf das Aldimin (Beispiel 15) sinkt der Ausgasungsverlust bei 80 °C auf unter die Hälfte des für das Vergleichsbeispiel 17 gemessenen Werts und erreicht bei 100 °C annähernd den für das Referenzbeispiel 18, welches kein Aldimin enthält, gemessenen Wert. Die thermische Stabilität der Klebstoffe der Beispiele 15 und 16 ist dabei vergleichbar gut wie jene des Vergleichsbeispiels 17.

**Patentansprüche**

**1.** Härtbare Zusammensetzung, umfassend

a) mindestens ein Polyisocyanat **P,**
b) mindestens ein mittels Aldehyd oder Keton blockiertes Amin **BA,**
c) mindestens ein Hydrazid **HY** einer Carbon- oder Sulfonsäure, welches einen Schmelzpunkt von mindestens 100 °C, insbesondere von mindestens 150 °C, aufweist,
mit der Massgabe, dass das Hydrazid **HY** in einer Menge von 0.3 bis 1.1 Equivalent Hydrazidgruppen pro Equivalent Aldehyd- oder Ketogruppen, mittels welchen das Amin **BA** blockiert ist, vorhanden ist.

**2.** Härtbare Zusammensetzung gemäss Anspruch 1, **dadurch gekennzeichnet, dass** das Polyisocyanat **P** ein Isocyanatgruppen aufweisendes Polyurethanpolymer **PUP** ist, welches insbesondere erhältlich ist aus der Umsetzung von mindestens einem Polyol mit mindestens einem Polyisocyanat.

**3.** Härtbare Zusammensetzung gemäss Anspruch 1, **dadurch gekennzeichnet, dass** das Polyisocyanat **P** ein Polyisocyanat **PI** in der Form eines monomeren Di- oder Triisocyanates oder eines Oligomeren eines monomeren Diisocyanates ist.

**4.** Härtbare Zusammensetzung gemäss Anspruch 1, **dadurch gekennzeichnet, dass** das Polyisocyanat **P** ein Polyisocyanat **PI** in der Form einer bei Raumtemperatur flüssigen Form von MDI oder einer Form von polymerem MDI (PMDI) ist.

**5.** Härtbare Zusammensetzung gemäss einem der Ansprüche 1 bis 4, **dadurch gekennzeichnet, dass** das blockierte Amin **BA** ein Aldimin **BA1** der Formel (I) ist,

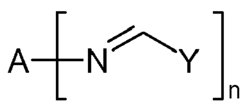

(I)

wobei A für den Rest eines Amins **B** nach Entfernung von n primären Aminogruppen steht,
Y für einen organischen, gegebenenfalls Heteroatome aufweisenden, Rest mit 1 bis 35 C-Atomen steht und
n für eine ganze Zahl von 1 bis 5 steht.

**6.** Härtbare Zusammensetzung gemäss Anspruch 5, **dadurch gekennzeichnet, dass** das blockierte Amin **BA** ein

Aldimin **BA1** der Formel (I a) oder (I b) ist,

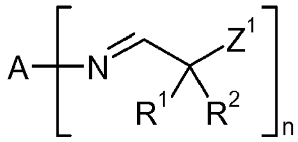

(I a)

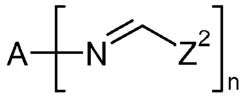

(I b)

wobei
$R^1$ und $R^2$ entweder
unabhängig voneinander jeweils für einen einwertigen Kohlenwasserstoffrest mit 1 bis 12 C-Atomen stehen,
oder
zusammen für einen zweiwertigen Kohlenwasserstoffrest mit 4 bis 20 C-Atomen, der Teil eines, gegebenenfalls substituierten, carbocyclischen Rings mit 5 bis 8, bevorzugt 6, C-Atomen ist, stehen;
$Z^1$ für einen einwertigen Kohlenwasserstoffrest mit 1 bis 32 C-Atomen steht, welcher gegebenenfalls mindestens ein Heteroatom, insbesondere Sauerstoff in der Form von Ether-, Carbonyl- oder Ester-Gruppen, oder insbesondere Stickstoff in Form von tertiären Aminogruppen, aufweist; $Z^2$ entweder
für eine substituierte oder unsubstituierte Aryl- oder Heteroaryl-Gruppe, welche eine Ringgrösse von 5 bis 8, bevorzugt 6, Atomen aufweist, steht,
oder für

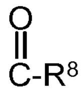

steht,
wobei $R^8$ für ein Wasserstoffatom oder für eine Alkoxygruppe steht, oder für eine substituierte oder unsubstituierte Alkenyl- oder Arylalkenylgruppe mit mindestens 6 C-Atomen steht.

**7.** Härtbare Zusammensetzung gemäss Anspruch 6, **dadurch gekennzeichnet, dass** $R^1$ und $R^2$ in Formel (I a) jeweils für eine Methylgruppe stehen.

**8.** Härtbare Zusammensetzung gemäss Anspruch 6 oder 7, **dadurch gekennzeichnet, dass** $Z^1$ in Formel (I a) für einen Rest der Formel (II) oder (III) oder (IV) steht,

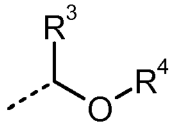

(II)

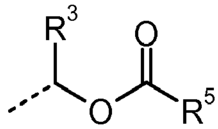

(III)

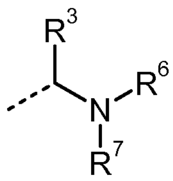

(IV)

wobei
$R^3$ für ein Wasserstoffatom oder für eine Alkylgruppe oder für eine Cycloalkylgruppe oder für eine Arylalkylgruppe mit 1 bis 12 C-Atomen steht;
$R^4$ für einen Kohlenwasserstoffrest mit 1 bis 30 C-Atomen, welcher gegebenenfalls Ethersauerstoffatome enthält, steht;
$R^5$ entweder
für ein Wasserstoffatom steht,
oder
für einen linearen oder verzweigten Alkylrest mit 1 bis 30 C-Atomen, gegebenenfalls mit cyclischen Anteilen und gegebenenfalls mit mindestens einem Heteroatom, insbesondere Sauerstoff in der Form von Ether-, Carbonyl- oder Ester-Gruppen, steht,
oder
für einen einfach oder mehrfach ungesättigten, linearen oder verzweigten Kohlenwasserstoffrest mit 5 bis 30 C-Atomen steht,
oder
für einen, gegebenenfalls substituierten, aromatischen oder heteroaromatischen 5- oder 6-gliedrigen Ring steht;
$R^6$ und $R^7$ entweder
unabhängig voneinander jeweils für einen einwertigen aliphatischen, cycloaliphatischen oder arylaliphatischen Rest mit 1 bis 20 C-Atomen stehen und welcher gegebenenfalls Heteroatome in der Form von Ether-Sauerstoff oder tertiärem Amin-Stickstoff enthält,
oder
zusammen für einen zweiwertigen aliphatischen Rest mit 3 bis 20 C-Atomen, der Teil eines, gegebenenfalls substituierten, heterocyclischen Rings mit 5 bis 8, bevorzugt 6, Ringatomen ist und neben dem Stickstoffatom gegebenenfalls weitere Heteroatome in der Form von Ether-Sauerstoff oder tertiärem Amin-Stickstoff enthält, stehen.

9. Härtbare Zusammensetzung gemäss Anspruch 8, **dadurch gekennzeichnet, dass** $R^4$ für einen Kohlenwasserstoffrest mit 6 bis 30, insbesondere mit 11 bis 30, C-Atomen, welcher gegebenenfalls Ethersauerstoffatome enthält, steht.

10. Härtbare Zusammensetzung gemäss Anspruch 8, **dadurch gekennzeichnet, dass** $R^5$ für einen linearen oder verzweigten Alkylrest mit 6 bis 30, insbesondere mit 11 bis 30, C-Atomen, gegebenenfalls mit cyclischen Anteilen und gegebenenfalls mit mindestens einem Heteroatom, oder für einen einfach oder mehrfach ungesättigten, linearen oder verzweigten Kohlenwasserstoffrest mit 6 bis 30, insbesondere mit 11 bis 30, C-Atomen, meist bevorzugt für einen $C_{11}$-Alkylrest, steht.

11. Härtbare Zusammensetzung gemäss einem der Ansprüche 5 bis 10, **dadurch gekennzeichnet, dass** das Amin **B** mindestens zwei primären Aminogruppen aufweist und ausgewählt ist aus der Gruppe bestehend aus 1,6-Hexamethylendiamin, 1,5-Diamino-2-methylpentan (MPMD), 1,3-Pentandiamin (DAMP), 1-Amino-3-aminomethyl-3,5,5-trimethylcyclohexan (= Isophorondiamin oder IPDA), 2,2,4- und 2,4,4-Trimethylhexamethylendiamin (TMD), 1,3-Xylylendiamin, 1,3-Bis-(aminomethyl)cyclohexan, Bis-(4-aminocyclohexyl)-methan, Bis-(4-amino-3-methylcyclohexyl)-methan, 3(4),8(9)-Bis-(aminomethyl)-tricyclo[5.2.1.$0^{2,6}$]decan, 1,2-, 1,3- und 1,4-Diaminocyclohexan, 1,4-Diamino-2,2,6-trimethylcyclohexan, 3,6-Dioxaoctan-1,8-diamin, 4,7-Dioxadecan-1,10-diamin, 4-Aminomethyl-1,8-octandiamin, Polyoxyalkylen-Polyamine mit zwei oder drei Aminogruppen, 1,3-und 1,4-Phenylendiamin, 2,4- und 2,6-Toluylendiamin, 4,4'-, 2,4'- und 2,2'-Diaminodiphenylmethan, 3,3'-Dichlor-4,4'-diaminodiphenylmethan und Mischungen der genannten Polyamine.

12. Härtbare Zusammensetzung gemäss einem der Ansprüche 5 bis 10, **dadurch gekennzeichnet, dass** das Amin **B** mindestens eine primäre Aminogruppe und mindestens eine weitere reaktive Gruppe aufweist, welche entweder eine Hydroxylgruppe, eine sekundäre Aminogruppe oder eine Mercaptogruppe darstellt, und welches insbesondere ausgewählt ist aus der Gruppe bestehend aus N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, Diethylentriamin (DETA), Dipropylentriamin (DPTA), Bis-hexamethylentriamin (BHMT), Fettdiamine wie N-Cocoalkyl-1,3-propandiamin, N-Oleyl-1,3-propandiamin, N-Soyaalkyl-1,3-propandiamin und N-Talgalkyl-1,3-propandiamin, 5-Amino-1-pentanol, 6-Amino-1-hexanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykolmonoamin, 3-(2-Hydroxyethoxy)-propylamin, 3-(2-(2-Hydroxyethoxy)-ethoxy)-propylamin und 3-(6-Hydroxyhexyloxy)-propylamin.

**13.** Härtbare Zusammensetzung gemäss einem der Ansprüche 1 bis 4, **dadurch gekennzeichnet, dass** das blockierte Amin **BA** ein Ketimin **BA2** der Formel (VII) ist,

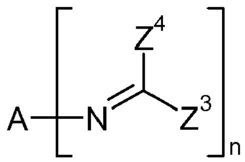

(VII)

wobei A für den Rest eines Amins **B** nach Entfernung von n primären Aminogruppen steht,
$Z^3$ und $Z^4$ entweder
unabhängig voneinander jeweils für einen einwertigen Kohlenwasserstoffrest mit 1 bis 12 C-Atomen stehen,
oder
zusammen für einen zweiwertigen Kohlenwasserstoffrest mit 4 bis 20 C-Atomen, der Teil eines, gegebenenfalls substituierten, carbocyclischen Rings mit 5 bis 8, bevorzugt 6, C-Atomen ist, stehen; und
n für eine ganze Zahl von 1 bis 5 steht.

**14.** Härtbare Zusammensetzung gemäss einem der Ansprüche 1 bis 4, **dadurch gekennzeichnet, dass** das blockierte Amin **BA** ein Enamin **BA3** ist, welches mindestens eine Enaminogruppe der Formel (IX) aufweist,

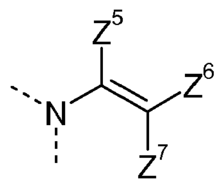

(IX)

wobei
$Z^5$ und $Z^6$ entweder
unabhängig voneinander jeweils für ein Wasserstoffatom oder für einen einwertigen Kohlenwasserstoffrest mit 1 bis 12 C-Atomen stehen, oder zusammen für einen zweiwertigen Kohlenwasserstoffrest mit 3 bis 20 C-Atomen, der Teil eines, gegebenenfalls substituierten, carbocyclischen Rings mit 5 bis 8, bevorzugt 6, C-Atomen ist; und
$Z^7$ für ein Wasserstoffatom oder für einen einwertigen Kohlenwasserstoffrest mit 1 bis 12 C-Atomen steht.

**15.** Härtbare Zusammensetzung gemäss einem der Ansprüche 1 bis 4, **dadurch gekennzeichnet, dass** das blockierte Amin **BA** ein Oxazolidin **BA4** der Formel (XI) ist,

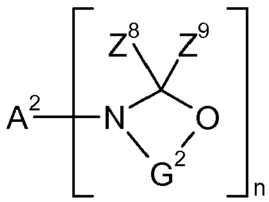

(XI)

wobei

$A^2$ für den Rest eines Amins nach Entfernung von n sekundären Aminogruppen steht;
$G^2$ für einen, gegebenenfalls substituierten, $C_2$- oder $C_3$-Alkylenrest steht; $Z^8$ und $Z^9$ unabhängig voneinander jeweils für ein Wasserstoffatom oder für einen einwertigen Kohlenwasserstoffrest mit 1 bis 12 C-Atomen stehen; und n für eine ganze Zahl von 1 bis 5 steht.

**16.** Härtbare Zusammensetzung gemäss einem der vorhergehenden Ansprüche, **dadurch gekennzeichnet, dass** das Hydrazid **HY** die Formel (XIV a) oder (XIV b) oder (XV) aufweist,

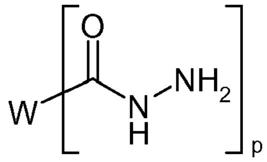
(XIV a)

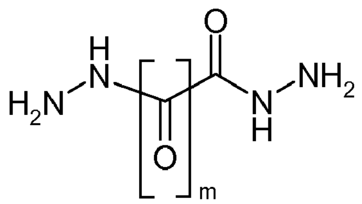
(XIV b)

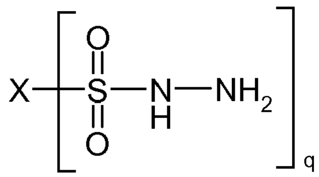
(XV)

wobei
W für den p-wertigen Rest eines Hydrazids **HY** einer Carbonsäure, welches einen Schmelzpunkt von mindestens 100 °C, insbesondere von mindestens 150 °C, aufweist, nach Entfernung von p Hydrazidgruppen steht;
X für den q-wertigen Rest eines Hydrazids **HY** einer Sulfonsäure, welches einen Schmelzpunkt von mindestens 100 °C, insbesondere von mindestens 150 °C, aufweist, nach Entfernung von q Sulfonsäurehydrazidgruppen steht;
m für null oder 1 steht;
p für 1 oder 2 oder 3 oder 4, bevorzugt für 2, steht; und
q für 1 oder 2 oder 3 oder 4 steht.

**17.** Härtbare Zusammensetzung gemäss einem der vorhergehenden Ansprüche, **dadurch gekennzeichnet, dass** das Hydrazid **HY** ein Carbonsäurehydrazid, insbesondere ein Carbonsäuredihydrazid, bevorzugt ausgewählt aus der Gruppe bestehend aus Carbodihydrazid, Oxalsäuredihydrazid, Bernsteinsäuredihydrazid, Adipinsäuredihydrazid, Korksäuredihydrazid, Azelainsäuredihydrazid, Sebacinsäuredihydrazid, Dodecansäuredihydrazid und Isophtalsäuredihydrazid, meist bevorzugt Adipinsäuredihydrazid, ist.

**18.** Härtbare Zusammensetzung gemäss einem der vorhergehenden Ansprüche, **dadurch gekennzeichnet, dass** das Hydrazid **HY** in einer Menge von 0.3 bis 1.1, insbesondere 0.5 bis 1.0, bevorzugt von 0.75 bis 1.0, Equivalent Hydrazidgruppen pro Equivalent Aldehyd- oder Ketogruppen, mittels welchen das Amin **BA** blockiert ist, vorhanden ist.

**19.** Verfahren zum Verkleben von einem Substrat **S1** mit einem Substrat **S2,** welches die Schritte umfasst:

i) Applikation einer härtbaren Zusammensetzung gemäss einem der Ansprüche 1 bis 18 auf ein Substrat **S1** bei einer Temperatur unterhalb von 40 °C;
ii) Kontaktieren der applizierten Zusammensetzung mit einem Substrat **S2** innerhalb der Offenzeit der Zusammensetzung;
iii) Aushärten der applizierten Zusammensetzung bei einer Temperatur unterhalb von 40 °C;

oder

i’) Applikation einer härtbaren Zusammensetzung gemäss einem der Ansprüche 1 bis 18 auf ein Substrat **S1** und auf ein Substrat **S2** bei einer Temperatur unterhalb von 40 °C;
ii’) Kontaktieren der applizierten Zusammensetzung miteinander innerhalb der Offenzeit der Zusammensetzung;
iii’) Aushärten der applizierten Zusammensetzung bei einer Temperatur unterhalb von 40 °C;

wobei das Substrat **S2** aus dem gleichen oder einem unterschiedlichen Material wie das Substrat **S1** besteht.

**20.** Verfahren zum Abdichten umfassend die Schritte:

i") Applikation einer härtbaren Zusammensetzung gemäss einem der Ansprüche 1 bis 18 bei einer Temperatur unterhalb von 40 °C zwischen ein Substrat **S1** und ein Substrat **S2,** so dass die Zusammensetzung mit dem Substrat **S1** und dem Substrat **S2** in Kontakt steht;
ii") Aushärten der applizierten Zusammensetzung bei einer Temperatur unterhalb von 40 °C;

wobei das Substrat **S2** aus dem gleichen oder einem unterschiedlichen Material wie das Substrat **S1** besteht.

**21.** Verfahren zum Beschichten eines Substrates **S1** umfassend die Schritte:

i’’’) Applikation einer härtbaren Zusammensetzung gemäss einem der Ansprüche 1 bis 18 bei einer Temperatur unterhalb von 40 °C auf ein Substrat **S1** innerhalb der Offenzeit der Zusammensetzung;
ii’’’) Aushärten der applizierten Zusammensetzung bei einer Temperatur unterhalb von 40 °C.

**22.** Artikel erhalten durch ein Verfahren gemäss Anspruch 19, 20 oder 21.

**23.** Ausgehärtete Zusammensetzung erhalten durch die Aushärtung einer härtbaren Zusammensetzung gemäss einem der Ansprüche 1 bis 18 bei einer Temperatur unterhalb von 40 °C mittels Einwirkung von Wasser, beispielsweise in der Form von Luftfeuchtigkeit, und anschliessender Aufwärmung der derart ausgehärteten Zusammensetzung auf eine Temperatur von 80 °C oder höher;
und wobei diese ausgehärtete Zusammensetzung mindestens eine Verbindung der Formel (XVIII a) oder (XVIII b) oder (XIX) enthält,

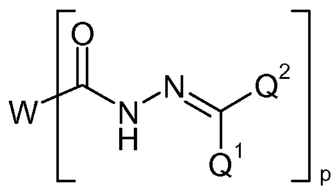

(XVIII a)

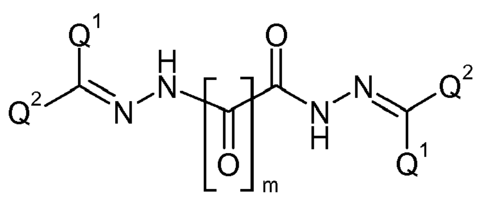

(XVIII b)

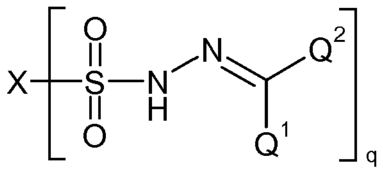

(XIX)

wobei

W für den p-wertigen Rest eines Hydrazids **HY** einer Carbonsäure, welches einen Schmelzpunkt von mindestens 100 °C, insbesondere von mindestens 150 °C, aufweist, nach Entfernung von p Hydrazidgruppen steht;

X für den q-wertigen Rest eines Hydrazids **HY** einer Sulfonsäure, welches einen Schmelzpunkt von mindestens 100 °C, insbesondere von mindestens 150 °C, aufweist, nach Entfernung von q Sulfonsäurehydrazidgruppen steht;

m für null oder 1 steht;

p für 1 oder 2 oder 3 oder 4, bevorzugt für 2, steht; und

q für 1 oder 2 oder 3 oder 4 steht;

und

$Q^1$ für ein Wasserstoffatom oder für $Z^3$ oder für $Z^5$ oder für $Z^8$ steht,

wobei

falls $Q^1$ für ein Wasserstoffatom steht, $Q^2$ für Y steht,

oder

falls $Q^1$ für $Z^3$ steht, $Q^2$ für $Z^4$ steht,

oder

falls $Q^1$ für $Z^5$ steht, $Q^2$ für

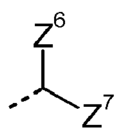

steht,

oder

falls $Q^1$ für $Z^8$ steht, $Q^2$ für $Z^9$ steht,

wobei

Y für einen organischen, gegebenenfalls Heteroatome aufweisenden, Rest mit 1 bis 35 C-Atomen, insbesondere für $-C(R^1)(R^2)(Z^1)$ oder $Z^2$ steht,

wobei $R^1$ und $R^2$ entweder

unabhängig voneinander jeweils für einen einwertigen Kohlenwasserstoffrest mit 1 bis 12 C-Atomen stehen,

oder

zusammen für einen zweiwertigen Kohlenwasserstoffrest mit 4 bis 20 C-Atomen, der Teil eines, gegebenenfalls substituierten, carbocyclischen Rings mit 5 bis 8, bevorzugt 6, C-Atomen ist, stehen;

$Z^1$ für einen einwertigen Kohlenwasserstoffrest mit 1 bis 32 C-Atomen steht, welcher gegebenenfalls mindestens ein Heteroatom, insbesondere Sauerstoff in der Form von Ether-, Carbonyl- oder Ester-Gruppen, oder insbesondere Stickstoff in Form von tertiären Aminogruppen, aufweist;

$Z^2$ entweder

für eine substituierte oder unsubstituierte Aryl- oder Heteroaryl-Gruppe, welche eine Ringgrösse von 5 bis 8, bevorzugt 6, Atomen aufweist, steht,

oder für

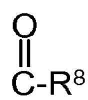

steht

wobei $R^8$ für ein Wasserstoffatom oder für eine Alkoxygruppe steht, oder für eine substituierte oder unsubstituierte

Alkenyl- oder Arylalkenylgruppe mit mindestens 6 C-Atomen steht;
$Z^3$ und $Z^4$ entweder
unabhängig voneinander jeweils für einen einwertigen Kohlenwasserstoffrest mit 1 bis 12 C-Atomen stehen,
oder
zusammen für einen zweiwertigen Kohlenwasserstoffrest mit 4 bis 20 C-Atomen, der Teil eines, gegebenenfalls substituierten, carbocyclischen Rings mit 5 bis 8, bevorzugt 6, C-Atomen ist, stehen; $Z^5$ und $Z^6$ entweder
unabhängig voneinander jeweils für ein Wasserstoffatom oder für einen einwertigen Kohlenwasserstoffrest mit 1 bis 12 C-Atomen stehen,
oder zusammen für einen zweiwertigen Kohlenwasserstoffrest mit
3 bis 20 C-Atomen, der Teil eines, gegebenenfalls substituierten,
carbocyclischen Rings mit 5 bis 8, bevorzugt 6, C-Atomen ist;
$Z^7$ für ein Wasserstoffatom oder für einen einwertigen Kohlenwasserstoffrest mit 1 bis 12 C-Atomen steht; und
$Z^8$ und $Z^9$ unabhängig voneinander jeweils für ein Wasserstoffatom oder für einen einwertigen Kohlenwasserstoffrest mit 1 bis 12 C-Atomen stehen.

**24.** Aldazid der Formel (XX a) oder (XX b),

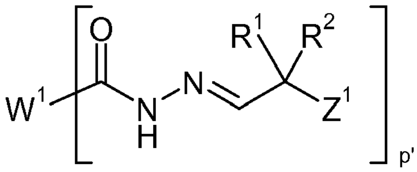

(XX a)

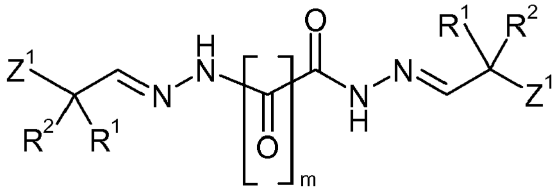

(XX b)

wobei
$W^1$ für den p'-wertigen Rest eines Hydrazids HY einer Carbonsäure, welches einen Schmelzpunkt von mindestens 100 °C, insbesondere von mindestens 150 °C, aufweist, nach Entfernung von p' Hydrazidgruppen steht;
$R^1$ und $R^2$ entweder
unabhängig voneinander jeweils für einen einwertigen Kohlenwasserstoffrest mit 1 bis 12 C-Atomen stehen,
oder
zusammen für einen zweiwertigen Kohlenwasserstoffrest mit 4 bis 20 C-Atomen, der Teil eines, gegebenenfalls substituierten, carbocyclischen Rings mit 5 bis 8, bevorzugt 6, C-Atomen ist, stehen;
$Z^1$ für einen einwertigen Kohlenwasserstoffrest mit 1 bis 32 C-Atomen steht, welcher gegebenenfalls mindestens ein Heteroatom, insbesondere Sauerstoff in der Form von Ether-, Carbonyl- oder Ester-Gruppen, oder insbesondere Stickstoff in Form von tertiären Aminogruppen, aufweist;
p' für 2 oder 3 oder 4, bevorzugt für 2, steht; und
m für null oder 1 steht.

**25.** Aldazid gemäss Anspruch 24, **dadurch gekennzeichnet, dass** $Z^1$ für einen Rest der Formel (II) steht,

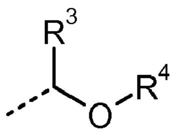

(II)

wobei $R^3$ für ein Wasserstoffatom oder für eine Alkylgruppe oder für eine Cycloalkylgruppe oder für eine Arylalkylgruppe mit 1 bis 12 C-Atomen steht; und

$R^4$ für einen Kohlenwasserstoffrest mit 1 bis 30, insbesondere mit 6 bis 30, bevorzugt mit 11 bis 30, C-Atomen, welcher gegebenenfalls Ethersauerstoffatome enthält, steht.

**26.** Aldazid gemäss Anspruch 24, **dadurch gekennzeichnet, dass** $Z^1$ für einen Rest der Formel (III) steht,

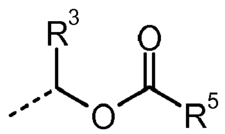

(III)

wobei $R^3$ für ein Wasserstoffatom oder für eine Alkylgruppe oder für eine Cycloalkylgruppe oder für eine Arylalkylgruppe mit 1 bis 12 C-Atomen steht; und
$R^5$ entweder
für ein Wasserstoffatom steht,
oder
für einen linearen oder verzweigten Alkylrest mit 1 bis 30, insbesondere mit 6 bis 30, bevorzugt mit 11 bis 30, C-Atomen, gegebenenfalls mit cyclischen Anteilen und gegebenenfalls mit mindestens einem Heteroatom, insbesondere Sauerstoff in der Form von Ether-, Carbonyl- oder Ester-Gruppen, steht
oder
für einen einfach oder mehrfach ungesättigten, linearen oder verzweigten Kohlenwasserstoffrest mit 5 bis 30 C-Atomen steht,
oder für einen, gegebenenfalls substituierten, aromatischen oder heteroaromatischen 5- oder 6-gliedrigen Ring steht;

**27.** Aldazid gemäss Anspruch 24, **dadurch gekennzeichnet, dass** $Z^1$ für einen Rest der Formel (IV) steht,

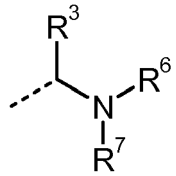

(IV)

wobei
$R^3$ für ein Wasserstoffatom oder für eine Alkylgruppe oder für eine Cycloalkylgruppe oder für eine Arylalkylgruppe mit 1 bis 12 C-Atomen steht; und
$R^6$ und $R^7$ entweder
unabhängig voneinander jeweils für einen einwertigen aliphatischen, cycloaliphatischen oder arylaliphatischen Rest mit 1 bis 20 C-Atomen stehen und welcher gegebenenfalls
Heteroatome in der Form von Ether-Sauerstoff oder tertiärem Amin-Stickstoff enthält,
oder
zusammen für einen zweiwertigen aliphatischen Rest mit 3 bis 20 C-Atomen, der Teil eines, gegebenenfalls substituierten, heterocyclischen Rings mit 5 bis 8, bevorzugt 6, Ringatomen ist und neben dem Stickstoffatom gegebenenfalls weitere Heteroatome in der Form von Ether-Sauerstoff oder tertiärem Amin-Stickstoff enthält, stehen.

**28.** Verwendung eines Hydrazids **HY**, wie es in einer härtbaren Zusammensetzung gemäss einem der Ansprüche 1 bis 18 vorhanden ist, oder eines Aldazids gemäss einem der Ansprüche 24 bis 27 zur Reduktion der Vergilbung von ausgehärteten Polyurethanzusammensetzungen.

**29.** Verwendung eines Hydrazids **HY**, wie es in einer härtbaren Zusammensetzung gemäss einem der Ansprüche 1 bis 18 vorhanden ist, zur Reduktion der Aldehyd- oder Keton-Ausgasung aus der ausgehärteten Zusammensetzung.

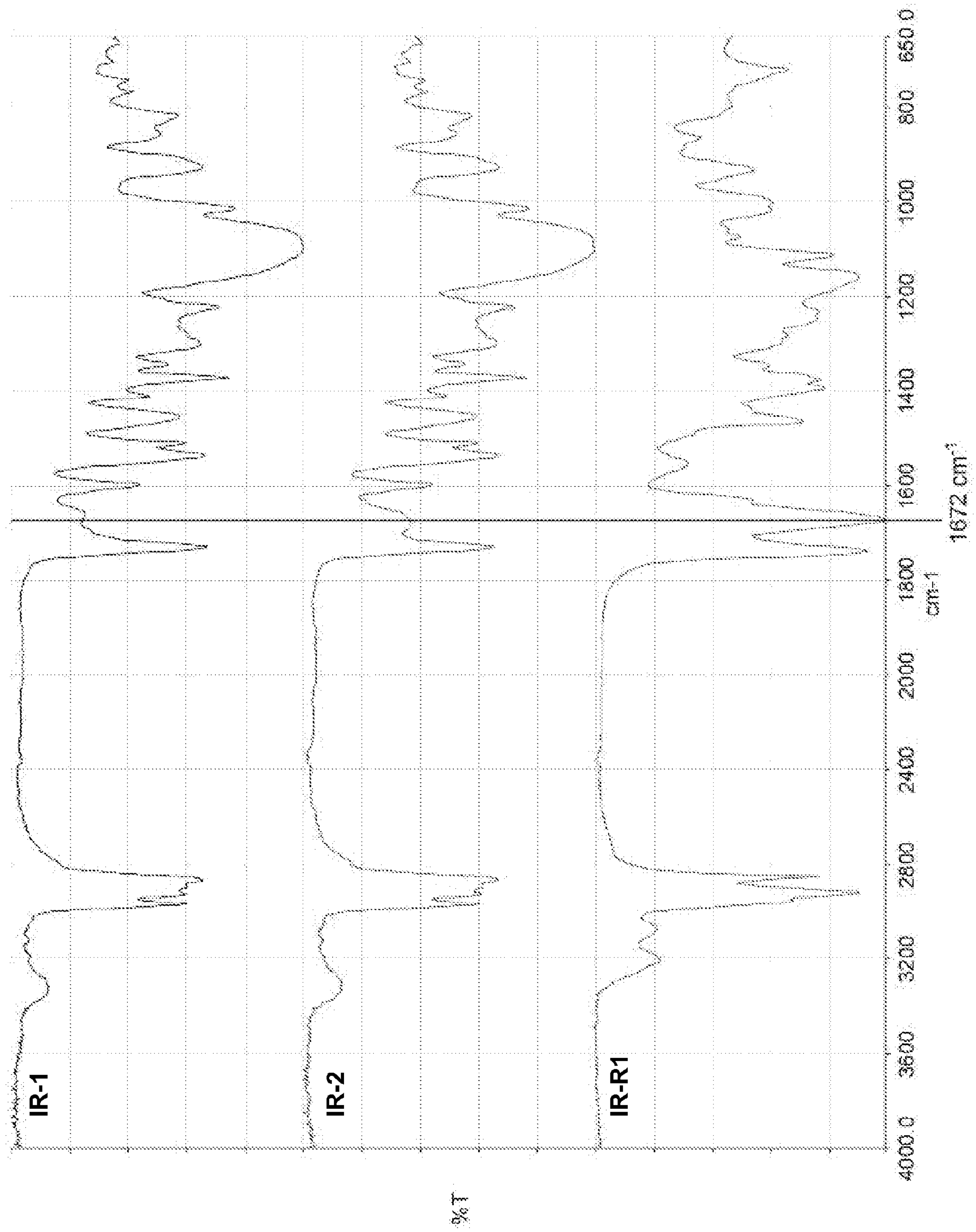
IR-1
IR-2
IR-R1
1672 cm$^{-1}$
cm-1
%T
4000.0
3600
3200
2800
2400
2000
1800
1600
1400
1200
1000
800
650.0

Figur 1

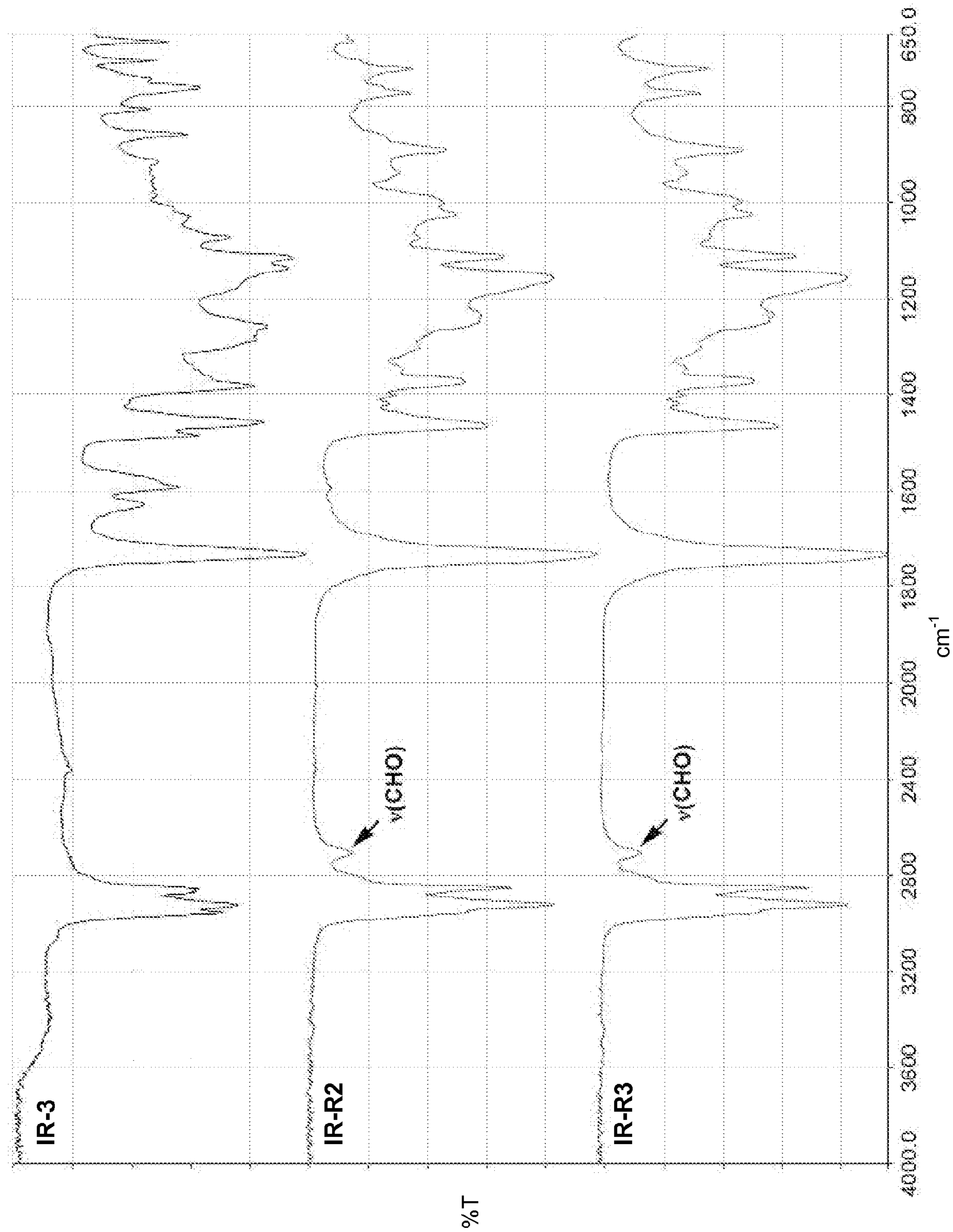
IR-3
IR-R2
IR-R3
ν(CHO)
ν(CHO)
%T
cm$^{-1}$
4000.0
3600
3200
2800
2400
2000
1800
1600
1400
1200
1000
800
650.0

Figur 2

Europäisches Patentamt
European Patent Office
Office européen des brevets

# EUROPÄISCHER RECHERCHENBERICHT

Nummer der Anmeldung
EP 07 15 0364

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | EP 1 775 284 A (SIKA TECHNOLOGY AG [CH]) 18. April 2007 (2007-04-18) * Seite 2, Zeilen 40-55 * Beispiele | 1-23 | INV. C08G18/12 C08G18/30 C08G18/32 C08G18/48 C08G18/50 C08G18/75 C08G18/76 C09J175/08 C07C243/24 C08K5/30 C07C251/72 |
| Y | EP 1 674 515 A (BAYER MATERIALSCIENCE AG [DE]) 28. Juni 2006 (2006-06-28) | 1-23 | |
| X | * Seite 12; Beispiel 5 * | 24-27,29 | |
| Y | EP 1 683 838 A (POLYPLASTICS CO [JP]) 26. Juli 2006 (2006-07-26) | 1-23 | |
| X | * Seite 2, Absatz 2-4 * * Beispiel 6; Tabelle 1 * | 24-27 | |
| Y | US 3 359 990 A (LEONARD RAY E ET AL) 26. Dezember 1967 (1967-12-26) * Spalten 5-6; Beispiele 1-3 * | 1-23 | |
| Y | US 2004/161591 A1 (ROZYNOV BORIS VASILYEVICH [US] ET AL) 19. August 2004 (2004-08-19) Seite 8, "Experimental" | 1-23 | RECHERCHIERTE SACHGEBIETE (IPC) |
| X | DATABASE CHEMCATS [ONLINE] Chemical Abstracts Service, Colombus, Ohio, US; XP002502670 gefunden im STN accession no. 2021155797 Order Number: VT-00099658 & "AKos Screening Library" 7. Februar 2006 (2006-02-07), AKOS-CONSULTING AND SOLUTIONS GMBH , P O BOX 141, BASEL, CH-4010, SWITZERLAND | 24 | C08G C08K |
| X | GB 1 020 201 A (BAYER AG) 16. Februar 1966 (1966-02-16) * Seite 2, Zeilen 29-90 * * Seiten 4-5; Beispiel 1 * | 28 | |

~~Der vorliegende Recherchenbericht wurde für alle Patentans~~prüche erstellt

| Recherchenort | Abschlußdatum der Recherche | Prüfer |
|---|---|---|
| München | 6. November 2008 | Lartigue, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

7

Europäisches Patentamt
European Patent Office
Office européen des brevets

Nummer der Anmeldung

EP 07 15 0364

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☒ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

1-29

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches Patentamt
European Patent Office
Office européen des brevets

**MANGELNDE EINHEITLICHKEIT DER ERFINDUNG ERGÄNZUNGSBLATT B**

Nummer der Anmeldung

EP 07 15 0364

Nach Auffassung der Recherchenabteilung entspricht die vorliegendeeuropäische Patentanmeldung nicht den Anforderungen an die Einheitlichkeit der Erfindungund enthält mehrere Erfindungen oder Gruppen von Erfindungen, nämlich:

1. Ansprüche: 1-23

(i) Eine härtbare Zusammensetzung, umfassend
a) mindestens ein Polyisocyanat P,
b) mindestens ein mittels Aldehyd oder keton blockiertes Amin BA,
c) mindestens ein Hydrazid HY einer Carbon- oder Sulfonsäure, welches ein Schmelzpunkt von mindestens 100°C aufweist.
(ii) Verfahren unter Einsatz obige Zusammensetzungen
(iii) Aus obigen Verfahren erhaltene Artikel

---

2. Ansprüche: 24-28

(i) Aldazyd der Formel (XXa) oder (XXb) gemäß der vorliegenden Ansprüche 24-27.
(ii) Verwendung eines Aldazids der Formel (XXa) oder (XXb) zur Reduktion der Vergilbung von ausgehärteten Polyurethanzusammensetzungen.

---

3. Anspruch: 28

Verwendung eines Hydrazids HY einer Carbon- oder Sulfonsäure, welches ein Schmelzpunkt von mindestens 100°C aufweist, zur Reduktion der Vergilbung von ausgehärteten Polyurethanzusammensetzungen.

---

4. Anspruch: 29

Verwendung eines Hydrazids HY einer Carbon- oder Sulfonsäure, welches ein Schmelzpunkt von mindestens 100°C aufweist, zur Reduktion der Aldehyd- oder Keton-Ausgasung aus der ausgehärteten Zusammensetzung.

---

## ANHANG ZUM EUROPÄISCHEN RECHERCHENBERICHT ÜBER DIE EUROPÄISCHE PATENTANMELDUNG NR.

EP 07 15 0364

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-11-2008

| Im Recherchenbericht angeführtes Patentdokument | Datum der Veröffentlichung | Mitglied(er) der Patentfamilie | Datum der Veröffentlichung |
|---|---|---|---|
| EP 1775284 A | 18-04-2007 | CA 2628324 A1 | 05-04-2007 |
| | | WO 2007036572 A1 | 05-04-2007 |
| | | KR 20080071132 A | 01-08-2008 |
| EP 1674515 A | 28-06-2006 | AU 2005248946 A1 | 13-07-2006 |
| | | BR PI0505635 A | 17-07-2007 |
| | | CA 2531471 A1 | 27-06-2006 |
| | | CN 1800231 A | 12-07-2006 |
| | | JP 3959092 B2 | 15-08-2007 |
| | | JP 2006182825 A | 13-07-2006 |
| | | KR 20060074850 A | 03-07-2006 |
| | | MX PA05013648 A | 04-09-2006 |
| | | US 2006141236 A1 | 29-06-2006 |
| EP 1683838 A | 26-07-2006 | CN 1902279 A | 24-01-2007 |
| | | JP 2005137785 A | 02-06-2005 |
| | | WO 2005044916 A1 | 19-05-2005 |
| | | KR 20070012322 A | 25-01-2007 |
| US 3359990 A | 26-12-1967 | GB 1095059 A | 13-12-1967 |
| US 2004161591 A1 | 19-08-2004 | KEINE | |
| GB 1020201 A | 16-02-1966 | BE 629689 A | |
| | | DE 1156552 B | 31-10-1963 |
| | | NL 136867 C | |
| | | NL 290227 A | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2004013088 A1
- WO 2007036571 A1
- DE 1156552
- US 6051620 A